(19) Europäisches Patentamt
European Patent Office
Office européen des brevets

(11) EP 0 384 362 B1

(12) # EUROPÄISCHE PATENTSCHRIFT

(45) Veröffentlichungstag und Bekanntmachung des Hinweises auf die Patenterteilung:
**15.12.1999 Patentblatt 1999/50**

(51) Int Cl.[6]: **C07K 5/10**, C07K 5/08, C07K 5/06, A61K 38/05, A61K 38/06, A61K 38/07

(21) Anmeldenummer: **90103167.4**

(22) Anmeldetag: **19.02.1990**

(54) **Glycinderivate**

Glycinderivatives

Dérivés de la glycine

(84) Benannte Vertragsstaaten:
**AT BE CH DE DK ES FR GB GR IT LI LU NL SE**

(30) Priorität: **23.02.1989 CH 66989**
**29.11.1989 CH 426589**

(43) Veröffentlichungstag der Anmeldung:
**29.08.1990 Patentblatt 1990/35**

(73) Patentinhaber: **F. HOFFMANN-LA ROCHE AG**
**4002 Basel (CH)**

(72) Erfinder:
- **Alig, Leo, Dr.**
  **CH-4303 Kaiseraugst (CH)**
- **Edenhofer, Albrecht, Dr.**
  **CH-4125 Riehen (CH)**
- **Müller, Marcel, Dr.**
  **CH-4402 Frenkendorf (CH)**
- **Trzeciak, Arnold**
  **D-7860 Schopfheim (DE)**
- **Weller, Thomas, Dr.**
  **CH-4058 Basel (CH)**

(74) Vertreter: **Lederer, Franz, Dr. et al**
**Lederer, Keller & Riederer**
**Patentanwälte**
**Prinzregentenstrasse 16**
**80538 München (DE)**

(56) Entgegenhaltungen:
**DE-A- 2 655 636**

- **PIERSCHBACHER ET AL.: "Influence of stereochemistry of the sequence Arg-Gly-Asp-Xaa on binding specificity in cell adhesion" JOURNAL OF BIOLOGICAL CHEMISTRY, Bd. 262, 1987, Seiten 17294-17298, XP000667256 BALTIMORE, US**
- **TANAKA ET AL.: "p-Amidino esters as irreversible inhibitors of Factors IXa and Xa and Thrombin" BIOCHEMISTRY, Bd. 25, 1986, Seiten 4929-4935, XP000667257 EASTON, PA US**
- **WALSMANN, P.: "Synthetische Inhibitoren der Serinproteasen" PHARMAZIE, Bd. 37, Nr. 6, 1982, Seite 457 XP000667258 BERLIN, DD**
- **STEINER ET AL.: "Ca2+ - dependent binding of a synthetic Arg-Gly-Asp (RGD) peptide to a single site on the purified platelet glycoprotein IIb-IIIa complex" JOURNAL OF BIOLOGICAL CHEMISTRY, Bd. 264, 1989, Seiten 13102-13108, XP000667259 BALTIMORE, US**
- **Chemicals Abstracts, vol.89, 1978, Columbus, Ohio, US;abstract no 60043, Seite 589**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).



Printed by Jouve, 75001 PARIS (FR)

## Beschreibung

Die vorliegende Erfindung betrifft neue Glycinderivate, Verfahren zu deren Herstellung, pharmazeutische Präparate, die solche Glycinderivate enthalten, sowie die Verwendung der Glycinderivate bei der Herstellung pharmazeutischer Präparate.

Die neuen Glycinderivate sind Verbindungen der Formel

$$R\text{-}CONH\text{-}CH_2\text{-}CONH\text{-}CH(R')\text{-}CH_2COOH \qquad I$$

worin R eine Gruppe der Formel

$$-CH(NH\text{-}R^a)\text{-}(CH_2)_{1\text{-}6}NH\text{-}R^b \qquad (R\text{-}1)$$

oder

$$-(T)_{1\ \text{oder}\ 0}\text{-}C_6H_4\text{-}CH_2NH\text{-}R^c \qquad (R\text{-}2)$$

$$-(T)_{1\ \text{oder}\ 0}\text{-}C_6H_4\text{-}(NH)_n\text{-}C(NH)\text{-}L \qquad (R\text{-}3)$$

ist, und

$R^a$ Wasserstoff, -COO-$C_{1-4}$-Alkyl, Z, -$COC_6H_5$, -$COC_6H_4N_3$, -$SO_2C_6H_5$, -$SO_2$-Naphthyl oder -$COCH_2N(Y)$-$CH_2CH_2NH$-Y,
Y Wasserstoff, Boc oder Z,
$R^b$ eine Gruppe der Formel -$C(NH)(CH_2)_{0-3}$ -$CH_3$ oder

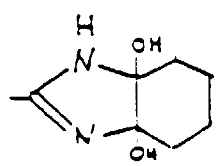

oder, falls $R^a$ eine Gruppe der Formel -$COC_6H_4N_3$, -$SO_2C_6H_5$, $SO_2$-Naphthyl oder -$COCH_2N(Y)$ -$CH_2CH_2NH$-Y ist, auch Amidino,
$R^c$ Wasserstoff oder Amidino,
n die Zahl 1 oder 0
L Amino,
T eine Gruppe der Formel -$CH_2$-$(O)_{1\ \text{oder}\ 0}$-, -CH=CH-, oder -$CH(R^d)$-$CH_2$-,
$R^d$ Wasserstoff oder -NH-$R^a$,
R' Wasserstoff oder -CO-$R^o$,
$R^o$ Amino, -NH-$C_{1-4}$-Alkyl, -$NH(CH_2)_{1-4}$-$C_6H_5$, -$NH(CH_2)_{1-4}$-$C_6H_4$-Hal, -NH-$C_6H_4$-COOH, -NH-$C_6H_4$-COO-$C_{1-4}$-Alkyl oder der Rest einer über die Aminogruppe gebundene α-Aminocarbonsäure ist,

sowie Hydrate oder Solvate und physiologisch verwendbare Salze davon.

Im Rahmen der vorliegenden Erfindung bezeichnet Me Methyl, Bzl Benzyl, tBu t-Butyl, Hal eines der 4 Halogene Boc t-Butoxycarbonyl, Z Benzyloxycarbonyl, Ac Acetyl, Su Succinimid, Fmoc 9H-Fluoren-9-ylmethoxycarbonyl, Arg L-Arginyl, Orn L-Ornithyl, Val L-Valyl, Phe L-Phenylalanyl, Leu L-Leucyl, Ile L-Isoleucyl, Lys Lysyl, Ser L-Seryl. Thr L-Threonyl, Gly Glycyl, Ala L-Alanyl, Asp L-α-Aspactyl, Aeg N-(2-Aminoäthyl)glycyl, Nal(1) 3-(1-NaphthyL)-L-alanyl.

Beispiele von über die Aminogruppe gebundenen α-Amino-carbonsäuren sind Val, Phe, Leu, Ile, Ser, Thr, Nal(1), N-Isopropyl-Gly, β-Cyclohexyl-Ala und Cycloleucin.

Die Verbindungen der Formel I können solvatisiert, insbesondere hydratisiert sein. Die Hydratisierung kann im Zuge des Herstellungsverfahrens erfolgen oder allmählich als Folge hygroskopischer Eigenschaften einer zunächst

wasserfreien Verbindung der Formel I auftreten.

Beispiele von physiologisch verwendbaren Salzen der Verbindungen der Formel I sind Salze mit physiologisch verträglichen Mineralsäuren, wie Salzsäure, Schwefelsäure oder Phosphorsäure, oder mit organischen Säuren, wie Methansulfonsäure, Essigsäure, Trifluoressigsäure, Zitronensäure, Fumarsäure, Bernsteinsäure oder Salicylsäure. Die Verbindungen der Formel I können auch Salze mit physiologisch verträglichen Basen bilden. Beispiele solcher Salze sind Alkalimetall-, Erdalkalimetall-, Ammonium- und Alkylammoniumsalze, wie das Na-, K-, Ca- oder Trimethylammoniumsalz. Verbindungen der Formel I, die eine Amino, Amidino- oder Guanidinogruppe enthalten, können in Form von Zwitterionen vorliegen.

Die Verbindungen der Formel I, die ein oder mehrere asymmetrische C-Atome enthalten, können als Enantiomere, als Diastereomere oder als Gemische davon, z.B. als Racemate, vorliegen.

Bevorzugte Verbindungen der Formel I sind diejenigen, worin R-CO- die Gruppe Aeg-Arg-, Z-Aeg(Z)-Arg-, 2-Naphthyl-$SO_2$-Arg-, o-Azidobenzoyl-Arg-, $N^2$-Boc-$N^6$-(1-Iminoäthyl)-Lys-, $N_2$-Boc-$N_5$-(3a,4,5,6,7,7a-Hexahydro-3a, 7a--dihydroxy -1H-benzimidazol-2-yl)-Orn-, p-(Aminomethyl)-hydrocinnamoyl, p-Amidinohydrocinnamoyl, 3-(p-Amidinophenyl oder p-Guanidinophenyl)-alanyl, N-Z- oder N-Boc-3-(p-Amidinophenyl)alanyl, N-Boc-3-(p-Guanidinophenyl) alanyl, p-Amidinophenoxyacetyl oder p-Amidinophenacetyl darstellt.

Bevorzugt sind ferner die Verbindungen der Formel I, worin R' Wasserstoff, -CO-Val-OH, -CO-Ser-OH, -CO-Phe-OH, 1-Carboxy-2-(1-naphthyl)äthylidencarbamoyl, -CO-Ile-OH, Carboxyphenylcarbamoyl, Isobutylcarbamoyl oder p-Fluorphenäthylcarbamoyl ist.

Besonders bevorzugt sind folgende Verbindungen:

[3-(p-Amidinophenyl)-DL-alanyl]-Gly-Asp-Val-OH,
Z-Aeg(Z)-Arg-Gly-Asp-Val-OH,
Aeg-Arg-Gly-Asp-Val-OH,
Aeg-Arg-Gly-Asp-Ser-OH,
N-Aeg-Arg-Gly-Asp-Nal(1)-OH,
Aeg-Arg-Gly-Asp-Ile-OH,
[$N^2$-Boc-$N^6$-(1-Iminoäthyl)-L-lysyl]-Gly-Asp-Val-OH,
N-[(o-Azidobenzoyl)-Arg-Gly-Asp]-anthranilsäure,
$N^2$-Boc-$N^5$-(3a,4,5,6,7,7a-Hexahydro-3a,7a-dihydroxy-1H-benzimidazol-2-yl)-L-ornithyl]-Gly-Asp-Val-OH,
[N-Boc-3-(p-Guanidinophenyl)-DL-alanyl]-Gly-Asp-Val-OH,
[3-(p-Amidinophenyl)-DL-alanyl]-Gly-Asp-Nal(1)-OH,
[N-Boc-3-(p-Amidinophenyl)-DL-alanyl]-Gly-Asp-Val-OH,
(p-Amidinohydrocinnamoyl)-Gly-Asp-Nal(1)-OH,
[3-(p-Amidinophenyl)-D-alanyl]-Gly-Asp-Val-OH,
(p-Aminomethylhydrocinnamoyl)-Gly-Asp-Val-OH,
(p-Amidinohydrocinnamoyl)-Gly-Asp-Val-OH,
[3-(p-Amidinophenyl)-L-alanyl]-Gly-Asp-Val-OH,
(p-Amidinophenoxy)acetyl-Gly-Asp-Val-OH und
(p-Amidinophenyl)acetyl-Gly-Asp-Val-OH.

Die Verbindungen der vorliegenden Erfindung kann man in an sich bekannter Weise dadurch herstellen, dass man

a) in einer Verbindung der Formel

$$R^2\text{-CONH-CH}_2\text{-CONH-CH}(R^4)\text{-CH}_2\text{COOR}^3 \qquad \text{II}$$

worin $R^2$ eine Gruppe der Formel

$$\text{-CH(NH-R}^a)\text{-(CH}_2)_{1\text{-}6}\text{-R}^5 \qquad \text{(R-1a)}$$

$$\text{-(T)}_{1\ \text{oder}\ 0}\text{-C}_6\text{H}_4\text{-CH}_2\text{-R}^6 \qquad \text{(R-2a)}$$

oder

$-(T)_{1 \text{ oder } 0}-C_6H_4-R^7$ (R-3a)

ist, in

der $R^5$ eine geschützte Guanidinogruppe oder eine Gruppe $-NH-R^b$,
$R^6$ eine geschützte Amino- oder Guanidinogruppe oder eine Gruppe $-NH-R^c$,
$R^7$ gegebenenfalls geschütztes Amidino oder Guanidino,
$R^3$ Wasserstoff oder eine leicht spaltbare Estergruppe,
$R^4$ die gleiche Bedeutung wie R' hat oder eine Gruppe $-COR^8$ ist, in der $R^8$ ein über die Aminogruppe gebundener leicht spaltbarer a-Aminocarbonsäureester ist,
wobei, falls $R^3$ Wasserstoff ist und $R^4$ die gleiche Bedeutung wie R' hat, $R^2$ zumindest eine geschützte Guanidino-, Amino- oder Amidinogruppe $R^5$, $R^6$ bzw.
$R^7$ enthalten soll,
und $R^a$, $R^b$, $R^c$, R' und T die obige Bedeutung haben,

die vorhandene(n) Estergruppe(n) und eine oder mehrere vorhandene geschützte Amino-, Amidino- oder Guanidinogruppen spaltet oder

b) ein Amin der Formel

$$\begin{array}{l} CH(NH_2)-(CH_2)_{1-6}-NH-C(NH)NH_2 \\ | \\ CONH-CH_2-CONH-CH(R')-CH_2COOH \end{array} \qquad III$$

mit einem die Gruppe $-COC_6H_5$, $-SO_2C_6H_5$,
$-SO_2$-Naphthyl oder $-COCH_2N(Y)-CH_2CH_2NH-Y$ einführenden Mittel umsetzt, worin R' und Y die obige Bedeutung haben, oder

c) ein Guanidinderivat der Formel

$$\begin{array}{l} CH(NH-R^9)-(CH_2)_{1-6}-NHC(NH)NH_2 \\ | \\ CONH-CH_2-CONH-CH(R')-CH_2COOH \end{array} \qquad \underline{IV}$$

worin $R^9$ $-COO-C_{1-4}$-Alkyl, Z, $-COC_6H_5$,
$-COC_6H_4N_3$, $-SO_2C_6H_5$, $-SO_2$-Naphthyl oder
$-COCH_2N(Y')-CH_2CH_2NH-Y'$ ist, in dem Y' Boc oder Z darstellt
und R' obige Bedeutung hat,
mit 1,2-Cyclohexandion umsetzt oder

d) in einem Amin der Formel

$$\begin{array}{l} NH-CH_2-CONH-CH(R')-CH_2-COOH \\ | \\ CO-(L)_{1 \text{ oder } 0}-C_6H_4-(CH_2)_{1 \text{ oder } 0}-NH_2 \end{array} \qquad \underline{V}$$

worin L eine Gruppe $-CH_2(O)_{1 \text{ oder } 0}-$, -CH=CH- oder $-CH(NH-R^9)-CH_2-$ ist, und R' und $R^9$ die obige Bedeutung haben,
die Aminogruppe in eine Guanidinogruppe umwandelt oder

e) ein Nitril der Formel

$$N{\equiv}C-C_6H_4-(T)_{1 \text{ oder } 0}-CONH-CH_2-CONH-CH(R')-CH_2COOH \qquad VI$$

zum Amin hydriert,

f) gewünschtenfalls einen in der Gruppe R einer Verbindung der Formel I enthaltenen reaktionsfähigen Substituenten funktionell abwandelt und

g) gewünschtenfalls eine Verbindung der Formel I in ein Salz oder ein Salz einer Verbindung der Formel I in die freie Verbindung der Formel I überführt.

Beispiele von geschützten Amino-, Amidino- und Guanidinoschutzgruppen sind -NH-Z und -NH-Boc, -C(NH) NH-Z: $-NHC(NH)NH-NO_2$, -NHC(N-Boc)-NH-Boc und -NHC(N-Z)-NH-Z. Beispiele von leicht spaltbaren Estergruppen $COOR^3$ sind Methoxy-, t-Butoxy- und Benzyloxycarbonyl. Beispiele für einen Rest $R^8$ sind -Val-OtBu, -Val-OBzl und -Ser(tBu)-OtBu.
Die Abspaltungen nach der Verfahrensvariante a) lassen sich in an sich bekannter Weise durchführen. So kann man Estergruppen, wie t-Butoxycarbonyl, mit einer Säure, wie Ameisensäure, Trifluoressigsäure (TFA) oder Salzsäure, in einem Lösungsmittel, wie Methylenchlorid, Tetrahydrofuran (THF) oder Aethylacetat, bei einer Temperatur bis zu etwa 40°C, vorzugsweise zwischen etwa 0°C und Raumtemperatur, spalten. Dabei werden gleichzeitig im Substituenten $R^2$ enthaltene Amino-, Guanidino- oder Amidinoschutzgruppen, wie Boc, abgespalten. Auf diese Weise lassen sich auch durch Festphasen-Synthese erhaltene Verbindungen der Formel II vom Träger, z.B. einem p-Benzyloxybenzylalkohol-Reste -Reste enthaltenden Styrol-1% Divinylbenzol-Harz, entfernen.
Estergruppen, wie Methoxycarbonyl, kann man mit einer Base, wie einem Alkalimetallhydroxyd, z.B. Natronlauge, in einem Lösungsmittel, wie Aceton, bei einer Temperatur bis zu etwa 40°C, vorzugsweise bei Raumtemperatur, verseifen. Benzylester können durch Hydrierung in Gegenwart eines Edelmetallkatalysators, wie Palladium auf Kohlenstoff (Pd/C) in einem Lösungsmittel, wie Methanol, Aethanol, Ameisensäure oder Essigsäure, bei einer Temperatur bis zu etwa 40°C, vorzugsweise bei Raumtemperatur gespalten werden. Dabei werden gleichzeitig in der Gruppe $R^2$ enthaltene Aminooder Amidinoschutzgruppen, wie Z; bzw. Guanidinoschutzgruppen, wie $NO_2$ und Z, abgespalten.
Die Variante b) kann man ebenfalls in an sich bekannter Weise durchführen. Zur Einführung der Gruppe $-COC_6H_4N_3$ kann man eine Verbindung der Formel III mit Pyridinhydrochlorid in Gegenwart einer Base, wie N-Aethyldiisopropylamin (DIPEA), und von 1,1,3,3-Tetramethyl-2-(4-oxo-1,2,3-benzotriazin-3(4H)-yl]uroniumhexafluorphosphat (HOBTU) in einem Lösungsmittel, wie DMF, umsetzen. Zur Einführung einer Gruppe $-SO_2$-Naphthyl kann eine Verbindung der Formel III z.B. mit Naphthalin-2-sulfonylchlorid und einer Base, wie $NaHCO_3$, in einem Lösungsmittel, wie Aceton und Wasser, versetzt werden. Zur Einführung einer Gruppe $-COCH_2N(Y)-CH_2CH_2NH-Y$ kann man eine Verbindung der Formel III z.B. mit Z-Aeg(Z)-OSu in Gegenwart von einer Base, wie Pyridinhydrochlorid, in einem Lösungsmittel, wie DMF, umsetzen. Zweckmässigerweise lassen sich diese Reaktionen bei einer Temperatur bis zu etwa 40°C, vorzugsweise bei Raumtemperatur, durchführen.
Die Reaktion nach Variante c) kann man in einem Natriumboratpuffer unter einer inerten Atmosphäre, wie Argon, bei einer Temperatur bis zu etwa 40°C, vorzugsweise bei Raumtemperatur durchführen.
Zur Durchführung der Variante d) kann man das Amin der Formel V in einem Lösungsmittel, wie Wasser, mit einer Base, z.B. Kaliumcarbonat, und Aminoiminomethansulfonsäure bei einer Temperatur bis zu etwa 40°C, vorzugsweise bei Raumtemperatur umsetzen.
Zur Durchführung der Variante e) kann man das Nitril der Formel VI in einer alkoholischen, z.B. einer methanolischen Ammoniaklösung in Gegenwart von einem Katalysator, wie Raney-Nickel, bei einer Temperatur bis zu etwa 40°C, vorzugsweise bei Raumtemperatur, hydrieren.
Die funktionellen Abwandlungen nach Variante f) lassen sich ebenfalls nach an sich geläufigen Methoden bewerkstelligen. So kann man aus einer geschützten Gruppe R-CO-, wie Z-Aeg(Z)-Arg oder N-Boc-3-(p-Amidinophenyl)-D,L-alanyl, die Schutzgruppen abspalten, z.B. wie weiter oben beschrieben im Zusammenhang mit der Variante a).
Eine in einem Substituenten R enthaltene primäre Aminogruppe kann man in die -NH-Boc-Gruppe überführen, z.B. mittels Di-t-butyldicarbonat in einem Lösungsmittel, wie DMF, in Gegenwart von einer Base, wie Triäthylamin, bei einer Temperatur bis zu etwa 40°C, vorzugsweise bei Raumtemperatur.

Die Verbindungen der Formel II sind neu und ebenfalls Gegenstand der Erfindung. Ihre Herstellung kann ausgehend von bekannten Verbindungen nach an sich bekannten und dem Fachmann geläufigen Methoden erfolgen. So lassen sich Amidine der Formel II, worin $R^2$ eine Gruppe $-(T)_{1\ \text{oder}\ 0}-C_6H_4-C(NH)NH_2$ ist, ausgehend von den entsprechenden Nitrilen der Formel

$$NC-C_6H_4-(T)_{1\ \text{oder}\ 0}-CONH-CH_2-CONH-CH(R^4)-CH_2COOR^3 \qquad VII$$

über die entsprechenden Thioamide und S-Methyliminoester herstellen. Beispielsweise lässt man das Nitril VII mit Schwefelwasserstoff und Triäthylamin in Pyridin reagieren und setzt das erhaltene Thioamid mit Methyljodid in Aceton und anschliessend mit Ammoniumacetat in Methanol um.

Ein Guanidinderivat der Formel II, worin $R^2$ eine Gruppe der Formel $-(T)_{1\ \text{oder}\ 0}-C_6H_4-NHC(NH)NH_2$ ist, kann man herstellen ausgehend von der entsprechenden Nitroverbindung der Formel

$$NHCO-(T)_{1\ \text{oder}\ 0}-C_6H_4-NO_2$$
$$|$$
$$CH_2-CONH-CH(R^4)-CH_2COOR^3 \qquad VIII$$

über das entsprechende primäre Amin und das entsprechende durch Nitro geschützte Guanidinderivat. So kann man die Nitroverbindung VIII in Gegenwart von Pd/C zum primären Amin hydrieren, letzteres mit 3,5-Dimethyl-N-nitro-1H-pyrazol--1-carboxamidin in Aethanol umsetzen und die Nitrogruppe durch Hydrierung in Gegenwart von Pd/C in Essigsäure abspalten.

Durch Festphasen-Synthese lassen sich an einen Träger gebundene Verbindungen der Formel II in an sich bekannter Weise herstellen. So kann man eine Suspension eines Trägers bestehend aus einem p-Benzyloxybenzylalkohol-Reste enthaltenden Styrol-Divinylbenzol-Harz in DMF mit N-(9H-Fluoren--9-ylmethoxycarbonyl)-3-(1-naphthyl)-L-alanin, dann mit 2-(1H-Benzotriazol-1-yl)-1,1,3,3-tetramethyluronium -hexafluorphosphat (HBTU), 4-Dimethylaminopyridin und DIPEA versetzen. Die freien Hydroxygruppen kann man mit Essigsäureanhydrid in Gegenwart von DIPEA in DMF acetylieren. Anschliessend kann man z.B. nach dem in Beispiel 10 angegebenen Reaktionsprotokoll die einzelnen geschützten Aminosäuren, wie Fmoc-Asp(OtBu)-OH, Fmoc-Gly-OH, Fmoc-Arg-OH•HCl und Boc-Aeg (Boc)-OSu, nacheinander zu einer Verbindung der Formel

Boc-Aeg(Boc)-Arg-Gly-Asp(OBut)-Nal(1)-O-Träger

koppeln.

Säuren der Formel III, wie H-Arg-Gly-Asp-Val-OH und H-Arg-Gly-Asp-Ser-OH, sind bekannt oder können in an sich bekannter Weise hergestellt werden, z.B. durch Umsetzung eines entsprechenden Esters der Formel

$$H_2N-CH_2-CONH-CH(R')-CH_2COO-t-Bu \qquad IX$$

mit Z-Arg($Z_2$)-OSu, Spaltung der Estergruppe und Entfernung der im so erhaltenen Produkt vorhandenen Argininschutzgruppen durch katalytische Hydrierung in Methanol in Gegenwart von Pd/C.

Ein Guanidinderivat IV, z.B. ein solches worin $R^9$ eine Gruppe $-COO-C_{1-4}$-Alkyl ist, kann man herstellen durch Umsetzung der entsprechenden Verbindung der Formel III mit Di-t-butyl-dicarbonat in Gegenwart von Pyridinhydrobromid in wässrigem Dioxan.

Ein Amin der Formel V, z.B. Boc-D,L-Phe(p-$NH_2$)-GLy-Asp-Val-OH, lässt sich herstellen ausgehend von einem entsprechenden Diester, z.B. H-GLy-Asp(OBzL)-VaL-OBzl•TFA, über die Nitroverbindung Boc-D,L-Phe(p-$NO_2$)-Gly-Asp(OBzl)--Val-OBzl. So kann man den besagten Diester mit Boc-D,L--Phe(p-$NO_2$)-OH in DMF in Gegenwart von HBTU und DIPEA versetzen und die erhaltene Nitroverbindung zum erwünschten Amin katalytisch hydrieren.

Ein Nitril VI, z.B. ein solches worin R' die Gruppe -CO-Val-OH ist, lässt sich herstellen durch Kupplung eines entsprechenden Diesters, z.B. H-Gly-Asp(OtBu)-Val-OtBu, und eines entsprechenden Nitrils, wie der p-Cyanhydrozimtsäure. zum p-Cyanhydrocinnamoyl-Gly-Asp(OtBu)-Val-OtBu und Acidolyse des letzteren.

Ein Nitril der Formel VII, z.B. ein solches worin $R^4$ die Gruppe -CO-Ser(tBu)-OtBu ist, lässt sich durch Kupplung eines entsprechenden Diesters, z.B. H-Gly-Asp(OtSu)--Ser(tBu)-OtBu, mit rac-Z-(p-Cyanophenyl)alanin in DMF unter Argon in Gegenwart von N-Methylmorpholin und HBTU herstellen.

Analog kann man einen Diester, wie H-Gly-Asp(OtBu)-Val--OtBu mit Boc-Phe(4-$NO_2$)-OH zur entsprechenden Nitroverbindung VIII, z.B. [N-Boc-3-(p-Nitrophenyl)-L-alanyl]-Gly--Asp(OtBu)-Val-OtBu, kuppeln.

Eine Verbindung der Formel IX, z.B. die N-[H-Gly--Asp(OtBu)]-anthranilsäure, kann man herstellen durch Kupplung von Z-Asp(OtBu)-OH und Anthranilsäurebenzylester•tosylat in DMF mit 1,1,3,3-Tetramethyl-2-[4-oxo--1,2,3-benzotriazin -3(4H)-yl]uronium-tetrafluorborat (TOBTU) und DIPEA, Abspaltung der Z-Gruppe aus dem erhaltenen Ester. Kupplung der erhaltenen Verbindung, N-[H-Asp(OtBu)]-anthranilsäure, mit Z-Gly-OSu und Abhydrierung der Z-Gruppe aus dem erhaltenen Produkt.

Ein Ausgangsdiester, wie H-GLy-Asp(OBzl)-Val-OBzl•TFA, lässt sich herstellen durch Kupplung von Boc-Asp (OBzl)-OH mit H-Val-OBzl•Tosylat in Gegenwart von N-methylmorpholin und Chlorameisensäureisobutylester in DMF, Acidolyse des erhaltenen Boc-Asp(OBzl)-Val-OBzl mit TFA, Kupplung des erhaltenen H-Asp(OBzl)-Val-OBzl•TFA mit Boc-Gly-OSu und N-Methylmorpholin in Aethylacetat und anschliessende Acidolyse.

Ein Diester, wie H-Gly-Asp(OtBu)-Nal(1)-OMe, kann man herstellen durch Kupplung von Z-Gly-OH mit H-Asp(OtBu)-OMe, Verseifung des erhaltenen Z-Gly-Asp(OtBu)-OMe mit Natriumhydroxid in Aceton, Kupplung des erhaltenen Z-Gly-Asp(OtBu)--OH mit H-Nal(1)-OMe und Hydrogenolyse des erhaltenen Z-Gly-Asp(OtBu)-Nal(1)-OMe.

Ein Diester, wie H-Gly-Asp(OtBu)-Val-OtBu lässt sich herstellen durch Kondensation von Z-Asp(OtBu)-OH und H-Val-OtBu zu Z-Asp(OtBu)-Val-OtBu, Hydrogenolyse des letzteren, Kupplung des erhaltenen H-Asp(OtBu)-Val-OtBu mit Z-Gly-OSu zu Z-Gly-Asp(OtBu)-Val-OtBu und Hydrogenolyse des letzteren.

Die Glycinderivate der Formel I, ihre Solvate und ihre Salze hemmen sowohl die Bindung von Fibrinogen, Fibronectin und des Willebrand-Faktors an den Fibrinogenrezeptor der Blutplättchen (Glykoprotein IIb/IIIa) als auch die Bindung derselben und weiterer adhäsiver Proteine, wie Vitronectin, Kollagen und Laminin, an die entsprechenden Rezeptoren auf der Oberfläche verschiedener Zelltypen. Die besagten Verbindungen beeinflussen somit Zell-Zell- und Zell-Matrix--Wechselwirkungen. Sie verhindern insbesondere die Entstehung von Blutplättchenthromben und können bei der Bekämpfung bzw. Verhütung von Krankheiten wie Thrombose, Hirnschlag, Herzinfarkt, Entzündung und Arteriosklerose verwendet werden. Ferner haben diese Verbindungen einen Effekt auf Tumorzellen, indem sie deren Metastasierung hemmen. Somit können sie auch als Antitumor-Mittel eingesetzt werden.

Die Hemmung der Fibrinogenbindung an den Fibrinogenrezeptor, Glykoprotein IIb/IIIa, kann wie folgt nachgewiesen werden:

Das Glykoprotein IIb/IIIa wird aus Triton X-100 Extrakten von menschlichen Blutplättchen gewonnen und durch Lectin-Affinitätschromatographie (Analytical Biochemistry 151, 1985, 169-177) und Chromatographie an einer Arg-Gly-Asp-Ser-Affinitätssäule (Science 231, 1986, 1559-62) gereinigt. Das so erhaltene Rezeptorprotein wird an Mikrotiterplatten gebunden. Die spezifische Bindung von Fibrinogen an den immobilisierten Rezeptor wird mit Hilfe eines ELISA-Systems ("enzyme-linked immunosorbent assay") bestimmt. Die nachstehenden $IC_{50}$-Werte entsprechen derjenigen Konzentration der Testsubstanz, welche benötigt wird, um die Bindung von Fibrinogen an den immobilisierten Rezeptor um 50% zu hemmen:

| Produkt von Beispiel: | 2 | 6 | 7 | 8 | 10 | 11 | 12 | 13 |
|---|---|---|---|---|---|---|---|---|
| $IC_{50}$ (µM) | 0,016 | 0,15 | 0,09 | 0,11 | 0,038 | 0,12 | 0,11 | 0,11 |

| Produkt von Beispiel: | 14 | 15 | 16 | 17 | 19 | 20 |
|---|---|---|---|---|---|---|
| $IC_{50}$ (µM) | 0,054 | 0,08 | 0,022 | 0,016 | 0,011 | 0,022 |

| Produkt von Beispiel: | 23 | 24 | 25 | 26 | 27 |
|---|---|---|---|---|---|
| $IC_{50}$ (µM) | 0,21 | 0,035 | 0,023 | 0,005 | 0,0019 |

Die Verbindungen der Formel I haben eine geringe Toxizität. So hat das Produkt von Beispiel 2 eine $LD_{50}$ von 600 mg/kg intravenös an der Maus.

Wie eingangs erwähnt, sind Arzneimittel, enthaltend ein Glycinderivat der Formel I, ein Solvat davon oder ein Salz davon, ebenfalls Gegenstand der vorliegenden Erfindung, weiterhin auch ein Verfahren zur Herstellung derartiger Arzneimittel, welches dadurch gekennzeichnet ist, dass man eine oder mehrere der besagten Verbindungen und erwünschtenfalls einen oder mehrere andere therapeutisch wertvolle Stoffe in eine galenische Darreichungsform bringt. Die Arzneimittel können enteral, z.B. oral in Form von Tabletten, Lacktabletten, Dragees, Hart- und Weichgelatinekapseln, Lösungen, Emulsionen oder Suspensionen, oder rektal, z.B. in Form von Suppositorien, oder als Spray verabreicht werden. Die Verabreichung kann aber auch parenteral, z.B. in Form von Injektionslösungen, erfolgen.

Zur Herstellung von Tabletten, Lacktabletten, Dragees und Hartgelatinekapseln kann der Wirkstoff mit pharmazeutisch inerten, anorganischen oder organischen Excipientien vermischt werden. Als solche Excipientien kann man für Tabletten, Dragees und Hartgelatinekapseln z.B. Lactose, Maisstärke oder Derivate davon, Talk, Stearinsäure oder deren Salze, verwenden. Für Weichgelatinekapseln eignen sich als Excipientien z.B. vegetabile Oele, Wachse, Fette, halbfeste und flüssige Polyole; je nach Beschaffenheit des Wirkstoffes sind jedoch bei Weichgelatinekapseln überhaupt keine Excipientien erforderlich. Zur Herstellung von Lösungen und Sirupen eignen sich als Excipientien z. B. Wasser, Polyole, Saccharose, Invertzucker und Glukose, für Injektionslösungen eignen sich z.B. Wasser, Alkohole, Polyole, Glycerin und vegetabile Oele, und für Suppositorien eignen sich z.B. natürliche oder gehärtete Oele, Wachse, Fette und halbflüssige oder flüssige Polyole. Die pharmazeutischen Präparate können daneben noch Konservierungsmittel, Lösungsvermittler, Stabilisierungsmittel, Netzmittel, Emulgiermittel, Süssmittel, Färbemittel, Aromatisierungsmittel, Salze zur Veränderung des osmotischen Druckes, Puffer, Ueberzugsmittel oder Antioxidantien enthalten.

Für die Bekämpfung bzw. Verhütung der weiter oben genannten Krankheiten kann die Dosierung des Wirkstoffes innerhalb weiter Grenzen variieren und ist natürlich in jedem einzelnen Fall den individuellen Gegebenheiten anzupassen. Im allgemeinen dürfte bei oraler oder parenteraler Verabreichung eine Dosis von etwa 0,1 bis 20 mg/kg, vorzugsweise von etwa 0,5 bis 4 mg/kg, pro Tag für den Erwachsenen angemessen sein, wobei aber die soeben

angegebene obere Grenze auch über- schritten werden kann, wenn sich dies als angezeigt erweisen sollte.

Beispiel 1

A) Eine Lösung von 55 mg [3-(p-Amidinophenyl)-DL-alanyl] -Gly-Asp(OtBu)-Ser(tBu)-OtBu-Hydrojodid wird in einer Mischung von 10 ml Methylenchlorid und 5 ml Trifluoressigsäure unter Argon 2 Stunden bei Raumtemperatur aufbewahrt. Nach Abdampfen des Lösungsmittels erhält man 43 mg (86%) [3-(p-Amidinophenyl)-DL-alanyl]-Gly-Asp-Ser-OH-Trifluoracetat (2:3), Smp. 223-224°C aus Essigester/Isopropyläther.

B) Der Ausgangsester wird wie folgt hergestellt:

a) Eine auf 0°C abgekühlte Lösung von 1,95 g H-Ser(tBu)--OtBu-tosylat in DMF wird durch Zugabe von N-Methylmorpholin auf pH 8 gebracht. Hierzu wird eine Lösung von 2,1 g Z-Asp(OtBu)-OSu in 160 ml DMF addiert. Unter Argon wird 1 Stunde bei 0°C gerührt und über Nacht im Kühlschrank aufbewahrt. Der nach Verdampfen des Lösungsmittels zurückbleibende Rückstand wird in Essigester aufgenommen und mit gesättigter Natriumbicarbonatlösung, Wasser, 10%iger Kaliumhydrogensulfatlösung und Wasser gewaschen, getrocknet, filtriert und eingedampft. Man erhält 2,09 g (80%) Z-Asp(OtBu)-Ser(tBu)-OtBu, Smp. 79-80°C aus Essigester/ n-Hexan.

b) 1,9 g Z-Asp(OtBu)-Ser(tBu)-OtBu werden in 100 ml Methanol in Gegenwart von 0,1 g Pd/C 10% hydriert. Nach Aufnahme der theoretischen Menge Wasserstoff wird filtriert und zur Trockene verdampft. Chromatographie an Kieselgel mit Methylenchlorid/MeOH (98:2) ergibt 1,28 g (91%) H-Asp(OtBu)--Ser(tBu)-OtBu, MS: 389 $(M+H)^+$.

c) Analog wie in a) beschrieben erhält man aus Z-Gly-OSu und H-Asp(OtBu)-Ser(tBu)-OtBu das Z-Gly-Asp (OtBu)-Ser(tBu)--OtBu, Ausbeute: 86%, $[\alpha]_D$ -6,9° (c 0,9, MeOH).

d) Analog wie in b) beschrieben erhält man durch Hydrogenolyse von Z-Gly-Asp(OtBu)-Ser(tBu)-OtBu das H-Gly-Asp(OtBu)--Ser(tBu)-OtBu. Ausbeute: 75%, MS: 446 $(M+H)^+$.

e) Zu einer Lösung von 200 mg rac N-Z-3-(p-Cyanophenyl)-alanin (Pharmazie 40, 1985, 305) und 294 mg H-Gly-Asp(OtBu)--Ser(tBu)-OtBu in 10 ml DMF fügt man unter Argon 67 mg N-Methylmorpholin und 250 mg HBTU zu und bewahrt die Mischung über Nacht auf. Das nach dem Abdampfen des Lösungsmittels erhältliche Oel wird in Essigester gelöst, die Lösung mit 5% Natriumbicarbonatlösung und Wasser gewaschen, getrocknet und eingedampft. Der Rückstand wird an Kieselgel mit Essigester chromatographiert. Man erhält 160 mg [N-Z-3-(p-Cyanophenyl)-DL-alanyl]-Gly-Asp(OtBu)-Ser(tBu)-OtBu (1:1 Gemisch von Epimeren), Smp. 117-119°C aus Aether/ n-Hexan.

f) Eine Lösung von 362 mg des Produkts von e) in 40 ml Pyridin und 3 ml Triäthylamin wird nach Sättigung mit $H_2S$ 2 Tage aufbewahrt, dann in Wasser eingerührt und mit Essigester extrahiert. Das Produkt wird an Kieselgel mit Methylenchlorid/Methanol chromatographiert. Man erhält 270 mg [N-Z-3-(p-Thiocarboxamidophenyl) -DL-alanyl]-Gly-Asp(OtBu)--Ser(tBu)-OtBu (Epimerengemisch 1:1), MS: 786 $(M+H)^+$.

g) Das Thioamid der Vorstufe wird in 30 ml Aceton gelöst, mit 0,6 ml Methyljodid versetzt und 3 Stunden unter Rückfluss erhitzt. Nach Filtration und Einengen wird das Produkt durch Zugabe von Aether ausgefällt. Man erhält 181 mg (57%) [N-Z-3-(p-Methylthiocarboximidophenyl) -DL-alanyl]-Gly--Asp(OtBu)-Ser(tBu)-OtBu-Hydrojodid (Epimerengemisch 1:1), Smp. 136-138°C.

h) Eine Lösung von 180 mg des Jodids der Vorstufe in 30 ml MeOH wird mit 36 mg Ammoniumacetat versetzt und 5 Stunden auf 60°C erwärmt. Nach dem Abkühlen und Filtrieren wird das Produkt mit Aether ausgefällt. Man erhält 89 mg (51%) [N-Z-3-(p-Amidinophenyl) -DL-alanyl]-Gly-Asp(OtBu)-Ser(tBu)--OtBu-Hydrojodid (1: 1 Epimerengemisch), Smp. 150°C (Zers.) aus Essigester/n-Hexan.

i) In analoger Weise wie unter b) beschrieben erhält man bei der Hydrogenolyse des Produkts von h) das [3-(p-Amidino-phenyl) -DL-alanyl]-Gly-Asp(OtBu)-Ser(tBu)-OtBu-Hydrojodid (1:1 Epimerengemisch), Smp. 163-164°C aus Essigester/ n-Hexan, Ausbeute: 70%.

Beispiel 2

A) Analog wie in Beispiel 1A beschrieben erhält man bei Einsatz von [N-Boc-3-(p-Amidinophenyl)-DL-alanyl]-Gly--Asp(OtBu)-Val-OtBu -Hydrojodid-Acetat (1:1) das [3-(p-Amidinophenyl)-DL-alanyl]-Gly-Asp-Val-OH-trifluoracetat (1:2), Smp. 174° (Zers.) aus Methanol/Essigester, Ausbeute: 75%.

B) Das Ausgangsmaterial kann auf folgende Weise hergestellt werden:

a) Bei Kondensation von Z-Asp(OtBu)-OH und H-Val-OtBu erhält man das Z-Asp(OtBu)-Val-OtBu, Smp. 75°C (n-Hexan), Ausbeute: 93%.

b) Durch Hydrogenolyse des Produkts von a) erhält man H-Asp(OtBu)-Val-OtBu, Smp. 71° (n-Hexan), Ausbeute: 93%.

c) Durch Kupplung von Z-Gly-OSu und H-Asp(OtBu)-Val-OtBu erhält man das Z-Gly-Asp(OtBu)-Val-OtBu, Smp. 132°C (Essigester), Ausbeute: 87%.

d) Durch Hydrogenolyse des Produkts von c) erhält man H-Gly-Asp(OtBu)-Val-OtBu, Ausbeute: 87% d.Th., $[\alpha]_D$ -33,2° (c 0,6, MeOH).

e) Durch Kupplung von rac N-Boc-3-(p-cyanophenyl)alanin (französische Offenlegungsschrift 2593 814) und H-Gly--Asp(OtBu)-Val-OtBu erhält man das [N-Boc-3-(p-Cyanophenyl)--DL-alanyl]-Gly-Asp(OtBu)-Val-OtBu, Smp. 140-145°C aus Essigester/Isopropyläther, Ausbeute: 66%.

f) Analog zu Beispiel 1 B) f), g), h) erhält man aus voranstehender Verbindung über [N-Boc-3-(p-Thiocarboxamidophenyl) -DL-alanyl]-Gly-Asp(OtBu)-Val-OtBu (Epimerengemisch 1:1), Ausbeute: 96%, MS: 708 $(M+H)^+$

und über [N-Boc-3-(p-Methylthiocarboximidophenyl) -DL-alanyl]-Gly-Asp(OtBu)-Val-OtBu-Hydrojodid (Epimerengemisch 1:1), Smp. 100°C (Zers.), Ausbeute: 82%

das [N-Boc-3-(p-Amidinophenyl)-DL-alanyl]-Gly-Asp(OtBu)-Val-OtBu-Hydrojodid-Acetat (Epimerengemisch 1:1), Smp. 154-156°C (Zers.) (Essigester), Ausbeute: 89%.

Beispiel 3

A) 95 mg [N-Boc-3-(p-Guanidinophenyl)-L-alanyl] -Gly-Asp(OtBu)-Val-OtBu werden in 10 ml Essigester gelöst und mit 5 ml 2,5N HCl in Essigester versetzt. Nach 4 Stunden Rühren bei Raumtemperatur wird filtriert und der Niederschlag mit Essigester gewaschen. Man erhält 44 mg (53%) [3-(p-Guanidinophenyl)-L-alanyl] -Gly-Asp-Val-OH-Pentahydrochlorid, Smp. 215°C (Zers.) aus Dioxan.

B) Das Ausgangsmaterial kann auf folgende Weise hergestellt werden:

a) Durch Kondensation von N-Boc-3-(p-Nitrophenyl)-L-alanin und H-Gly-Asp(OtBu)-Val-OtBu erhält man [N-Boc-3-(p-Nitrophenyl)-L-alanyl]-Gly-Asp(OtBu)-Val-OtBu, Smp. 106°C (n-Hexan). Ausbeute: 72%.

b) Eine Lösung von 890 mg des Produkts von a) in 15 ml Methanol wird 3 Stunden in Gegenwart von 200 mg 10% Pd/C hydriert. Der nach Filtration und Verdampfen des Lösungsmittels zurückbleibende Schaum wird an Kieselgel mit Essigester-Methanol (95:5) chromatographiert und aus Isopropyläther kristallisiert. Man erhält 670 mg (79%) [N-Boc-3--(p-Aminophenyl)-L-alanyl]-Gly-Asp(OtBu)-Val-OtBu, Smp. 110-112°C.

c) Eine Lösung von 200 mg des Produkts von b) und 60 mg 3,5-Dimethyl-N-nitro-1H-pyrazol-1-carboxamidin in 3 ml Aethanol wird 24 Stunden unter Rückfluss erhitzt. Das Lösungsmittel wird abgedampft und der Rückstand an Kieselgel mit Methylenchlorid/Methanol (98:2) chromatographiert. Nach dem Umkristallisieren aus Essigester/n-Hexan erhält man 148 mg (66%) [N-Boc-3-[p-(3-Nitroguanidino)phenyl] -L-alanyl]-Gly-Asp(OtBu)-Val-OtBu, Smp. 141-143°C.

d) Eine Lösung von 118 mg der Vorstufe in 3 ml Essigsäure wird in Gegenwart von 30 mg Pd/C 3 Tage hydriert. Nach Entfernen des Lösungsmittels wird das Filtrat an Kieselgel mit Essigester/Methanol (99:1) chromatographiert. Man erhält 59 mg (54%) [N-Boc-3-(p-Guanidinophenyl)-L-alanyl]-Gly-Asp(OtBu)-Val-OtBu, MS: 706 $(M+H)^+$.

Beispiel 4

A) Analog wie in Beispiel 3A beschrieben erhält man durch saure Hydrolyse von [N-Boc-3-(p-Amidinophenyl) -DL-alanyl]-Gly-Asp(OtBu)-isobutylamid-Hydrojodid, das [3-(p-Amidinophenyl)-DL-alanyl]-Gly-Asp -isobutylamid-Hydrochlorid (Epimerengemisch 1:1), Smp. 195-198°C (Zers.) aus Dioxan, Ausbeute: quantitativ.

B) Das Ausgangsmaterial kann wie folgt hergestellt werden:

a) Zu einer bei -10°C hergestellten Mischung aus 5,12 g Z-Asp(OtBu)-OH-Hydrat, 1,65 ml N-Methylmorpholin und 2 ml Chlorameisensäureisobutylester in 15 ml THF werden 5,5 g Isobutylamin, gelöst in 5 ml THF, zugetropft. Nach 3 Stunden wird vom Lösungsmittel befreit, der Rückstand in Essigester/Natriumbicarbonat 5% aufgenommen und die organische Phase mit Wasser neutral gewaschen. Nach dem Trocknen, Eindampfen des Lösungsmittels und Chromatographie des erhaltenen Oels an Kieselgel mit Essigester erhält man 4,53 g (80%) Z-Asp(OtBu)-isobutylamid, Smp. 69-70°C aus n-Hexan.

b) Durch Hydrogenolyse des Produkts von a) erhält man das H-Asp(OtBu)-isobutylamid, Ausbeute: 97%, MS: 189, 171.

c) Durch Kupplung von Z-Gly-OH mit dem Produkt von b) erhält man das Z-Gly-Asp(OtBu)-isobutylamid, Ausbeute: 87%, MS: 436 $(M+H)^+$.

d) Durch Hydrogenolyse der Vorstufe erhält man das H-Gly-Asp(OtBu)-isobutylamid, Ausbeute: 42%, MS: 302 $(M+H)^+$.

e) Durch Kupplung von rac N-Boc-3-(p-cyanophenyl)alanin und H-Gly-Asp(OtBu)-isobutylamid erhält man das [N-Boc-3--(p-cyanophenyl)-DL-alanyl]-Gly-Asp(OtBu)-isobutylamid (1:1 Gemisch von Epimeren), Smp. 117-119°C (Essigester/n-Hexan), Ausbeute: 27%.

f) Analog Beispiel 1Bf)g)h) erhält man über das [N-Boc--3-(p-Thiocarboxamidophenyl) -DL-alanyl]-Gly-Asp (OtBu)--isobutylamid (Epimerengemisch 1:1), und über das [N-Boc--3-(p-Methylthiocarboximidophenyl) -DL-alanyl]-Gly--Asp(OtBu)-isobutylamid-Hydrojodid (Epimerengemisch 1:1), Smp. 145-147°C (Zers.), Ausbeute: 72%
das [N-Boc-3-(p-Amidinophenyl) -DL-alanyl]-Gly--Asp(OtBu)-isobutylamid-Hydrojodid (Epimerengemisch 1:1), Smp. 175-178°C, Ausbeute: 60%.

Beispiel 5

A) Analog zu Beispiel 1 A) erhält man bei Einsatz von [N-Z-3-(p-Amidinophenyl) -DL-alanyl]-Gly-Asp(OtBu)-isobutyl-amid das [N-Z-3-(p-Amidinophenyl) -DL-alanyl]-Gly-Asp-iso-butylamid-Trifluoracetat (Epimerengemisch 1: 1), Smp. 141-143°C (Aether), Ausbeute: 91%.

B) Das Ausgangsmaterial kann man auf folgende Weise herstellen:

a) Durch Kupplung von rac N-Z-3-(p-Cyanophenyl)alanin und H-Gly-Asp(OtBu)-isobutylamid erhält man das [N-Z-3-(p-Cyanophenyl)-DL-alanyl]-Gly-Asp(OtBu)-isobutylamid (Epimerengemisch 1:1), Smp. 121-123°C (Essigester/n-Hexan), Ausbeute: 91%.

b) Analog Beispiel 1B)f)g)h) erhält man über das [N-Z-3--(p-Thiocarboxamidophenyl) -DL-alanyl]-Gly-Asp(OtBu)-isobutylamid (Epimerengemisch 1:1), und über das [N-Z-3--(p-Methylthiocarboximidophenyl) -DL-alanyl]-Gly-Asp(OtBu)--isobutylamid-Hydrojodid (Epimerengemisch 1:1) das [N-Z-3-(p-Amidinophenyl) -DL-alanyl]-Gly-Asp(OtBu)-isobutylamid-Hydrojodid (Epimerengemisch 1:1), Smp. 160-163°C, Ausbeute: 37%.

Beispiel 6

Eine Lösung von 400 mg H-Arg-Gly-Asp-Val-OH (Proc. Natl. Acad. Sci. USA, 82, 1985, 8057) und 143 mg Pyridin•HBr in 15 ml DMF wird mit 435 mg Z-Aeg(Z)-OSu versetzt. Mit N-Methylmorpholin wird das Reaktionsgemisch auf pH 8,5 eingestellt, über Nacht gerührt und anschliessend eingedampft. Der Rückstand wird in 0,2N Essigsäure gelöst und an einem Polysaccharidharz (Sephadex G-10) mit 0,2N Essigsäure chromatographiert. Die einheitlichen Fraktionen werden vereinigt und lyophilisiert. Eine wässrige Lösung des Lyophilisats wird an einem Polystyrolharz in Acetatform (Dowex 44) chromatographiert. Das Eluat wird lyophilisiert. Man erhält 143 mg Z-Aeg(Z)-Arg-Gly-Asp-Val-OH; MS: 814 $(M+H)^+$.

Beispiel 7

Analog Beispiel 1Bb) wird eine Lösung von 120 mg Z-Aeg(Z)-Arg-Gly-Asp-Val-OH (Beispiel 6) in 20 ml 0,1N Essigsäure in Gegenwart von Pd/C hydriert. Der Katalysator wird abfiltriert und das Filtrat lyophilisiert. Man erhält 72 mg Aeg-Arg-Gly-Asp-Val-OH·Acetat (1:1); MS: 546 $(M+H)^+$.

Beispiel 8

Eine Lösung von 216 mg H-Arg-Gly-Asp-Ser-OH (US-Patent Nr. 4578079) in 5 ml DMF und 5 ml $H_2O$ wird mit 242 mg Z-Aeg(Z)-OSu und 0,11 ml N-Methylmorpholin versetzt. Das Reaktionsgemisch wird 18 Stunden gerührt und dann mit Essigsäure auf pH 5,3 eingestellt. Mit Essigester wird das Reaktionsgemisch extrahiert. Die wässrige Phase wird mit Pd/C versetzt und 2 Stunden hydriert. Der Katalysator wird abgenutscht und das Filtrat lyophilisiert. Das Lyophilisat wird in 0,2N Essigsäure gelöst und an einem Polysaccharidharz (Sephadex G 25S) mit 0,2N Essigsäure chromatographiert. Die vereinigten einheitlichen Fraktionen werden lyophilisiert. Man erhält 150 mg Aeg-Arg-Gly-Asp-Ser-OH·Acetat (1:2), MS: 534 $(M+H)^+$.

Beispiel 9

Eine Lösung von 237,5 mg H-Arg-Gly-Asp-Val-OH in 5 ml Aceton und 5 ml $H_2O$ wird nacheinander mit 226 mg Naphthalin-2-sulfonylchlorid und 168 mg $NaHCO_3$ versetzt. Nach 2 Stunden Rühren wird mit Essigsäure angesäuert und das Aceton abdestilliert. Der wässrige Rückstand wird an Sephadex G-25S mit 0,2N Essigsäure chromatographiert. Die vereinigten einheitlichen Fraktionen werden lyophilisiert. Man erhält 172 mg (2-Naphthylsulfonyl)-Arg-Gly-Asp-Val-OH; MS: 636 $(M+H)^+$.

Beispiel 10

Eine Suspension von 3 g eines Trägers bestehend aus einem p-Benzyloxybenzylalkohol-Reste enthaltenden Styrol-1% Divinylbenzol-Harz in 30 ml DMF wird nacheinander mit 0,6 g Fmoc-Nal(1)-OH (europäische Patentanmeldung 128762), 523 mg HBTU, 16,8 mg 4-Dimethylaminopyridin und 0,24 ml DIPEA versetzt. Das Reaktionsgemisch wird 24 Stunden geschüttelt, anschliessend wird das Harz abgenutscht und mit DMF gewaschen. Die freien Hydroxygruppen werden mit 1,13 ml Essigsäureanhydrid, 2,05 ml N-Aethyldiisopropylamin in 30 ml DMF während 30 Minuten acetyliert. Im folgenden Protokoll ist ein Synthesezyklus beschrieben:

| Stufe | Reagenz | Zeit |
|---|---|---|
| 1 | DMF | 2 x 1 Min. |
| 2 | 20% Piperidin/DMF | 1 x 7 Min. |
| 3 | DMF | 5 x 1 Min. |
| 4 | 2,5 Aeq. Fmoc-Aminosäure/DMF<br>+ 2,5 Aeq. HBTU<br>+ 2,5 Aeq. N-Aethyldiisopropylamin | 1 x 90 Min. |
| 5 | DMF | 3 x 1 Min. |
| 6 | Isopropylalkohol | 2 x 1 Min. |

30 ml Lösungsmittel werden in jeder Stufe benutzt. Fmoc-Asp(OtBu)-OH, Fmoc-Gly-OH, Fmoc-Arg(HCl)-OH werden nach obigem Protokoll gekuppelt. Boc-Aeg(Boc)-OSu wird in die Peptidkette eingeführt. Nach beendeter Synthese wird das Peptidharz getrocknet und halbiert. Es wird in 10 ml TFA/5 ml $CH_2Cl_2$ und 1 ml $H_2O$ suspendiert und 90 Minuten geschüttelt. Das Harz wird abfiltriert und das Filtrat eingeengt. Der Rückstand wird aus $H_2O$ lyophilisiert. Das Lyophilisat wird an einer Sephadex G-25S in 0,2N Essigsäure chromatogrpahiert. Die vereinigten einheitlichen Fraktionen werden lyophilisiert. das Lyophilisat wird an Dowex 44 chromatographiert. Das Eluat wird lyophilisiert. Man erhält 49 mg N-Aeg-Arg-Gly-Asp-Nal(1)-OH•Acetat (1:1), MS: 644 $(M+H)^+$.

Beispiel 11

Analog Beispiel 10 erhält man ausgehend von 1,05 g Fmoc-Ile-OH 73,5 mg Aeg-Arg-Gly-Asp-Ile-OH•TFA (1:1), MS: 560 $(M+H)^+$.

Beispiel 12

A) Eine Lösung von 230 mg Boc-Lys(Z)-Gly-Asp(OBzl)-Val-OBzl in 10 ml Methanol wird in Gegenwart von Pd/C hydriert. Der Katalysator wird abfiltriert und das Filtrat eingeengt. Der Rückstand wird in 2 ml $H_2O$ gelöst, mit 2N NaOH auf pH 9,5 eingestellt und mit 109 mg Methylacetimidat•HCl versetzt. Der pH-Wert wird erneut auf 9,5 eingestellt. Nach 90 Minuten Rühren wird das Reaktionsgemisch mit 1 N HCl auf pH 4 angesäuert und an Sephadex G-25S mit 0,2N Essigsäure chromatographiert. Die einheitlichen Fraktionen werden vereinigt und lyophilisiert. Man erhält 72 mg [$N^2$-Boc-$N^6$-(1-Iminoäthyl)-L-lysyl]-Gly-Asp-Val-OH, MS: 559 $(M+H)^+$.

B) Das Ausgangsmaterial wird wie folgt hergestellt:
Eine Lösung von 583 mg H-Gly-Asp(OBzl)-Val-OBzl (Beispiel 15Bb) und 477,5 mg Boc-Lys(Z)-OSu in 10 ml DMF wird mit N-Methylmorpholin auf pH 8,5 eingestellt. Nach 18-stündigem Rühren wird eingeengt und der Rückstand zwischen Essigester und Wasser verteilt. Die organische Phase wird mit gesättigter $NaHCO_3$-Lösung, 5% $KHSO_4$/10% $K_2SO_4$-Lösung und gesättigter NaCl-Lösung gewaschen, getrocknet und filtriert. Das Filtrat wird eingeengt und der Rückstand aus Aether kristallisiert. Man erhält 385 mg Boc-Lys(Z)-Gly-Asp(OBzl)-Val-OBzl, Smp. 95-101°C.

Beispiel 13

A) 370 mg o-[Z-Arg($Z_2$)-Gly-Asp(OtBu)-NH]-benzoesäure werden in 50 ml Methanol gelöst und in Gegenwart von Pd/C hydriert. Das Filtrat vom Katalysator wird im Vakuum eingeengt, der Rückstand in 50 ml DMF gelöst und mit 46 mg Pyridin•HCl und 0,07 ml DIPEA versetzt. In 100 ml DMF werden 203 mg HOBTU vorgelegt und die obige Lösung wird unter Argon zugetropft. Nach 20 Stunden Rühren werden weitere 101,5 mg HOBTU und 0,035 ml DIPEA hinzugefügt. Das Reaktionsgemisch wird 18 Stunden gerührt und eingeengt. Der Rückstand wird in Methanol/Wasser gelöst und an Dowex 44 chromatographiert. Das Eluat wird eingeengt, der Rückstand in 20 ml TFA gelöst und nach 30 Minuten eingeengt. Nach Chromatographie an einem chemisch modifizierten Kieselgel (Lichrosorb RP18) mit 0,1% TFA-Aethanol erhält man 102 mg N-[(o-Azidobenzoyl)-Arg-Gly-Asp]-anthranilsäure-Trifluoracetat (1:1), MS: 611 $(M+H)^+$.

B) Die Ausgangssäure wird wie folgt hergestellt:

a) Eine Lösung von 1,6 g Z-Asp(OtBu)-OH und 2 g Anthranilsäurebenzylester-Tosylat in 10 ml DMF wird mit 1,92 g TOBTU und 1,78 ml DIPEA versetzt. Nach 20 Stunden Rühren wird das Reaktionsgemisch zwischen Aethylacetat und Wasser verteilt. Die organische Phase wird mit 5%iger $KHSO_4$/10% $K_2SO_4$ Lösung, Wasser, gesättigter $NaHCO_3$-Lösung, Wasser und gesättigter NaCl-Lösung gewaschen und über $Na_2SO_4$ getrocknet. Das Trockenmittel wird abfiltriert und das Filtrat eingeengt. Nach Kristallisation aus Aethanol erhält man 0,75 g N-[Z-Asp(OtBu)]-anthranilsäure, Smp. 123-124°C.

b) Analog Beispiel 7 erhält man durch Hydrierung des Produkts von a) 401 mg N-[H-Asp(OtBu)]-anthranilsäure.

c) Eine Suspension von 401 mg des Produkts von b) in 10 ml DMF wird mit 612 mg Z-Gly-OSu versetzt. Mit

N-Methylmorpholin wird das Reaktionsgemisch auf pH 8,5 eingestellt und 4 Stunden gerührt. Die Reaktionslösung wird mit 0,52 ml Diäthylaminoäthylamin versetzt, 10 Minuten gerührt und dann zwischen Essigester und 5% $KHSO_4$/10% $K_2SO_4$-Lösung verteilt. Die organische Phase wird mit gesättigter Kochsalzlösung gewaschen und getrocknet. Nach Filtration wird das Filtrat eingeengt. Der Rückstand wird in Methanol gelöst und in Gegenwart von Pd/C hydriert. Der Katalysator wird abfiltriert und das Filtrat eingeengt. Der Rückstand wird in 10 ml DMF gelöst, mit 673 mg Z-Arg($Z_2$)-OSu versetzt und mit N-Methylmorpholin auf pH 8,5 eingestellt. Nach 18 Stunden Rühren wird das Produkt durch Eingiessen in 5% $KHSO_4$/10% $K_2SO_4$-Lösung ausgefällt. Durch Chromatographie an Kieselgel mit Methylenchlorid/Methanol erhält man nach Umkristallisieren aus Aethanol 410 mg o-[Z-Arg($Z_2$)--Gly-Asp(OtBu)-NH]-benzoesäure, Smp. 102-103°C.

Beispiel 14

A) Eine Lösung von 109 mg Boc-Arg-Gly-Asp-Val-OH in 3 ml 0,2M Natriumboratpuffer (pH 9) wird mit 55,5 mg 1,2-Cyclohexandion versetzt und dann 24 Stunden unter Argon gerührt. Die Reaktionslösung wird mit Essigsäure auf pH 4 angesäuert und an Sephadex G-25S mit 0,2N Essigsäure chromatographiert. Die einheitlichen Fraktionen werden vereinigt und lyophilisiert. Man erhält 46 mg [$N^2$-Boc-$N^5$-(3a,4,5,6,7,7a-Hexahydro-3a,7a-dihydroxy-1H-benzimidazol-2-yl)-L-ornithyl]--Gly-Asp-Val-OH, MS: 658 $(M+H)^+$.

B) Zur Herstellung der Ausgangssäure wird eine Lösung von 1 g H-Arg-Gly-Asp-Val-OH und 340 mg Pyridin•HBr in 15 ml Dioxan und 15 ml $H_2O$ nacheinander mit 480 mg Di-t-butyldicarbonat und 0,59 g $NaHCO_3$ versetzt. Nach 18 Stunden Rühren wird das Reaktionsgemisch eingeengt. Der Rückstand wird an einem porösen Styrol-divinylbenzol-copolymer-Harz (MCI-Gel CHP20P) mit Wasser/Aethanol gereinigt. Die vereinigten einheitlichen Fraktionen werden eingeengt und aus Wasser lyophilisiert. Man erhält 260 mg Boc-Arg-Gly-Asp-Val-OH, MS: 546 $(M+H)^+$.

Beispiel 15

A) Eine Lösung von 275,5 mg N-Boc-3-(p-Aminophenyl)-DL--alanyl-Gly-Asp-Val-OH in 3 ml Wasser wird nacheinander mit 138 mg $K_2CO_3$ und 124 mg Aminoiminomethansulfonsäure versetzt. Nach 18-stündigem Rühren wird das Reaktionsgemisch mit Eisessig angesäuert und an Sephadex G-25S mit 0,2N Essigsäure chromatographiert. Die einheitlichen Fraktionen werden vereinigt und lyophilisiert. Man erhält 205 mg [N-Boc-3-(p-Guanidinophenyl)-DL-alanyl]-Gly-Asp-Val-OH -Kaliumsalz (1:1), MS: 632 $(M+H)^+$.

B) Das Ausgangsmaterial wird wie folgt hergestellt:

a) Eine auf -20°C gekühlte Lösung von 32,3 g Boc-Asp(OBzl)--OH in 150 ml DMF wird mit 11 ml N-Methylmorpholin und 13,07 ml Chlorameisensäureisobutylester versetzt. Die erhaltene Suspension wird bei -15°C gerührt und mit einer auf -20°C gekühlten Suspension von 37,95 g H-Val-OBzl•Tosylat und 11 ml N-Methylmorpholin in 150 ml DMF versetzt. Die Reaktionsmischung wird 10 Minuten unterhalb -10°C und 2 Stunden bei Raumtemperatur gerührt, filtriert und das Filtrat wird eingeengt. Der Rückstand wird in Aethylacetat gelöst und mit 5%iger $KHSO_4$/10%iger $K_2SO_4$-Lösung, Wasser, gesättigter $NaHCO_3$-Lösung, Wasser und gesättigter NaCl-Lösung gewaschen und getrocknet. Die organische Phase wird eingeengt. Man erhält 52,9 g Boc-Asp (OBzl)-Val-OBzl. 10,24 g davon werden in 30 ml TFA gelöst und dann eingeengt. Der Rückstand wird aus Aethylacetat/Hexan kristallisiert. Man erhält 8,3 g H-Asp(OBzl)-Val-OBzl•TFA (1:1), Smp. 147-148°C.

b) Eine Lösung von 4,2 g H-Asp(OBzl)-Val-OBzl•TFA in 50 ml Aethylacetat wird nacheinander mit 2,72 g Boc-Gly-OSu und 0,88 ml N-Methylmorpholin versetzt und dann 72 Stunden bei 20°C gerührt. Das Reaktionsgemisch wird zwischen Aethylacetat und Wasser verteilt und die organische Phase mit 5% $KHSO_4$/10% $K_2SO_4$-Lösung, Wasser, gesättigter $NaHCO_3$--Lösung, Wasser und gesättigter NaCl-Lösung gewaschen. Nach dem Trocknen wird eingeengt. Der Rückstand wird in 30 ml Trifluoressigsäure gelöst, 20 Minuten bei 20°C gehalten und dann eingeengt. Nach Kristallisieren aus Aethylacetat/Hexan erhält man 3,5 g H-Gly-Asp(OBzl)-Val-OBzl, TFA (1:1), Smp. 150-151°C.

c) Eine Lösung von 1,08 g rac N-Boc-3-(4-Nitrophenyl)--alanin und 2,04 g H-Gly-Asp(OBzl)-Val-OBzl•TFA in 15 ml DMF wird nacheinander mit 1,4 g HBTU und 1,23 ml N-Aethyldiisopropylamin versetzt. Nach 3 Stunden Rühren wird das Reaktionsgemisch zwischen Aethylacetat und Wasser verteilt. Der Ansatz wird aufgearbeitet

wie unter b) beschrieben und man erhält nach Kristallisieren aus Aethylacetat/Hexan 1,8 g N-Boc-3-(4-Nitrophenyl)-DL-alanyl-Gly-Asp(OBzl)-Val-OBzl, Smp. 155-157°C.

d) Eine Lösung von 1,5 g N-Boc-3-(4-Nitrophenyl)-DL-alanyl-Gly-Asp(OBzl)-Val-OBzl in 50 ml 90%igem Eisessig wird in Gegenwart von 10% Pd/C hydriert. Das Filtrat vom Katalysator wird aus Wasser lyophilisiert. Man erhält 910 mg N-Boc-3-(4-Aminophenyl)-DL-alanyl-Gly-Asp-Val-OH, MS: 552 $(M+H)^+$.

Beispiel 16

A) Analog Beispiel 1A) erhält man durch Acidolyse von [N-Boc-3-(p-Amidinophenyl)-DL-alanyl]-Gly-Asp(OtBu)-Nal(1)-OH das [3-(p-Amidinophenyl)-DL-alanyl]-Gly-Asp-Nal(1)-OH•TFA (1:2) (1:1 Epimerengemisch), Smp. 170°C (Zers.) (Aethanol/Essigester).

B) Das Ausgangsmaterial wird auf folgende Weise hergestellt:

a) Analog Beispiel 1 B)a) erhält man durch Kupplung von Z-Gly-OH und H-Asp(OtBu)-OMe das Z-Gly-Asp(OtBu)-OMe, Smp. 92-95°C (Hexan), Ausbeute: 89% d.Th.

b) Einer Lösung von 27,0 g der Vorstufe in 200 ml Aceton werden unter Kühlung 70 ml 1N NaOH zugetropft und das Rühren wird 2 Stunden fortgesetzt. Durch Zugabe von 10%iger wässriger Zitronensäure wird das pH auf 4 eingestellt und das Lösungsmittel abgedampft, wobei das Rohprodukt ausfällt. Nach dem Umkristallisieren aus Aether/Hexan erhält man 19,04 g Z-Gly-Asp(OtBu)-OH, Smp. 101-104°C, Ausbeute: 70%.

c) Analog Beispiel 1 B)a) erhält man durch Kupplung der Vorstufe mit H-Nal(1)-OMe das Z-Gly-Asp(OtBu)-Nal(1)-OMe, Smp. 59°C (Hexan), Ausbeute: 34%.

d) Analog Beispiel 1 B)b) erhält man durch Hydrogenolyse der Vorstufe das H-Gly-Asp(OtBu)-Nal(1)-OMe, Smp. 67-68°C (Hexan), Ausbeute: 72%.

e) Analog Beispiel 1 B)e) erhält man durch Kupplung von N-Boc-3-(p-cyanophenyl)-DL-alanin mit der Vorstufe das [N-Boc-3-(p-cyanophenyl)-DL-alanyl]-Gly-Asp(OtBu)-Nal(1)-OMe (Epimere), Ausbeute: quantitativ, MS: 730 $(M+H)^+$.

f) Analog Beispiel 1 B)f) erhält man durch Thionierung der Vorstufe das [N-Boc-3-[p-(thiocarbamoyl)phenyl]-DL-alanyl]Gly-Asp(OtBu)-Nal(1)-OMe (Epimere), Smp. 110-112°C, Ausbeute: 75%.

g) Analog Beispiel 1 B)g) erhält man durch Methylierung der Vorstufe das [N-Boc-3-[p-[(Methylthio)formimidoyl]phenyl]-DL-alanyl]-Gly-Asp(OtBu)-Na1(1)-OMe-Hydrojodid (Epimere), Smp. 128-130°C (Aether), Ausbeute: 64%.

h) Analog zu Beispiel 1 B)h) erhält man durch Reaktion der Vorstufe mit $NH_4OAc$ das [N-Boc-3-(p-Amidinophenyl)-DL-alanyl]-Gly-Asp(OtBu)-Nal(1)-OMe-Hydrojodid (Epimere), Smp. 139-141°C (Aether), Ausbeute: 70%.

i) Wie beschrieben in Absatz b) erhält man aus der Vorstufe das [N-Boc-3-(p-Amidinophenyl)-DL-alanyl]-Gly-Asp(OtBu)--Nal(l)-OH (Epimere), Smp. 206°C (Essigester), Ausbeute: 97%.

Beispiel 17

Zu einer Lösung von 70 mg [3-(p-Amidinophenyl)-DL--alanyl]-Gly-Asp-Val-OH-Trifluoracetat (1:2) (1:1 Gemisch der Epimeren) (Beispiel 2) in 0,6 ml DMF werden bei Raumtemperatur unter Begasung mit Argon 30 mg Triäthylamin und 24 mg Di-t-Butyldicarbonat addiert und die Mischung wird 75 Minuten gerührt. Nach Zugabe von Essigsäure bis pH 4 wird zur Trockene verdampft und der Rückstand wird mit Essigester zur Kristallisation gebracht. Nach Umkristallisieren aus Methanol/Essigester erhält man das [N-Boc-3-(p-Amidinophenyl)-DL-alanyl]-Gly-Asp-Val-OH (1:1 Epimere), Ausbeute: 63%.

Beispiel 18

A) Analog Beispiel 1A erhält man bei Einsatz von [N-Boc-3-(p-amidinophenyl)-DL-alanyl]-Gly-Asp(OtBu)-p-fluorphenäthylamid-Hydrojodid (1:1 Epimere) das 3-(p-Amidinophenyl)-DL-alanyl-Gly-Asp-p-fluorphenäthylamid-Trifluoracetat (5:8)(Epimere), Smp. 192-195°C (Aethanol/Essigester), Ausbeute: 61%.

B) Der Ausgangsester wird wie folgt hergestellt:

a) Analog Beispiel 1 B)e) durch Kupplung von Z-Gly-Asp(OtBu)-OH (Beispiel 16 B)b)) und 4-Fluorphenäthylamin mit HBTU erhält man das Z-Gly-Asp(OtBu)-p-fluorphenäthylamid, MS: 502 $(M+H)^+$.

b) Analog Beispiel 1 B)b) durch katalytische Hydrogenolyse der Vorstufe erhält man das H-Gly-Asp(OtBu)-p-fluorphenäthylamid, MS: 368 $(M+H)^+$.

c) Analog Beispiel 1 B)e) erhält man durch Kupplung von rac N-Boc-3-(p-Cyanophenyl)alanin mit der Vorstufe das [N-Boc-3-(p-cyanophenyl)-DL-alanyl]-Gly-Asp(OtBu)-p-fluorphenäthylamid (1:1 Epimere), MS: 662 $(M+Na^+)$.

d) Analog Beispiel 1 B)f) erhält man durch Reaktion der Vorstufe mit $H_2S$ das [N-Boc-3-[p-(thiocarbamoyl) phenyl]-DL-alanyl]-Gly-Asp(OtBu)-p-fluorphenäthylamid (1:1 Epimere), MS: 674 $(M+H)^+$.

e) Analog Beispiel 1 B)g) erhält man durch Reaktion der Vorstufe mit MeJ das [N-Boc-3-[p-(methylthioformimidoyl)-phenyl]-DL-alanyl]-Gly-Asp(OtBu)-p-fluorphenäthylamid-Hydrojodid (1:1 Epimere), Smp. 134-136°C (Zers.) (Aether).

f) Analog Beispiel 1 B)h) erhält man durch Reaktion der Vorstufe mit Ammoniumacetat das [N-Boc-3-(p-amidinophenyl)-DL-alanyl]-Gly-Asp(OtBu)-p-fluorphenäthylamid-Hydrojodid (1:1 Epimere), Smp. 90-92°C (Zers.) aus Aether.

Beispiel 19

A) Analog Beispiel 1A) erhält man bei Einsatz von (p-Amidinohydrocinnamoyl)-Gly-Asp(OtBu)-Nal(1)-OH das (p-Amidinohydrocinnamoyl)-Gly-Asp-Nal(1)-OH-Trifluoracetat (1:1), Smp. 196-199°C (Aethanol/Aether), Ausbeute: 48%.

B) Der Ausgangsester wird wie folgt hergestellt:

a) Analog Beispiel 1 B)e) erhält man durch Kupplung von 3-(p-Cyanophenyl)propionsäure und H-Gly-Asp (OtBu)-Nal(1)-OMe (Beispiel 16 B)d)) das (p-Cyanohydrocinnamoyl)-Gly--Asp(OtBu)-Nal(1)-OMe, Smp. 112-113°C ($CH_2Cl_2$/Hexan).

b) Analog zu Beispiel 1 B)f)g)h) erhält man durch Reaktion der Vorstufe nacheinander mit $H_2S$, MeJ und Ammoniumacetat das (p-Amidinohydrocinnamoyl)-Gly-Asp(OtBu)-Nal(1)-OMe--Hydrojodid, Smp. 140-142°C (Aether).

c) Analog zu Beispiel 16 B)b) erhält man durch alkalische Verseifung der Vorstufe das (p-Amidinohydrocinnamoyl)-Gly--Asp(OtBu)-Nal(l)-OH, Smp. 236-237°C (Wasser).

Beispiel 20

Analog Beispiel 1A) erhält man bei Einsatz von [N-Boc-3-(p-Amidinophenyl)-D-alanyl]-Gly-Asp(OtBu)-Val-OtBu-Hydrojodid das [3-(p-Amidinophenyl)-D-alanyl]-Gly-Asp-Val-OH-Trifluoracetat (1:2), Smp. 128°C (Zers.) (Aether), Ausbeute: quantitativ.

Beispiel 21

A) Analog Beispiel 16 B)b) erhält man durch Verseifung von [N-[[N-Boc-3-(p-Amidinophenyl)-DL-alanyl]-Gly-Asp(OtBu)]-p-amino]benzoesäuremethylester-Hydrojodid (Epimere 1:1) die [N-[[N-Boc-3-(p-Amidinophenyl)-DL-alanyl]-Gly-Asp]-p-amino]-benzoesäure (1:1 Epimere), Smp. 205-206°C (Methanol), Ausbeute: 48%.

B) Das Ausgangsmaterial wird auf folgende Weise hergestellt:

a) Analog Beispiel 1 B)a) erhält man durch Kupplung von Z-Gly-Asp(OtBu)-OH und p-Aminobenzoesäurebenzylester den [N-[Z-Gly-Asp(OtBu)]-p-amino]benzoesäurebenzylester. Ausbeute: 32%, MS: 590 $(M+H)^+$.

b) Analog Beispiel 1 B)b) erhält man durch Hydrogenolyse der Vorstufe die [N-[Gly-Asp(OtBu)]-p-amino]benzoesäure, Smp. 160°C (Zers.) aus Methanol/AcOEt.

c) Aus der Vorstufe erhält man durch Methylierung mit Diazomethan den [N-[Gly-Asp(OtBu)]-p-amino]benzoesäuremethylester, Smp. 78-82°C (Hexan), Ausbeute: 77%.

d) Analog Beispiel 1 B)e) erhält man durch Kupplung von rac N-Boc-3-(4-Cyanophenyl)alanin und der Vorstufe den [N-[[N-Boc-3-(p-Cyanophenyl)-DL-alanyl]-Gly-Asp(OtBu)]-p-amino]benzoesäuremethylester (1:1 Gemisch von Epimeren), Smp. 108°C (Essigester/Hexan), Ausbeute: 51%.

e) Analog Beispiel 1 B)f)g) erhält man aus der Vorstufe durch Thionierung und Methylierung das [N-[[N-Boc-3-[p-(Methylthioformimidoyl)phenyl]-DL-alanyl]-GLy-Asp(OtBu)]-p-amino]benzoesäuremethylester-Hydrojodid (Epimere 1:1), Smp. 150-151°C (Aether), Ausbeute: 77%.

f) Analog Beispiel 1 B)h) erhält man aus der Vorstufe durch Ammonolyse das [N-[[N-Boc-3-(p-Amidinophenyl)-DL-alanyl]-Gly-Asp(OtBu)]-p-amino]benzoesäuremethylester-Hydrojodid (Epimere 1:1), Smp. 179-181°C (Zers.) (Aether), Ausbeute: 79%.

Beispiel 22

Analog Beispiel 1A) erhält man durch Hydrolyse des Produkts von Beispiel 21 die [N-[[3-(p-Amidinophenyl)-DL-alanyl]-Gly-Asp]-p-amino]benzoesäure (Epimere 1:1), Smp. 214-216°C (MeOH), Ausbeute: 78%.

Beispiel 23

A) Eine Lösung von 180 mg (p-Cyanohydrocinnamoyl)-Gly-Asp--Val-OH in einer Mischung von 10 ml Methanol/konz. wässrige Ammoniaklösung (2:1) wird in Gegenwart von 180 mg Raney--Nickel hydriert. Nach 20 Stunden wird vom Katalysator filtriert und zur Trockene verdampft. Der Rückstand wird an einem Ionenaustauscherharz in $H^+$-Form gereinigt und mit Hexan zur Kristallisation gebracht. Man erhält das (p-Amino-methylhydrocinnamoyl)-Gly-Asp-Val-OH, Smp. 175°C (Zers.), Ausbeute: 25%.

B) Das Ausgangsnitril, Smp. 132-134°C (Essigester/Hexan) wird in Analogie zu Beispiel 1A) hergestellt (Ausbeute 69%) durch Acidolyse des (p-Cyanohydrocinnamoyl)-Gly-Asp(OtBu)--Val-OtBu (Beispiel 24 B)a).

Beispiel 24

A) Analog Beispiel 19A) erhält man aus (p-Amidinohydrocinnamoyl)-Gly-Asp(OtBu)-Val-OtBu-Hydrojodid das (p-Amidinohydrocinnamoyl)-Gly-Asp-Val-OH•Trifluoracetat (1:1,1), Smp. 141-143°C (Aether), Ausbeute: quantitativ.

B) Der Ausgangsester wird wie folgt hergestellt:

a) Analog zu Beispiel 1B)e) erhält man durch Kupplung von p-Cyanohydrozimtsäure mit H-Gly-Asp(OtBu)-Val-OtBu (Beispiel 2 B)d)) den (p-Cyanohydrocinnamoyl)-Gly-Asp(OtBu)-Val-OtBu, Smp. 132-134°C (AcOEt/ Hexan), Ausbeute: 87%.

b) Analog zu Beispiel 1Bf) erhält man durch Thionierung der Vorstufe den p-(Thiocarbamoyl)hydrocinnamoyl-Gly-Asp(OtBu)--Val-OtBu, Smp. 70-73°C (Hexan), Ausbeute: 72%.

c) Analog zu Beispiel 1Bg) erhält man durch Methylierung der Vorstufe das p-[(Methylthio)formimidoyl]hydrocinnamoyl-Gly-Asp(OtBu)-Val-OtBu-Hydrojodid, Smp. 55-60°C (Aether/ Hexan), Ausbeute: 94%.

d) Analog zu Beispiel 1Bh) erhält man durch Ammonolyse der Vorstufe das (p-Amidinohydrocinnamoyl)-Gly-Asp(OtBu)-Val-OtBu-Hydrojodid, Smp. 116-120°C (Hexan), Ausbeute: 90%.

Beispiel 25

Analog Beispiel 1A) erhält man aus [N-Boc-3-(p-Amidinophenyl)-L-alanyl]-Gly-Asp(OtBu)-Val-OtBu-Hydrojodid das [3-(p-Amidinophenyl)-L-alanyl]-Gly-Asp-Val-OH-Trifluoracetat (2:3), Smp. 164-166°C (EtOH/AcOEt), Ausbeute: 75%.

Beispiel 26

A) Analog Beispiel 1A) erhält man durch Acidolyse von [(p-Amidinophenoxy)acetyl]-Gly-Asp(OtBu)-Val-OtBu-Hydrojodid mit TFA das (p-Amidinophenoxy)acetyl-Gly-Asp-Val-OH-Trifluoracetat, Smp. 140°C (Essigester/Hexan), Ausbeute: 67%.

B) Der Ausgangsester kann wie folgt hergestellt werden:

a) Analog Beispiel 1 B)e) erhält man durch Kupplung von p-Cyanophenoxyessigsäure und H-Gly-Asp(OtBu)-Val-OtBu das [p-(Cyanophenoxy)acetyl]-Gly-Asp(OtBu)-Val-OtBu, Smp. 55°C (Essigester/Hexan), Ausbeute: 81%.

b) Analog Beispiel 1 B)f) erhält man durch Reaktion der Vorstufe mit $H_2S$ den [p-(Thiocarbamoyl)phenoxyacetyl]-Gly--Asp(OtBu)-Val-OtBu, Ausbeute: 80% d.Th., MS: 595 $(M+H)^+$.

c) Analog Beispiel 1 B)g) erhält man durch Reaktion der Vorstufe mit Methyljodid das p-[(Methylthio)formimidoyl]-phenoxyacetyl-Gly-Asp(OtBu)-Val-OtBu-Hydrojodid, Ausbeute: 83%, MS: 609 $(M+H)^+$.

d) Analog Beispiel 1 B)h) erhält man durch Reaktion der Vorstufe mit Ammoniumacetat das [(p-Amidinophenoxy)acetyl]--Gly-Asp(OtBu)-Val-OtBu-Hydrojodid, Smp. 102-106°C (Essigester/Hexan), Ausbeute: 83%.

Beispiel 27

A) Analog Beispiel 1A) erhält man bei Einsatz von (p-Amidinophenyl)acetyl-Gly-Asp(OtBu)-Val-OtBu-Hydrojodid das (p-Amidinophenyl)acetyl-Gly-Asp-Val-OH-Trifluoracetat (5:4), Smp. 175-178°C (Acetonitril/Methanol), Ausbeute: 53%.

B) Der Ausgangsester wird wie folgt hergestellt:

a) Analog Beispiel 1 B)e) erhält man durch Kupplung von p-Cyanophenylessigsäure (J. Chem. Soc.1941, 744) und H-Gly-Asp(OtBu)-Val-OtBu (Beispiel 2 B)d)) das (p-Cyanophenyl)acetyl-Gly-Asp(OtBu)-Val-OtBu, Smp. 111°C (Essigester/Hexan).

b) Analog zu Beispiel 1 B)f)g)h) erhält man durch Reaktion der Vorstufe mit $H_2S$, MeJ und Ammoniumacetat das (p-Amidinophenyl)acetyl-Gly-Asp(OtBu)-Val-OtBu, MS: 562 $(M+H)^+$.

Beispiel 28

A) 216 mg N-[N-[N-(Benzyloxycarbonyl) -3-[p-[N-(benzyloxycarbonyl)amidino]phenyl] -DL-alanyl]glycyl]-β-alaninbenzylester und 72 mg 5% Pd/C werden in 4,3 ml Aethanol/Essigsäure (19:1) 28 Stunden unter Wasserstoff gerührt. Das Produkt wird mit Methanol/Essigsäure (9:1) an Kieselgel chromatographiert. Die reinen Fraktionen werden eingedampft, der Rückstand in verdünnter Salzsäure gelöst, filtriert, mit verdünntem Ammoniak neutralisiert, filtriert und das Filtrat eingedampft. Der Rückstand wird in Methanol aufgenommen, filtriert und das Filtrat mit Aether versetzt. Die Fällung wird abzentrifugiert, mit Aether gewaschen und getrocknet. Man erhält 28 mg N-[N-[3-(p-Amidinophenyl)-DL-alanyl]glycyl] -β-alanindihydrochlorid, MS: 336 (27, M+H).

B) Zur Herstellung des Ausgangsesters wird N-(Benzyloxycarbonyl)-3-(p-cyanophenyl)-DL-alanin und N-Glycyl-β-alaninbenzylestertrifluoracetat zu N-[N-[N-(Benzyloxycarbonyl)--3-(p-cyanophenyl)-DL-alanyl]glycyl]-β-alaninbenzylester gekuppelt, Smp. 134-135°C. Daraus erhält man mit Schwefelwasserstoff und Triäthylamin in Pyridin den N-[N-[N-(Benzyl-oxycarbonyl)-3-[p-(thiocarbamoyl)phenyl] -DL-alanyl]glycyl]--β-alaninbenzylester, Smp. 150-151°C. Reaktion mit Methyljodid in Aceton, anschliessendes Umsetzen mit Ammoniumacetat in Methanol und Behandlung mit Chlorameisensäurebenzylester und Triäthylamin in THF ergeben N-[N-[N-(Benzyloxycarbonyl) -3-[p-[N-(benzyloxycarbonyl)amidino]phenyl]-DL-alanyl]glycyl -β-alaninbenzylester, MS: 694 (100, M+H).

Beispiel 29

Aus 178 mg N-[N-[N-[(p-Amidinophenoxy)acetyl]glycyl]-3--t-butoxy-L-alanyl]-3-phenyl-L-alanin t-butylester-hydrojodid erhält man nach Behandlung mit Trifluoressigsäure in Methylenchlorid, wie in Beispiel 1 beschrieben, 91 mg des Trifluoracetates von N-[N-[N-[(p-Amidinophenoxy)acetyl]-glycyl]-L-α-aspartyl]-3-phenyl-L-alanin, Smp. 175-179°C.

Das Ausgangsmaterial kann wie folgt hergestellt werden:

a) Durch Kupplung von 7,0 g Z-Asp(OtBu)-O-Su mit 4,72 g Phe-O-tBu•HCl auf die in Beispiel 1B)a) beschriebene Weise isoliert man nach Aufarbeitung, Chromatographie an Kieselgel (Essigester) und Umkristallisation 7,1 g N-[N-[(Benzyloxy)-carbonyl]-3-(t-butoxycarbonyl)-L-alanyl]-3-phenyl-L-alanin--t-butylester, Smp. 94-95°C.

b) Nach katalytischer Hydrierung der Vorstufe (10,3 g) in Aethanol in Gegenwart von 10% Pd/C bei Raumtemperatur und Normaldruck erhält man nach Chromatographie an Kieselgel mit Essigester 5,94 g H-Asp(OtBu)-Phe-O-tBu, $[\alpha]_D$ = +9,16° (c = 0,6, $CH_3OH$).

c) Wie in Beispiel 1B)a) ausgeführt, gewinnt man aus der Reaktion von 4 g H-Asp(OtBu)-Phe-O-tBu mit 3,43 g Z-Gly-OSu nach Chromatographie an Kieselgel mit Essigester 3,9 g N-[N-[N-[(Benzyloxy)carbonyl]glycyl]-3-(t-butoxycarbonyl)--L-α-aspartyl]-3-phenyl-L-alanin-t-butylester.

d) In Analogie zu Beispil 1 erhält man durch Hydrogenolyse der Vorstufe (2,19 g) nach Chromatographie ($CH_2Cl_2$/ $CH_3OH$ 9:1) und Umkristallisation 909 mg N-[N-Glycyl-3--(t-butoxycarbonyl)-L-α-aspartyl]-3-phenyl-L-alanin-t--butylester, Smp. 99-100°C.

e) Analog zu Beispiel 1B)e) erhält man durch Kupplung von 675 mg der Vorstufe mit 266 mg p-Cyanophenoxyessigsäure nach Chromatographie an Kieselgel (Essigester) und Umkristallisation 687 mg N-[3-(t-Butoxycarbonyl)-N-[N-[(p-cyanophenoxy)-acetyl]glycyl]-L-alanyl]-3-phenyl-L-alanin-t-butylester, Smp. 83-85°C (Essigester/Hexan).

f) Analog zu Beispiel 1B)f) isoliert man nach Reaktion der Vorstufe (650 mg) mit $H_2S$ nach Chromatographie (Essigester) und Kristallisation 405 mg N-[3-(t-Butoxycarbonyl)--N-[N-[[(p-thiocarbamoyl)phenoxy]acetyl]glycyl]-L-alanyl]--3-phenyl-L-alanin-t-butylester, Smp. 83-86°C (Hexan).

g) Aus 390 mg der Vorstufe erhält man nach Methylierung gemäss Beispiel 1B)g) 375 mg N-[3-(t-Butoxycarbonyl)-N-[N--[[[p-(1-(methylthio)formimidoyl]phenoxy]acetyl]glycyl]-L--alanyl]-3-phenyl-L-alanin-t-butylester-hydrojodid, Smp. 162°C (Essigester/Methanol).

h) Die Umsetzung von 358 mg an Material der Vorstufe mit Ammoniumacetat analog zu Beispiel 1B)h) liefert 267

mg N-[N-[N-[(p-Amidinophenoxy)acetyl]glycyl]-3-t-butoxy-L--alanyl]-3-phenyl-L-alanin-t-butylester-hydrojodid, Zersetzungspunkt 76°C (Essigester/Hexan).

**Beispiel 30**

In Analogie zu Beispiel 1A) erhält man aus 17 mg 1-[[N-[N-(p-Amidinohydrocinnamoyl)glycyl]-3-(t-butoxycarbonyl)-L-alanyl]amino]cyclopentancarbonsäure nach Kristallisation 15 mg 1-[[N-[N-(p-Amidinohydrocinnamoyl)glycyl]--L-α-aspartyl]amino]cyclopentancarbonsäure-trifluoracetat (1:1), Smp. 136-138°C (Aether. Zersetzung).

Das Ausgangsmaterial kann wie folgt hergestellt werden:

a) Analog Beispiel 1B)e) erhält man aus 761 mg Z-Gly-Asp(OtBu)-OH (Beispiel 16) und 359 mg 1-Aminocyclopentancarbonsäuremethylester-hydrochlorid in Tetrahydrofuran nach Chromatographie an Kieselgel mit Essigester 920 mg N-[N-Benzyloxycarbonylglycyl]-3-[[1-(methoxycarbonyl)cyclopentyl]carbamoyl]-β-alanin-t-butylester, MS (FAB): 506 $(M+1)^+$.

b) Durch Hydrogenolyse analog Beispiel 1B)b) erhält man aus 880 mg des Produktes der Vorstufe 620 mg 1-[[3-(t-Butoxycarbonyl)-N-glycyl-L-alanyl]amino]cyclopentancarbonsäuremethylester, MS (FAB): 372 $(M+1)^+$.

c) Analog Beispiel 1B)e) erhält man durch Kupplung von 310 mg 3-(p-Cyanophenyl)propionsäure und 600 mg des Produktes der Vorstufe nach chromatographischer Reinigung (Kieselgel; Essigester→Essigester/Methanol 9:1) 635 mg 1-[[3-(t-Butoxycarbonyl)-N-[N-(p-cyanohydrocinnamoyl)-glycyl]-L-alanyl]amino]cyclopentancarbonsäuremthylester, MS: 546 $(M+NH_4)^+$.

d) Analog zu den Beispielen 1Bf)g)h) erhält man durch aufeinander folgende Umsetzung von 600 mg des Produktes der Vorstufe mit $H_2S$, MeI und Ammoniumacetat 279 mg 1-[[N-[N--(p-Amidinohydrocinnamoyl)glycyl]-3-(t-butoxycarbonyl)-L--alanyl]amino]cyclopentancarbonsäuremethylester-hydrojodid, Smp. 98°C (Zersetzung), MS (FAB): 546 $(M+1)^+$.

e) Die alkalische Hydrolyse von 157 mg des Produktes der Vorstufe wie in Beispiel 16B)b) beschrieben, liefert 24 mg 1-[[N-[N-(p-Amidinohydrocinnamoyl)glycyl]-3-(t-butoxycarbonyl)-L-alanyl]amino]cyclopentancarbonsäure, Smp. 163-164°C.

Beispiel A

Eine Verbindung der Formel I kann man in an sich bekannter Weise als Wirkstoff zur Herstellung von Tabletten folgender Zusammensetzung verwenden:

| | pro Tablette |
|---|---|
| Wirkstoff | 200 mg |
| mikrokristalline Cellulose | 155 mg |
| Maisstärke | 25 mg |
| Talk | 25 mg |
| Hydroxypropylmethylcellulose | 20 mg |
| | 425 mg |

**Beispiel B**

Eine Verbindung der Formel I kann man in an sich bekannter Weise als Wirkstoff zur Herstellung von Kapseln folgender Zusammensetzung verwenden:

| | pro Kapsel |
|---|---|
| Wirkstoff | 100,0 mg |
| Maisstärke | 20,0 mg |
| Milchzucker | 95,0 mg |
| Talk | 4,5 mg |

(fortgesetzt)

| | pro Kapsel |
|---|---|
| Magnesiumstearat | 0,5 mg |
| | 220,0 mg |

## Patentansprüche

### Patentansprüche für folgende Vertragsstaaten : AT, BE, CH, DE, DK, FR, GB, IT, LI, LU, NL, SE

**1.** Glycinderivate der Formel

$$R\text{-}CONH\text{-}CH_2\text{-}CONH\text{-}CH(R')\text{-}CH_2COOH \quad \text{I}$$

worin

R eine Gruppe der Formel

$$-CH(NH\text{-}R^a)\text{-}(CH_2)_{1\text{-}6}\text{-}NH\text{-}R^b \quad \text{(R-1)}$$

$$-(T)_{1\ oder\ 0}\text{-}C_6H_4\text{-}CH_2NH\text{-}R^c \quad \text{(R-2)}$$

oder

$$-(T)_{1\ oder\ 0}\text{-}C_6H_4\text{-}(NH)_n\text{-}C(NH)\text{-}L \quad \text{(R-3)}$$

ist, und
$R^a$ Wasserstoff, $-COO\text{-}C_{1\text{-}4}$-Alkyl, Z, $-COC_6H_5$, $-COC_6H_4N_3$, $-SO_2C_6H_5$, $-SO_2$-Naphthyl oder $-COCH_2N(Y)\text{-}CH_2CH_2NH\text{-}Y$,
Y Wasserstoff, Boc oder Z,
$R^b$ eine Gruppe der Formel $-C(NH)(CH_2)_{0\text{-}3}\text{-}CH_3$ oder

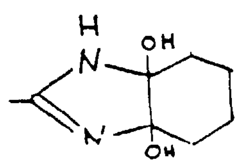

oder, falls
$R^a$ eine Gruppe der Formel $-COC_6H_4N_3$, $-SO_2C_6H_5$, $SO_2$-Naphthyl oder $-COCH_2N(Y)\text{-}CH_2CH_2NH\text{-}Y$ ist, auch Amidino,
$R^c$ Wasserstoff oder Amidino,
n die Zahl 1 oder 0
L Amino,
T eine Gruppe der Formel $-CH_2\text{-}(O)_{1\ oder\ 0}\text{-}$,-CH=CH- oder $-CH(R^d)\text{-}CH_2\text{-}$,
$R^d$ Wasserstoff oder $-NH\text{-}R^a$,
R' Wasserstoff oder $-CO\text{-}R^o$,
$R^o$ Amino, $-NH\text{-}C_{1\text{-}4}$-Alkyl, $-NH(CH_2)_{1\text{-}4}\text{-}C_6H_5$, $-NH(CH_2)_{1\text{-}4}\text{-}C_6H_4$-Hal, $-NH\text{-}C_6H_4\text{-}COOH$,

-NH-$C_6H_4$-COO-$C_{1-4}$ -Alkyl oder der Rest einer über die Aminogruppe gebundene α-Aminocarbonsäure ist,

sowie Hydrate oder Solvate und physiologisch verwendbare Salze davon.

2. Verbindungen nach Anspruch 1, worin R eine Gruppe der Formel R-1, insbesondere solche worin R-CO- die Gruppe Aeg-Arg-, Z-Aeg(Z)-Arg-, 2-Naphthyl-$SO_2$-Arg-, o-Azido- benzoyl-Arg-, $N^2$-Boc-$N^6$-(1-Iminoäthyl)-Lys-oder $N^2$-Boc-$N^5$-(3a,4,5,6,7,7a-Hexahydro-3a,7a-dihydroxy -1H-benzimidazol-2-yl)-Orn- ist.

3. Verbindungen nach Anspruch 1, worin R eine Gruppe R-2, insbesondere solche worin R-CO- die Gruppe p-(Aminomethyl)hydrocinnamoyl ist.

4. Verbindungen nach Anspruch 1, worin R eine Gruppe R-3, insbesondere solche worin R-CO- die Gruppe p-Amidinohydrocinnamoyl, 3-(p-Amidinophenyl oder p-Guanidinophenyl)-alanyl, N-Z- oder N-Boc-3-(p-Amidinophenyl) alanyl, N-Boc-3-(p-Guanidinophenyl)alanyl, p-Amidinophenoxyacetyl, p-Amidinophenacetyl, 2-[(p-Amidinophenyl)-1,3-dioxolan--2-yl]acetyl, (p-Amidinobenzoyl)acetyl oder N-Boc-3-(p--Acetimidoylaminophenyl)alanyl ist.

5. Verbindungen nach einem der Ansprüche 1-3 worin R' Wasserstoff, -CO-Val-OH, -CO-Ser-OH, -CO-Phe-OH, L-Carboxy--2-(1-naphthyl)äthylidencarbamoyl, -CO-ILe-OH, Carboxyphenylcarbamoyl, Isobutylcarbamoyl, p-Fluorphenäthylcarbamoyl oder Carboxycyclopentancarbamoyl ist.

6. Verbindungen nach Anspruch 1 aus der Gruppe der folgenden:

[3-(p-Amidinophenyl)-DL-alanyl]-Gly-Asp-Val-OH,
Z-Aeg(Z)-Arg-Gly-Asp-Val-OH,
Aeg-Arg-Gly-Asp-Val-OH,
Aeg-Arg-Gly-Asp-Ser-OH,
N-Aeg-Arg-Gly-Asp-Nal(1)-OH,
Aeg-Arg-Gly-Asp-ILe-OH,
[$N^2$-Boc-$N^6$-(1-Iminoäthyl)-L-lysyl]-Gly-Asp-Val-OH,
N-[(o-Azidobenzoyl)-Arg-Gly-Asp]-anthranisäure,
$N^2$-Boc-$N^5$-(3a,4,5,6,7,7a-Hexahydro-3a,7a-dihydroxy-1H-benzimidazol-2-yl)-L-ornithyl]-Gly-Asp-Val-OH,
[N-Boc-3-(p-Guanidinophenyl)-DL-alanyl]-Gly-Asp-Val-OH,
[3-(p-Amidinophenyl)-DL-alanyl]-Gly-Asp-Nal(1)-OH,
[N-Boc-3-(p-Amidinophenyl)-DL-alanyl]-Gly-Asp-Val-OH,
(p-Amidinohydrocinnamoyl)-Gly-Asp-Nal(1)-OH,
[3-(p-Amidinophenyl)-D-alanyl]-Gly-Asp-Val-OH,
(p-Aminomethylhydrocinnamoyl)-Gly-Asp-Val-OH,
(p-Amidinohydrocinnamoyl)-Gly-Asp-Val-OH,
[3-(p-Amidinophenyl)-L-alanyl]-Gly-Asp-Val-OH,
(p-Amidinophenoxy)acetyl-Gly-Asp-Val-OH und
(p-Amidinophenyl)acetyl-Gly-Asp-Val-OH.

7. Verbindungen der Formel

$$R^2\text{-CONH-CH}_2\text{-CONH-CH}(R^4)\text{-CH}_2\text{COOR}^3 \qquad \text{II}$$

worin

$R^2$ eine Gruppe der Formel

$$\text{-CH(NH-R}^a)\text{-(CH}_2)_{1\text{-}6}\text{-R}^5 \qquad \text{(R-1a)}$$

$$\text{-(T)}_{1\text{ oder }0}\text{-C}_6\text{H}_4\text{-CH}_2\text{-R}^6 \qquad \text{(R-2a)}$$

oder

$$-(T)_{1\ oder\ 0}-C_6H_4-R^7 \quad (R\text{-}3a)$$

ist, in der
$R^5$ eine geschützte Guanidinogruppe oder eine Gruppe -NH-$R^b$,
$R^6$ eine geschützte Amino- oder Guanidinogruppe oder eine Gruppe -NH-$R^c$,
$R^7$ gegebenenfalls geschütztes Amidino oder Guanidino,
$R^3$ Wasserstoff oder eine leicht spaltbare Estergruppe,
$R^4$ die gleiche Bedeutung wie R' hat oder eine Gruppe -$COR^8$ ist, in der $R^8$ ein über die Aminogruppe gebundener leicht spaltbarer α-Aminocarbonsäureester ist,
wobei, falls $R^3$ Wasserstoff ist und $R^4$ die gleiche Bedeutung wie R' hat, $R^2$ zumindest eine geschützte Guanidino-. Amino- oder Amidinogruppe $R^5$, $R^6$ bzw.
$R^7$ enthalten soll,
und $R^a$, $R^b$, $R^c$, R' und T die in Anspruch 1 angegebene Bedeutung haben.

**8.** Verbindungen nach einem der Ansprüche 1-6 zur Verwendung als Heilmittel, insbesondere als Hemmer der Bindung von adhäsiven Proteinen an Blutplättchen, sowie der Blutplättchenaggregation und der ZeLl-Zell-Adhäsion.

**9.** Verfahren zur Herstellung der Verbindungen nach einem der Ansprüche 1-6, dadurch gekennzeichnet, dass man

a) in einer Verbindung der Formel

$$R^2\text{-CONH-}CH_2\text{-CONH-CH}(R^4)\text{-}CH_2COOR^3 \quad II$$

worin

$R^2$ eine Gruppe der Formel

$$-CH(NH-R^a)-(CH_2)_{1-6}-R^5 \quad (R\text{-}1a)$$

$$-(T)_{1\ oder\ 0}-C_6-H_4-CH_2-R^6 \quad (R\text{-}2a)$$

oder

$$-(T)_{1\ oder\ 0}-C_6H_4-R^7 \quad (R\text{-}3a)$$

ist, in der
$R^5$ eine geschützte Guanidinogruppe oder eine Gruppe -NH-$R^b$,
$R^6$ eine geschützte Amino- oder Guanidinogruppe oder eine Gruppe -NH-$R^c$.
$R^7$ gegebenenfalls geschütztes Amidino oder Guanidino,
$R^3$ Wasserstoff oder eine Leicht spaltbare Estergruppe,
$R^4$ die gleiche Bedeutung wie R' hat oder eine Gruppe -$COR^8$ ist, in der $R^8$ ein über die Aminogruppe gebundener leicht spaltbarer α-Aminocarbonsäureester ist,
wobei, falls $R^3$ Wasserstoff ist und $R^4$ die gleiche Bedeutung wie R' hat. $R^2$ zumindest eine geschützte Guanidino-, Amino- oder Amidinogruppe $R^5$, $R^6$ bzw.
$R^7$ enthalten soll,
und $R^a$, $R^b$, $R^c$, R' und T die in Anspruch L angegebene Bedeutung haben,

die vorhandene(n) Estergruppe(n) und eine oder mehrere vorhandene geschützte Amino-, Amidino- oder Guanidinogruppen spaltet oder

b) ein Amin der Formel

$$\begin{array}{l} CH(NH_2)-(CH_2)_{1-6}-NH-C(NH)NH_2 \\ | \\ CONH-CH_2-CONH-CH(R')-CH_2COOH \end{array} \qquad III$$

mit einem die Gruppe $-COC_6H_5$, $-COC_6H_4N_3$, $-SO_2C_6H_5$, $-SO_2$-Naphthyl oder $-COCH_2N(Y)$-$-CH_2CH_2NH$-Y einführenden Mittel umsetzt, worin R' und Y die obige Bedeutung haben, oder

c) ein Guanidinderivat der Formel

$$\begin{array}{l} CH(NH-R^9)-(CH_2)_{1-6}-NHC(NH)-NH_2 \\ | \\ CONH-CH_2-CONH-CH(R')-CH_2COOH \end{array} \qquad \underline{IV}$$

worin $R^9$ $-COO-C_{1-4}$,-Alkyl, Z, $-COC_6H_5$, $-COC_6H_4N_3$, $-SO_2C_6H_5$, $-SO_2$-Naphthyl oder $-COCH_2N(Y')-CH_2CH_2NH-Y'$ ist, in dem Y' Boc oder Z darstellt und R' obige Bedeutung hat, mit 1,2-Cyclohexandion umsetzt oder

d) in einem Amin der Formel

$$\begin{array}{l} NH-CH_2-CONH-CH_2-CONH-CH(R')-CH_2COOH \\ | \\ CO-(L)_{1\ oder\ 0}-C_6H_4-(CH_2)_{1\ oder\ 0}-NH_2 \end{array} \qquad \underline{V}$$

worin L eine Gruppe $-CH_2(O)_{1\ oder\ 0}$-, -CH=CH- oder $-CH(NH-R^9)-CH_2$- ist, und R' und $R^9$ die obige Bedeutung haben,
die Aminogruppe in eine Guanidinogruppe umwandelt oder

e) ein Nitril der Formel

$$N{\equiv}C-C_6H_4-(T)_{1\ oder\ 0}-CONH-CH_2-CONH-CH(R')-CH_2COOH \qquad VI$$

zum Amin hydriert,

f) gewünschtenfalls einen in der Gruppe R einer Verbindung der Formel I enthaltenen reaktionsfähigen Substituenten funktionell abwandelt und

g) gewünschtenfalls eine Verbindung der Formel I in ein Salz oder ein Salz einer Verbindung der Formel I in die freie Verbindung der Formel I überführt.

**10.** Pharmazeutische Präparate, insbesondere für die Behandlung oder Prophylaxe von Krankheiten, die durch die Bindung von adhäsiven Proteinen an Blutplättchen. sowie durch die Blutplättchenaggregation und die Zell-Zell-Adhäsion verursacht sind, enthaltend eine Verbindung nach einem der Ansprüche 1-6 als Wirkstoff.

**11.** Verwendung einer Verbindung nach einem der Ansprüche 1-6 zur Herstellung von Arzneimitteln zur Behandlung oder Prophylaxe von Krankheiten, die durch die Bindung von adhäsiven Proteinen an Blutplättchen, sowie durch die Blutplättchenaggregation und die Zell-Zell-Adhäsion verursacht sind.

**Patentansprüche für folgenden Vertragsstaat : GR**

**1.** Glycinderivate der Formel

$$R\text{-}CONH\text{-}CH_2\text{-}CONH\text{-}CH(R')\text{-}CH_2COOH \qquad I$$

worin

R eine Gruppe der Formel

$$-CH(NH\text{-}R^a)\text{-}(CH_2)_{1\text{-}6}\text{-}NH\text{-}R^b \qquad (R\text{-}1)$$

$$-(T)_{1 \text{ oder } 0}\text{-}C_6H_4\text{-}CH_2NH\text{-}R^c \qquad (R\text{-}2)$$

oder

$$-(T)_{1 \text{ oder } 0}\text{-}C_6H_4\text{-}(NH)_n\text{-}C(NH)\text{-}L \qquad (R\text{-}3)$$

ist, und
$R^a$ Wasserstoff. -COO-$C_{1-4}$-Alkyl, Z, -$COC_6H_5$,
-$COC_6H_4N_3$, -$SO_2C_6H_5$, -$SO_2$-Naphthyl oder
-$COCH_2N(Y)$-$CH_2CH_2NH$-Y,
Y Wasserstoff, Boc oder Z,
$R^b$ eine Gruppe der Formel -C(NH)$(CH_2)_{0-3}$-$CH_3$ oder

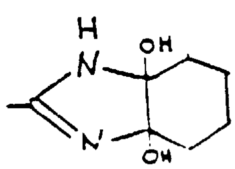

oder, falls
$R^a$ eine Gruppe der Formel -$COC_6H_4N_3$, -$SO_2C_6H_5$, $SO_2$-Naphthyl
oder -$COCH_2N(Y)$-$CH_2CH_2NH$-Y ist, auch Amidino.
$R^c$ Wasserstoff oder Amidino,
n die Zahl 1 oder 0
L Amino,
T eine Gruppe der Formel -$CH_2$-$(O)_{1 \text{ oder } 0}$-, -CH=CH-oder
-$CH(R^d)$-$CH_2$-,
$R^d$ Wasserstoff oder -NH-$R^a$,
R' Wasserstoff oder -CO-$R^o$,
$R^o$ Amino, -NH-$C_{1-4}$ -Alkyl, -NH$(CH_2)_{1-4}$ $C_6H_5$,
-NH$(CH_2)_{1-4}$ $C_6H_4$-Hal, NH-$C_6H_4$-COOH,

$-NH-C_6H_4-COO-C_{1-4}$-Alkyl oder der Rest einer über die Aminogruppe gebundene α-Aminocarbonsäure ist,

sowie Hydrate oder Solvate und physiologisch verwendbare Salze davon.

**2.** Verbindungen nach Anspruch 1, worin R eine Gruppe der Formel R-1, insbesondere solche worin R-CO- die Gruppe Aeg-Arg-, Z-Aeg(Z)-Arg-, 2-Naphthyl-$SO_2$-Arg-, o-Azido- benzoyl-Arg-, $N^2$-Boc-$N^6$-(1-Iminoäthyl)-Lys-oder $N^2$-Boc-$N^5$-(3a,4,5,6,7,7a-Hexahydro-3a,7a-dihydroxy -1H-benzimidazol-2-yl)-Orn- ist.

**3.** Verbindungen nach Anspruch 1, worin R eine Gruppe R-2, insbesondere solche worin R-CO- die Gruppe p-(Aminomethyl)hydrocinnamoyl ist.

**4.** Verbindungen nach Anspruch 1, worin R eine Gruppe R-3, insbesondere solche worin R-CO- die Gruppe p-Amidinohydrocinnamoyl, 3-(p-Amidinophenyl oder p-Guanidinophenyl)-alanyl, N-Z- oder N-Boc-3-(p-Amidinophenyl) alanyl, N-Boc-3-(p-Guanidinophenyl)alanyl, p-Amidinophenoxyacetyl, p-Amidinophenacetyl, 2-[(p-Amidinophenyl)-1,3-dioxolan--2-yl]acetyl, (p-Amidinobenzoyl)acetyl oder N-Boc-3-(p--Acetimidoylaminophenyl)alanyl ist.

**5.** Verbindungen nach einem der Ansprüche 1-3, worin R' Wasserstoff, -CO-Val-OH, -CO-Ser-OH, -CO-Phe-OH, 1-Carboxy--2-(1-naphthyl)äthylidencarbamoyl, -CO-Ile-OH, Carboxyphenylcarbamoyl, Isobutylcarbamoyl, p-Fluorphenäthylcarbamoyl oder Carboxycyclopentancarbamoyl ist.

**6.** Verbindungen nach Anspruch 1 aus der Gruppe der folgenden:

[3-(p-Amidinophenyl)-DL-alanyl]-Gly-Asp-Val-OH,
Z-Aeg(Z)-Arg-Gly-Asp-Val-OH,
Aeg-Arg-Gly-Asp-Val-OH,
Aeg-Arg-Gly-Asp-Ser-OH,
N-Aeg-Arg-Gly-Asp-Nal(1)-OH,
Aeg-Arg-Gly-Asp-ILe-OH,
[$N^2$-Boc-$N^6$-(1-Iminoäthyl)-L-lysyl]-Gly-Asp-Val-OH,
N-[(o-Azidobenzoyl)-Arg-Gly-Asp]-anthranilsäure,
$N^2$-Boc-$N^5$-(3a,4,5,6,7,7a-Hexahydro-3a.7a-dihydroxy-1H-benzimidazol-2-yl)-L-ornithyl]-Gly-Asp-Val-OH,
[N-Boc-3-(p-Guanidinophenyl)-DL-alanyl]-Gly-Asp-Val-OH,
[3-(p-Amidinophenyl)-DL-alanyl]-Gly-Asp-Nal(1)-OH,
[N-Boc-3-(p-Amidinophenyl)-DL-alanyl]-Gly-Asp-Val-OH,
(p-Amidinohydrocinnamoyl)-Gly-Asp-Nal(1)-OH,
[3-(p-Amidinophenyl)-D-alanyl]-Gly-Asp-Val-OH,
(p-Aminomethylhydrocinnamoyl)-Gly-Asp-Val-OH,
(p-Amidinohydrocinnamoyl)-Gly-Asp-Val-OH,
[3-(p-Amidinophenyl)-L-alanyl]-Gly-Asp-Val-OH,
(p-Amidinophenoxy)acetyl-Gly-Asp-Val-OH und
(p-Amidinophenyl)acetyl-Gly-Asp-Val-OH.

**7.** Verbindungen der Formel

$$R^2\text{-CONH-CH}_2\text{-CONH-CH}(R^4)\text{-CH}_2\text{COOR}^3 \quad \text{II}$$

worin

$R^2$ eine Gruppe der Formel

$$\text{-CH(NH-}R^a\text{)-(CH}_2\text{)}_{1\text{-}6}\text{-}R^5 \quad \text{(R-1a)}$$

$$\text{-(T)}_{1\text{ oder }0}\text{-C}_6\text{H}_4\text{-CH}_2\text{-}R^6 \quad \text{(R-2a)}$$

oder

$$-(T)_{1\ oder\ 0}-C_6H_4-R^7 \qquad (R\text{-}3a)$$

ist, in der
$R^5$ eine geschützte Guanidinogruppe oder eine Gruppe -NH-$R^b$,
$R^6$ eine geschützte Amino- oder Guanidinogruppe oder eine Gruppe -NH-$R^c$,
$R^7$ gegebenenfalls geschütztes Amidino oder Guanidino,
$R^3$ Wasserstoff oder eine leicht spaltbare Estergruppe.
$R^4$ die gleiche Bedeutung wie R' hat oder eine Gruppe -$COR^8$ ist, in der $R^8$ ein über die Aminogruppe gebundener leicht spaltbarer α-Aminocarbonsäureester ist,
wobei, falls $R^3$ Wasserstoff ist und $R^4$ die gleiche Bedeutung wie R' hat, $R^2$ zumindest eine geschützte Guanidino-. Amino- oder Amidinogruppe $R^5$, $R^6$ bzw.
$R^7$ enthalten soll,
und $R^a$, $R^b$, $R^c$, R' und T die in Anspruch 1 angegebene Bedeutung haben.

**8.** Verbindungen nach einem der Ansprüche 1-6 zur Verwendung als Heilmittel, insbesondere als Hemmer der Bindung von adhäsiven Proteinen an Blutplättchen, sowie der Blutplättchenaggregation und der Zell-Zell-Adhäsion.

**9.** Verfahren zur Herstellung der Verbindungen nach einem der Ansprüche 1-6, dadurch gekennzeichnet, dass man

a) in einer Verbindung der Formel

$$R^2\text{-CONH-}CH_2\text{-CONH-CH}(R^4)\text{-}CH_2COOR^3 \qquad II$$

worin

$R^2$ eine Gruppe der Formel

$$-CH(NH-R^a)-(CH_2)_{1-6}-R^5 \qquad (R\text{-}1a)$$

$$-(T)_{1\ oder\ 0}-C_6H_4-CH_2-R^6 \qquad (R\text{-}2a)$$

oder

$$-(T)_{1\ oder\ 0}-C_6H_4-R^7 \qquad (R\text{-}3a)$$

ist, in der
$R^5$ eine geschützte Guanidinogruppe oder eine Gruppe -NH-$R^b$,
$R^6$ eine geschützte Amino- oder Guanidinogruppe oder eine Gruppe -NH-$R^c$,
$R^7$ gegebenenfalls geschütztes Amidino oder Guanidino,
$R^3$ Wasserstoff oder eine leicht spaltbare Estergruppe,
$R^4$ die gleiche Bedeutung wie R' hat oder eine Gruppe -$COR^8$ ist, in der $R^8$ ein über die Aminogruppe gebundener leicht spaltbarer α-Aminocarbonsäureester ist,
wobei. falls $R^3$ Wasserstoff ist und $R^4$ die gleiche Bedeutung wie R' hat, $R^2$ zumindest eine geschützte Guanidino-, Amino- oder Amidinogruppe $R^5$, $R^6$ bzw.
$R^7$ enthalten soll,
und $R^a$, $R^b$, $R^c$, R' und T die in Anspruch 1 angegebene Bedeutung haben,

die vorhandene(n) Estergruppe(n) und eine oder mehrere vorhandene geschützte Amino-, Amidino- oder Guanidinogruppen spaltet oder

b) ein Amin der Formel

$$\begin{array}{l} CH(NH_2)-(CH_2)_{1-6}-NH-C(NH)NH_2 \\ | \\ CONH-CH_2-CONH-CH(R')-CH_2COOH \end{array} \qquad III$$

mit einem die Gruppe $-COC_6H_5$, $-COC_6H_4N_3$, $-SO_2C_6H_5$, $-SO_2$-Naphthyl oder $-COCH_2N(Y)$--$CH_2CH_2NH$-Y einführenden Mittel umsetzt, worin R' und Y die obige Bedeutung haben, oder

c) ein Guanidinderivat der Formel

$$\begin{array}{l} CH(NH-R^9)-(CH_2)_{1-6}-NHC(NH)-NH_2 \\ | \\ CONH-CH_2-CONH-CH(R')-CH_2COOH \end{array} \qquad IV$$

worin $R^9$ -COO-$C_{1-4}$-Alkyl, Z, $-COC_6H_5$,
$-COC_6H_4N_3$, $-SO_2C_6H_5$, $-SO_2$-Naphthyl oder
$-COCH_2N(Y')-CH_2CH_2NH$-Y' ist, in dem Y' Boc oder Z darstellt
und R' obige Bedeutung hat,
mit 1,2-Cyclohexandion umsetzt oder

d) in einem Amin der Formel

$$\begin{array}{l} NH-CH_2-CONH-CH_2-CONH-CH(R')-CH_2COOH \\ | \\ CO-(L)_{1\ oder\ 0}-C_6H_4-(CH_2)_{1\ oder\ 0}-NH_2 \end{array} \qquad V$$

worin L eine Gruppe $-CH_2(O)_{1\ oder\ 0}-$, -CH=CH- oder $-CH(NH-R^9)-CH_2-$ ist, und R' und $R^9$ die obige Bedeutung haben,
die Aminogruppe in eine Guanidinogruppe umwandelt oder

e) ein Nitril der Formel

$$N{\equiv}C-C_6H_4-(T)_{1\ oder\ 0}-CONH-CH_2-CONH-CH(R')-CH_2COOH \qquad VII$$

zum Amin hydriert,

f) gewünschtenfalls einen in der Gruppe R einer Verbindung der Formel I enthaltenen reaktionsfähigen Substituenten funktionell abwandelt und

g) gewünschtenfalls eine Verbindung der Formel I in ein Salz oder ein Salz einer Verbindung der Formel I in die freie Verbindung der Formel I überführt.

**10.** Verfahren zur Herstellung eines pharmazeutischen Präparates, insbesondere für die Behandlung oder Prophylaxe von Krankheiten, die durch die Bindung von adhäsiven Proteinen an Blutplättchen, sowie durch die Blutplättchenaggregation und die Zell-Zell-Adhäsion verursacht sind, enthaltend eine Verbindung nach einem der Ansprüche 1-6 als Wirkstoff.

**11.** Verwendung einer Verbindung nach einem der Ansprüche 1-6 zur Herstellung von Arzneimitteln zur Behandlung oder Prophylaxe von Krankheiten, die durch die Bindung von adhäsiven Proteinen an Blutplättchen, sowie durch die Blutplättchenaggregation und die Zell-Zell-Adhäsion verursacht sind.

**Patentansprüche für folgenden Vertragsstaat : ES**

**1.** Verfahren zur Herstellung von Glycinderivaten der Formel

$$R\text{-}CONH\text{-}CH_2\text{-}CONH\text{-}CH(R')\text{-}CH_2COOH \qquad I$$

worin

R eine Gruppe der Formel

$$-CH(NH\text{-}R^a)\text{-}(CH_2)_{1\text{-}6}\text{-}NH\text{-}R^b \qquad (R\text{-}1)$$

$$-(T)_{1\ oder\ 0}\text{-}C_6H_4\text{-}CH_2NH\text{-}R^c \qquad (R\text{-}2)$$

oder

$$-(T)_{1\ oder\ 0}\text{-}C_6H_4\text{-}(NH)_n\text{-}C(NH)\text{-}L \qquad (R\text{-}3)$$

ist, und
$R^a$ Wasserstoff -COO-$C_{1\text{-}4}$ Alkyl, Z, -$COC_6H_5$,
-$COC_6H_4N_3$, -$SO_2C_6H_5$, -$SO_2$-Naphthyl oder
- $COCH_2N(Y)\text{-}CH_2CH_2NH\text{-}Y$,
Y Wasserstoff. Boc oder Z,
$R^b$ eine Gruppe der Formel -$C(NH)(CH_2)_{0\text{-}3}\text{-}CH_3$ oder

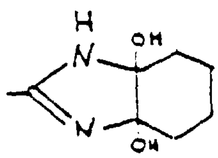

oder, falls $R^a$ eine Gruppe der Formel -$COC_6H_4N_3$, -$SO_2C_6H_5$, $SO_2$-Naphthyl oder -$COCH_2N(Y)\text{-}CH_2CH_2NH$-Y ist, auch Amidino,
$R^c$ Wasserstoff oder Amidino,
n die Zahl 1 oder 0
L Amino,
T eine Gruppe der Formel -$CH_2\text{-}(O)_{1\ oder\ 0}$-, -CH=CH-, -$CH(R^d)\text{-}CH_2$-
$R^d$ Wasserstoff oder -NH-$R^a$,
R' Wasserstoff oder -CO-$R^o$,
$R^o$ Amino, -NH-$C_{1\text{-}4}$-Alkyl, $NH(CH_2)_{1\text{-}4}\text{-}C_6H_5$,
-$NH(CH_2)_{1\text{-}4}\text{-}C_6H_4$-Hal, -NH-$C_6H_4$-COOH,
-NH-$C_6H_4$-COO-$C_{1\text{-}4}$-Alkyl oder der Rest einer über die Aminogruppe gebundenen $\alpha$-Aminocarbonsäure ist,

sowie von Hydraten oder Solvaten und physiologisch verwendbaren Salzen davon, dadurch gekennzeichnet, dass man

a) in einer Verbindung der Formel

$$R^2\text{-CONH-CH}_2\text{-CONH-CH}(R^4)\text{-CH}_2\text{COOR}^3 \qquad II$$

worin

$R^2$ eine Gruppe der Formel

$$\text{-CH(NH-}R^a\text{)-(CH}_2)_{1\text{-}6}\text{-}R^5 \qquad (R\text{-1a})$$

$$\text{-(T)}_{1\ \text{oder}\ 0}\text{-C}_6\text{H}_4\text{-CH}_2\text{-}R^6 \qquad (R\text{-2a})$$

oder

$$\text{-(T)}_{1\ \text{oder}\ 0}\text{-C}_6\text{H}_4\text{-}R^7 \qquad (R\text{-3a})$$

ist, in der
$R^5$ eine geschützte Guanidinogruppe oder eine Gruppe -NH-$R^b$,
$R^6$ eine geschützte Amino- oder Guanidinogruppe oder eine Gruppe -NH-$R^c$,
$R^7$ gegebenenfalls geschütztes Amidino oder Guanidino,
$R^3$ Wasserstoff oder eine leicht spaltbare Estergruppe,
$R^4$ die gleiche Bedeutung wie R' hat oder eine Gruppe -COR$^8$ ist, in der $R^8$ ein über die Aminogruppe gebundener leicht spaltbarer $\alpha$-Aminocarbonsäureester ist,
wobei, falls $R^3$ Wasserstoff ist und $R^4$ die gleiche Bedeutung wie R' hat, $R^2$ zumindest eine geschützte Guanidino-, Amino- oder Amidinogruppe $R^5$, $R^6$ bzw.
$R^7$ enthalten soll ,
und $R^a$, $R^b$, $R^c$, R' und T die weiter oben angegebene Bedeutung haben,

die vorhandene(n) Estergruppe(n) und eine oder mehrere vorhandene geschützte Amino-, Amidino- oder Guanidinogruppen spaltet oder

b) ein Amin der Formel

```
CH(NH2)-(CH2)1-6-NH-C(NH)NH2
|                                        III
CONH-CH2-CONH-CH(R')-CH2COOH
```

mit einem die Gruppe -COC$_6$H$_5$, -COC$_6$H$_4$N$_3$, -SO$_2$C$_6$H$_5$, -SO$_2$-Naphthyl oder -COCH$_2$N(Y)--CH$_2$CH$_2$NH-Y einführenden Mittel umsetzt, worin R' und Y die obige Bedeutung haben, oder

c) ein Guanidinderivat der Formel

$$CH(NH\text{-}R^9)\text{-}(CH_2)_{1\text{-}6}\text{-}NHC(NH)\text{-}NH_2$$
$$|$$
$$CONH\text{-}CH_2\text{-}CONH\text{-}CH(R')\text{-}CH_2COOH \qquad \text{IV}$$

worin $R^9$ $-COO-C_{1-4}$,-Alkyl, Z, $-COC_6H_5$, $-COC_6H_4N_3$, $-SO_2C_6H_5$, $-SO_2$-Naphthyl oder $-COCH_2N(Y')-CH_2CH_2NH-Y'$ ist, in dem Y' Boc oder Z darstellt und R' obige Bedeutung hat,
mit 1,2-Cyclohexandion umsetzt oder

d) in einem Amin der Formel

$$NH\text{-}CH_2\text{-}CONH\text{-}CH_2\text{-}CONH\text{-}CH(R')\text{-}CH_2COOH$$
$$|$$
$$CO\text{-}(L)_{1\ \text{oder}\ 0}\text{-}C_6H_4\text{-}(CH_2)_{1\ \text{oder}\ 0}\text{-}NH_2 \qquad \text{V}$$

worin L eine Gruppe $-CH_2(O)_{1\ \text{oder}\ 0}-$, -CH=CH- oder $-CH(NH-R^9)-CH_2-$ ist, und R' und $R^9$ die obige Bedeutung haben,
die Aminogruppe in eine Guanidinogruppe umwandelt oder

e) ein Nitril der Formel

$$N\equiv C\text{-}C_6H_4\text{-}(T)_{1\ \text{oder}\ 0}\text{-}CONH\text{-}CH_2\text{-}CONH\text{-}CH(R')\text{-}CH_2COOH \qquad \text{VI}$$

zum Amin hydriert,

f) gewünschtenfalls einen in der Gruppe R einer Verbindung der Formel I enthaltenen reaktionsfähigen Substituenten funktionell abwandelt und

g) gewünschtenfalls eine Verbindung der Formel I in ein Salz oder ein Salz einer Verbindung der Formel I in die freie Verbindung der Formel I überführt.

**2.** Verfahren nach Anspruch 1 worin R eine Gruppe der Formel R-1, insbesondere solche worin R-CO- die Gruppe Aeg-Arg-, Z-Aeg(Z)-Arg-, 2-Naphthyl-$SO_2$-Arg-, o-Azidobenzoyl-Arg-, $N^2$-Boc-$N^6$-(1-Iminoäthyl)-Lys- oder $N^2$-Boc-$N^5$-(3a,4,5,6,7,7a-Hexahydro-3a,7a-dihydroxy -1H-benzimidazol-2-yl)-Orn- ist.

**3.** Verfahren nach Anspruch 1 worin R eine Gruppe R-2, insbesondere solche worin R-CO- die Gruppe p-(Aminomethyl)hydrocinnamoyl ist.

**4.** Verfahren nach Anspruch 1, worin R eine Gruppe R-3, insbesondere solche worin R-CO- die Gruppe p-Amidinohydrocinnamoyl, 3-(p-Amidinophenyl oder p-Guanidinophenyl)-alanyl, N-Z- oder N-Boc-3-(p-Amidinophenyl)alanyl, N-Boc-3-(p-Guanidinophenyl)alanyl, p-Amidinophenoxyacetyl, p-Amidinophenacetyl, 2-[(p-Amidinophenyl)-1,3-dioxolan--2-yl]acetyl, (p-Amidinobenzoyl)acetyl oder N-Boc-3-(p--Acetimidoylantinophenyl)alanyl ist.

**5.** Verfahren nach einem der Ansprüche 1-3, worin R' Wasserstoff, -CO-Val-OH, -CO-Ser-OH, -CO-Phe-OH, 1-Carboxy--2-(1-naphthyl)äthylidencarbamoyl, -CO-Ile-OH, Carboxyphenylcarbamoyl, Isobutylcarbamoyl, p-Fluorphenäthylcarbamoyl oder Carboxycyclopentancarbamoyl ist.

**6.** Verfahren nach Anspruch 1, wobei die hergestellte Verbindung

[3-(p-Amidinophenyl)-DL-alanyl]-Gly-Asp-Val-OH,
Z-Aeg(Z)-Arg-Gly-Asp-Val-OH,
Aeg-Arg-Gly-Asp-Val-OH,
Aeg-Arg-Gly-Asp-Ser-OH,
N-Aeg-Arg-Gly-Asp-Nal(1)-OH,
Aeg-Arg-Gly-Asp-Ile-OH,
[$N^2$-Boc-$N^6$-(1-Iminoäthyl)-L-lysyl]-Gly-Asp-Val-OH,
N-[(o-Azidobenzoyl)-Arg-Gly-Asp]-anthranilsäure.
$N^2$-Boc-$N^5$-(3a,4,5,6,7,7a-Hexahydro-3a,7a-dihydroxy-1H-benzimidazol-2-yl)-L-ornithyl]-Gly-Asp-Val-OH,
[N-Boc-3-(p-Guanidinophenyl)-DL-alanyl]-Gly-Asp-Val-OH,
[3-(p-Amidinophenyl)-DL-alanyl]-Gly-Asp-Nal(1)-OH,
[N-Boc-3-(p-Amidinophenyl)-DL-alanyl]-Gly-Asp-Val-OH,
(p-Amidinohydrocinnamoyl)-Gly-Asp-Nal(1)-OH,
[3-(p-Amidinophenyl)-D-alanyl]-Gly-Asp-Val-OH,
(p-Aminomethylhydrocinnamoyl)-Gly-Asp-Val-OH,
(p-Amidinohydrocinnamoyl)-Gly-Asp-Val-OH,
[3-(p-Amidinophenyl)-L-alanyl]-Gly-Asp-Val-OH,
(p-Amidinophenoxy)acetyl-Gly-Asp-Val-OH oder
(p-Amidinophenyl)acetyl-Gly-Asp-Val-OH ist.

**7.** Verfahren nach Anspruch 1, dadurch gekennzeichnet, dass man

a) die Abspaltung von Schutzgruppen mit einer Säure, mit einer Base oder durch Hydrierung in Gegenwart eines Edelmetallkatalysators, jeweils in einem Lösungsmittel, bei einer Temperatur zwischen etwa 0°C und 40°C durchführt, oder

b) ein Amin der Formel III mit Pyridinhydrochlorid in Gegenwart einer Base und von 1,1,3,3-Tetramethyl-2-[4-oxo--1,2,3-benzotriazin-3(4H)-yl]uroniumhexafluorphosphat, oder mit Naphthalin-2-sulfonylchlorid und einer Base, oder mit Z-Aeg(Z)-O-Su in Gegenwart einer Base, jeweils in einem Lösungsmittel, bei einer Temperatur bis zu etwa 40°C umsetzt, oder

c) ein Guanidinderivat der Formel IV mit 1,2-Cyclohexandion in einem Natriumboratpuffer unter einer inerten Atmosphäre bei einer Temperatur bis zu etwa 40°C umsetzt,

d) ein Nitril der Formel VI in einer alkoholischen Ammoniaklösung katalytisch hydriert oder

e) aus einer geschützten Gruppe R-CO- die Schutzgruppen abspaltet oder eine primäre Aminogruppe mit Di-t-butyldicarbonat in einem Lösungsmittel in Gegenwart einer Base bei einer Temperatur bis zu etwa 40°C umsetzt.

**8.** Verfahren zur Herstellung einer Verbindung der Formel

$$R^2\text{-CONH-CH}_2\text{-CONH-CH}(R^4)\text{-CH}_2\text{COOR}^3 \qquad \text{II}$$

worin

$R^2$ eine Gruppe der Formel

$$\text{-CH(NH-R}^a\text{)-(CH}_2\text{)}_{1\text{-}6}\text{-R}^5 \qquad \text{(R-1a)}$$

$$\text{-(T)}_{1\text{ oder }0}\text{-C}_6\text{H}_4\text{-CH}_2\text{-R}^6 \qquad \text{(R-2a)}$$

oder

$$-(T)_{1 \text{ oder } 0}-C_6H_4-R^7 \qquad \text{(R-3a)}$$

ist, in der
$R^5$ eine geschützte Guanidinogruppe oder eine Gruppe -NH-$R^b$,
$R^6$ eine geschützte Amino- oder Guanidinogruppe oder eine Gruppe -NH-$R^c$,
$R^7$ gegebenenfalls geschütztes Amidino oder Guanidino,
$R^3$ Wassecstoff oder eine leicht spaltbare Estergruppe,
$R^4$ die gleiche Bedeutung wie R' hat oder eine Gruppe -$COR^8$ ist, in der $R^8$ ein über die Aminogruppe gebundener leicht spaltbarer $\alpha$-Aminocarbonsäureester ist,
wobei, falls $R^3$ Wasserstoff ist und $R^4$ die gleiche Bedeutung wie R' hat, $R^2$ zumindest eine geschützte Guanidino-, Amino- oder Amidinogruppe $R^5$, $R^6$ bzw.
$R^7$ enthalten soll,
und $R^a$, $R^b$, $R^c$, R' und T die in Anspruch 1 angegebene Bedeutung haben,

dadurch gekennzeichnet, daß man

a) ein Nitril der Formel

$$NC-C_6H_4-(T)_{1 \text{ oder } 0}-CONH-CH_2-CONH-CH(R^4)-CH_2COOR^3 \qquad \text{VII}$$

über die entsprechenden Thioamide und S-Methyliminoester in eine Verbindung der Formel II überführt, in der $R^2$ eine Gruppe $-(T)_{1 \text{ oder } 0}-C_6H_4-C(NH)NH_2$ ist,

b) eine Verbindung der Formel

$$\begin{array}{l} NHCO-(T)_{1 \text{ oder } 0}-C_6H_4-NO_2 \\ | \\ CH_2-CONH-CH(R^4)-CH_2COOR^3 \end{array} \qquad \text{VIII}$$

über das entsprechende Amin und das entsprechende durch Nitro geschützte Guanidinderivat in eine Verbindung der Formel II überführt, in der $R^2$ eine Gruppe der Formel $-(T)_{1 \text{ oder } 0}-C_6H_4-NHC(NH)NH_2$ ist, oder

c) eine Verbindung der Formel II an einen Träger gebunden durch Festphasen-Synthese herstellt.

**9.** Verfahren zur Herstellung eines pharmazeutischen Präparates, insbesondere für die Behandlung oder Prophylaxe von Krankheiten. die durch die Bindung von adhäsiven Proteinen an Blutplättchen, sowie durch die Blutplättchenaggregation und die Zell-Zell-Adhäsion verursacht sind, dadurch gekennzeichnet, dass man eine Verbindung, erhältlich nach einem der Ansprüche 1-6 in eine galenische Darreichungsfrom bringt.

**10.** Verwendung einer Verbindung, erhältlich nach einem der Ansprüche 1-6 zur Herstellung von Arzneimitteln zur Behandlung oder Prophylaxe von Krankheiten, die durch die Bindung von adhäsiven Proteinen an Blutplättchen, sowie durch die Blutplättchenaggregation und die Zell-Zell-Adhäsion verursacht sind.

**Claims**

**Claims for the following Contracting States : AT, BE, CH, DE, DK, FR, GB, IT, LI, LU, NL, SE**

**1.** Glycine derivatives of the formula

$$R\text{-}CONH\text{-}CH_2\text{-}CONH\text{-}CH(R')\text{-}CH_2COOH \qquad (I)$$

wherein

R is a group of the formula

$$-CH(NH\text{-}R^a)\text{-}(CH_2)_{1\text{-}6}\text{-}NH\text{-}R^b \qquad (R\text{-}1)$$

$$-(T)_{1\ or\ 0}\text{-}C_6H_4\text{-}CH_2NH\text{-}R^c \qquad (R\text{-}2)$$

or

$$-(T)_{1\ or\ 0}\text{-}C_6H_4\text{-}(NH)_n\text{-}C(NH)\text{-}L \qquad (R\text{-}3)$$

and
$R^a$ is hydrogen, $-COO-C_{1-4}$-alkyl, Z, $-COC_6H_5$, $-COC_6H_4N_3$, $-SO_2C_6H_5$, $-SO_2$-naphthyl or $-COCH_2N(Y)-CH_2CH_2NH-Y$,
Y is hydrogen, Boc or Z,
$R^b$ is a group of the formula $-C(NH)(CH_2)_{0-3}-CH_3$ or

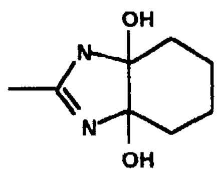

or, where
$R^a$ is a group of the formula $-COC_6H_4N_3$, $-SO_2C_6H_5$, $-SO_2$-naphthyl or $-COCH_2N(Y)-CH_2N(Y)-CH_2CH_2NH-Y$, also amidino,
$R^c$ is hydrogen or amidino,
n is the number 1 or 0,
L is amino,
T is a group of the formula $-(CH_2)-(O)_{1\ or\ 0}-$, $-CH=CH-$ or $-CH(R^d)-CH_2-$,
$R^d$ is hydrogen or $-NH-R^a$,
R' is hydrogen or $-CO-R^o$,
$R^o$ is amino, $-NH-C_{1-4}$-alkyl, $-NH(CH_2)_{1-4}-C_6H_5$, $-NH(CH_2)_{1-4}-C_6H_4$-Hal, $-NH-C_6H_4-COOH$, $-NH-C_6H_4-COO-C_{1-4}$-alkyl or the residue of an $\alpha$-aminocarboxylic acid attached via the amino group,

as well as hydrates or solvates and physiologically usable salts thereof.

2. Compounds according to claim 1, wherein R is a group of formula R-1, especially those in which R-CO- is the Aeg-Arg-, Z-Aeg(Z)-Arg-, 2-naphthyl-$SO_2$-Arg-, o-azido-benzoyl-Arg-, $N^2$-Boc-$N^6$-(1-iminoethyl)-Lys- or $N^2$-Boc-$N^5$-(3a,4,5,6,7,7a-hexahydro-3a,7a-dihydroxy-1H-benzimidazol-2-yl)-Orn- group.

3. Compounds according to claim 1, wherein R is a group R-2, especially those in which R-CO- is the p-(aminomethyl) hydrocinnamoyl group.

4. Compounds according to claim 1, wherein R is a group R-3, especially those in which R-CO- is the p-amidinohydrocinnamoyl, 3-(p-amidinophenyl or p-guanidino-phenyl)-alanyl, N-Z- or N-Boc-3-(p-amidinophenyl)alanyl, N-Boc-3-(p-guanidinophenyl)-alanyl, p-amidinophenoxyacetyl, p-amidinophenacetyl, 2-[(p-amidinophenyl)-1,3-dioxolan-2-yl]acetyl, (p-amidinobenzoyl)acetyl or N-Boc-3-(p-acetimidoylaminophenyl)alanyl group.

**5.** Compounds according to any one of claims 1-3, wherein R' is hydrogen, -CO-Val-OH, -CO-Ser-OH, -CO-Phe-OH, 1-carhoxy-2-(1-naphthyl)ethylidenecarbamoyl, -CO-Ile-OH, carboxyphenylcarbamoyl, isobutylcarbamoyl, p-fluorophenethylcarbamoyl or carboxycyclopentanecarbamoyl.

**6.** Compounds according to claim 1 from the following group:

[3-(p-Amidinophenyl)-DL-alanyl] -Gly-Asp-Val-OH,
Z-Aeg(Z)-Arg-Gly-Asp-Val-OH,
Aeg-Arg-Gly-Asp-Val-OH,
Aeg-Arg-Gly-Asp-Ser-OH,
N-Aeg-Arg-Gly-Asp-Nal(1)-OH,
Aeg-Arg-Gly-Asp-Ile-OH,
[$N^2$-Boc-$N^6$-(1-iminoethyl)-L-lysyl] -Gly-Asp-Val-OH,
N-[(o-azidobenzoyl)-Arg-Gly-Asp]-anthranilic acid,
$N^2$-Boc-$N^5$-(3a,4,5,6,7,7a-hexahydro-3a,7a-dihydroxy-1H-benzimidazol-2-yl)-L-ornithyl]-(fly-Asp-Val-OH,
[N-Boc-3-(p-guanidinophenyl)-DL-alanyl]-Gly-Asp-Val-OH,
[3-(p-amidinophenyl)-DL-alanyl]-Gly-Asp-Nal(1)-OH,
[N-Boc-3-(p-amidinophenyl)-DL-alanyl]-Gly-Asp-Val-OH,
(p-amidinohydrocinnamoyl)-Gly-Asp-Nal(1)-OH,
[3-(p-amidinophenyl)-D-alanyl]-Gly-Asp-Val-OH,
(p-aminomethylhydrocinnamoyl)-Gly-Asp-Val-OH,
(p-amidinohydrocinnamoyl)-Gly-Asp-Val-OH,
[3-(p-amidinophenyl)-L-alanyl]-Gly-Asp-Val-OH,
(p-amidinophenoxy)acetyl- Gly-Asp-Val-OH and
(p-amidinophenyl)acetyl-Gly-Asp-Val-OH.

**7.** Compounds of the formula

$$R^2\text{-CONH-}CH_2\text{-CONH-CH}(R^4)\text{-}CH_2COOR^3 \qquad II$$

wherein

$R^2$ is a group of the formula

$$\text{-CH(NH-}R^a)\text{-}(CH_2)_{1\text{-}6}\text{-}R^5 \qquad (R\text{-}1a)$$

$$\text{-(T)}_{1\text{ or }0}\text{-}C_6H_4\text{-}CH_2\text{-}R^6 \qquad (R\text{-}2a)$$

or

$$\text{-(T)}_{1\text{ or }0}\text{-}C_6H_4\text{-}R^7 \qquad (R\text{-}3a)$$

in which
$R^5$ is a protected guanidino group or a group -NH-$R^b$,
$R^6$ is a protected amino or guanidino group or a group -NH-$R^c$,
$R^7$ is optionally protected amidino or guanidino,
$R^3$ is hydrogen or a readily cleavable ester group,
$R^4$ has the same significance as R' or is a group -CO$R^8$ in which $R^8$ is an α-aminocarboxylic acid ester attached via the amino group,
whereby $R^2$ must contain at least one protected guanidino, amino or amidino group $R^5$, $R^6$ or $R^7$ where $R^3$ is hydrogen and $R^4$ has the same significance as R',

and $R^a$, $R^b$, $R^c$, R' and T have the significance given in claim 1.

**8.** Compounds according to any one of claims 1-6 for use as medicaments, especially as inhibitors of the binding of adhesive proteins to blood platelets, as well as of blood platelet aggregation and of cell-cell adhesion.

**9.** A process for the manufacture of the compounds according to any one of claims 1-6, characterized by

a) cleaving off the ester group(s) present and one or more protected amino, amidino or guanidino groups present in a compound of the formula

$$R^2\text{-CONH-CH}_2\text{-CONH-CH(}R^4\text{)-CH}_2\text{COO}R^3 \qquad \text{II}$$

wherein

$R^2$ is a group of the formula

$$\text{-CH(NH-}R^a\text{)-(CH}_2)_{1\text{-}6}\text{-}R^5 \qquad \text{(R-1a)}$$

$$\text{-(T)}_{1 \text{ or } 0}\text{-C}_6\text{H}_4\text{-CH}_2\text{-}R^6 \qquad \text{(R-2a)}$$

or

$$\text{-(T)}_{1 \text{ or } 0}\text{-C}_6\text{H}_4\text{-}R^7 \qquad \text{(R-3a)}$$

in which
$R^5$ is a protected guanidino group or a group -NH-$R^b$,
$R^6$ is a protected amino or guanidino group or a group -NH-$R^c$,
$R^7$ is optionally protected amidino or guanidino,
$R^3$ is hydrogen or a readily cleavable ester group,
$R^4$ has the same significance as R' or is a group -$COR^8$ in which $R^8$ is an $\alpha$-aminocarhoxylic acid ester attached via the amino group,
whereby $R^2$ must contain at least one protected guanidino, amino or amidino group $R^5$, $R^6$ or $R^7$ where $R^3$ is hydrogen and $R^4$ has the same significance as R',
and $R^a$, $R^b$ $R^c$ and T have the significance given in claim 1,

or

b) reacting an amine of the formula

$CH(NH_2)\text{-}(CH_2)_{1\text{-}6}\text{-}NH\text{-}C(NH)NH_2$
|
$CONH\text{-}CH_2\text{-}CONH\text{-}CH(R')\text{-}CH_2COOH$ III

with an agent which introduces the group $-COC_6H_5$, $-COC_6H_4N_3$, $-SO_2C_6H_5$, $-SO_2$-naphthyl or $-COCH_2N(Y)\text{-}CH_2CH_2NH\text{-}Y$, wherein R' and Y have the above significance, or

c) reacting a guanidine derivative of the formula

$$\begin{array}{l} CH(NH\text{-}R^9)\text{-}(CH_2)_{1\text{-}6}\text{-}NHC(NH)\text{-}NH_2 \\ | \\ CONH\text{-}CH_2\text{-}CONH\text{-}CH(R')\text{-}CH_2COOH \end{array} \qquad IV$$

wherein $R^9$ is $-COO-C_{1-4}$-alkyl, Z, $-COC_6H_5$, $-COC_6H_4N_3$, $-SO_2C_6H_5$, $-SO_2$-naphthyl or $-COCH_2N(Y')-CH_2CH_2NH-Y'$, in which Y' represents Boc or Z, and R' has the above significance, with 1,2-cyclohexanedione, or

d) converting the amino group in an amine of the formula

$$\begin{array}{l} NH\text{-}CH_2\text{-}CONH\text{-}CH_2\text{-}CONH\text{-}CH(R')\text{-}CH_2COOH \\ | \\ CO\text{-}(L)_{1\ or\ 0}\text{-}C_6H_4\text{-}(CH_2)_{1\ or\ 0}\text{-}NH_2 \end{array} \qquad V$$

wherein L is a group $-CH_2(O)_{1\ or\ 0}-$, $-CH=CH-$ or $-CH(NH-R^9)-CH_2-$ and R' and $R^9$ have the above significance, or

e) hydrogenating a nitrile of the formula

$$N{\equiv}C\text{-}C_6H_4\text{-}(T)_{1\ or\ 0}\text{-}CONH\text{-}CH_2\text{-}CONH\text{-}CH(R')\text{-}CH_2COOH \qquad VI$$

to the amine, or

f) if desired, functionally modifying reactive substituents present in the group R of a compound of formula 1, and

g) if desired, converting a compound of formula I into a salt or converting a salt of a compound of formula I into the free compound of formula I.

**10.** Pharmaceutical preparations, especially for the treatment or prophylaxis of illnesses which are caused by the binding of adhesive proteins to blood platelets as well as by blood platelet aggregation and cell-cell adhesion, containing a compound according to any one of claims 1-6 as the active ingredient.

**11.** The use of a compound according to any one of claims 1-6 for the production of medicaments for the treatment or prophylaxis of illnesses which are caused by the binding of adhesive proteins to blood platelets as well as by blood platelet aggregation and cell-cell adhesion.

**Claims for the following Contracting State : GR**

**1.** Glycine derivatives of the formula

$$R\text{-}CONH\text{-}CH_2\text{-}CONH\text{-}CH(R')\text{-}CH_2COOH \qquad (I)$$

wherein

R is a group of the formula

$$-CH(NH-R^a)-(CH_2)_{1-6}-NH-R^b \quad (R-1)$$

$$-(T)_{1\ or\ 0}-C_6H_4-CH_2NH-R^c \quad (R-2)$$

or

$$-(T)_{1\ or\ 0}-C_6H_4-(NH)_n-C(NH)-L \quad (R-3)$$

and

$R^a$ is hydrogen, -COO-$C_{1-4}$-alkyl, Z, $-COC_6H_5$, $-COC_6H_4N_3$, $-SO_2C_6H_5$, $-SO_2$-naphthyl or $-COCH_2N(Y)-CH_2CH_2NH-Y$,
Y is hydrogen, Boc or Z,
$R^b$ is a group of the formula $-C(NH)(CH_2)_{0-3}-CH_3$ or

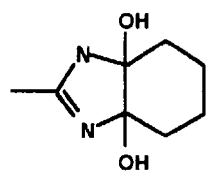

or, where
$R^a$ is a group of the formula $-COC_6H_4N_3$, $-SO_2C_6H_5$, $-SO_2$-naphthyl or $-COCH_2N(Y)-CH_2N(Y)-CH_2CH_2NH-Y$, also amidino,
$R^c$ is hydrogen or amidino,
n is the number 1 or 0,
L is amino,
T is a group of the formula $-(CH_2)-(O)_{1\ or\ 0}-$, -CH=CH- or $-CH(R^d)-CH_2-$,
$R^d$ is hydrogen or $-NH-R^a$,
R' is hydrogen or $-CO-R^o$,
$R^o$ is amino, -NH-$C_{1-4}$-alkyl, $-NH(CH_2)_{1-4}-C_6H_5$, $-NH(CH_2)_{1-4}-C_6H_4$-Hal, $-NH-C_6H_4-COOH$, $-NH-C_6H_4-COO-C_{1-4}$-alkyl or the residue of an $\alpha$-aminocarboxylic acid attached via the amino group,

as well as hydrates or solvates and physiologically usable salts thereof.

2. Compounds according to claim 1, wherein R is a group of formula R-1, especially those in which R-CO- is the Aeg-Arg-, Z-Aeg(Z)-Arg-, 2-naphthyl-$SO_2$-Arg-, o-azido-benzoyl-Arg-, $N^2$-Boc-$N^6$-(1-iminoethyl)-Lys- or $N^2$-Boc-$N^5$-(3a,4,5,6,7,7a-hexahydro-3a,7a-dihydroxy-1H-benzimidazol-2-yl)-Orn- group.

3. Compounds according to claim 1, wherein R is a group R-2, especially those in which R-CO- is the p-(aminomethyl) hydrocinnamoyl group.

4. Compounds according to claim 1, wherein R is a group R-3, especially those in which R-CO- is the p-amidinohydrocinnamoyl, 3-(p-amidinophenyl or p-guanidino-phenyl)-alanyl, N-Z- or N-Boc-3-(p-amidinophenyl)alanyl, N-Boc-3-(p-guanidinophenyl)-alanyl, p-amidinophenoxyacetyl, p-amidinophenacetyl, 2-[(p-amidinophenyl)-1,3-dioxolan-2-yl]acetyl, (p-amidinobenzoyl)acetyl or N-Boc-3-(p-acetimidoylaminophenyl)alanyl group.

5. Compounds according to any one of claims 1-3, wherein R' is hydrogen, -CO-Val-OH, -CO-Ser-OH, -CO-Phe-OH, 1-carboxy-2-(1-naphthyl)ethylidenecarbamoyl, -CO-Ile-OH, carboxyphenylcarbamoyl, isobutylcarbamoyl, p-fluorophenethylcarbamoyl or carboxycyclopentanecarbamoyl.

6. Compounds according to claim 1 from the following group:

[3-(p-Amidinophenyl)-DL-alanyl]-Gly-Asp-Val-OH,
Z-Aeg(Z)-Arg-Gly-Asp-Val-OH,
Aeg-Arg-Gly-Asp-Val-OH,
Aeg-Arg-Gly-Asp-Ser-OH,
N-Aeg-Arg-Gly-Asp-Nal(1)-OH,
Aeg-Arg-Gly-Asp-Ile-OH,
[$N^2$-Boc-$N^6$-(1-iminoethyl)-L-lysyl]-Gly-Asp-Val-OH,
N-[(o-azidohenzoyl) -Arg-Gly-Asp]-anthranilic acid,
$N^2$-Boc-$N^5$-(3a,4,5,6,7,7a-hexahydro-3a,7a-dihydroxy-1H-henzimidazol-2-yl)-L-ornithyl]-Gly-Asp-Val-OH,
[N-Boc-3-(p-guanidinophenyl)-DL-alanyl]-Gly-Asp-Val-OH,
[3-(p-amidinophenyl)-DL-alanyl]-Gly-Asp-Nal(1)-OH,
[N-Boc-3-(p-amidinophenyl)-DL-alanyl]-Gly-Asp-Val-OH,
(p-amidinohydrocinnamoyl)-Gly-Asp-Nal(1)-OH,
[3-(p-amidinophenyl)-D-alanyl]-Gly-Asp-Val-OH,
(p-aminomethylhydrocinnamoyl) -Gly-Asp-Val-OH,
(p-amidinohydrocinnamoyl) -Gly-Asp-Val-OH,
[3-(p-amidinophenyl) -L-alanyl]-Gly-Asp-Val-OH,
(p-amidinophenoxy)acetyl-Gly-Asp-Val-OH and
(p-amidinophenyl)acetyl-Gly-Asp-Val-OH.

**7.** Compounds of the formula

$$R^2\text{-CONH-CH}_2\text{-CONH-CH(R}^4\text{)-CH}_2\text{COOR}^3 \qquad \text{II}$$

wherein

$R^2$ is a group of the formula

$$\text{-CH(NH-R}^a\text{)-(CH}_2\text{)}_{1\text{-}6}\text{-R}^5 \qquad \text{(R-1a)}$$

$$\text{-(T)}_{1 \text{ or } 0}\text{ -C}_6\text{H}_4\text{-CH}_2\text{-R}^6 \qquad \text{(R-2a)}$$

or

$$\text{-(T)}_{1 \text{ or } 0}\text{-C}_6\text{H}_4\text{-R}^7 \qquad \text{(R-3a)}$$

in which
$R^5$ is a protected guanidino group or a group -NH-$R^b$,
$R^6$ is a protected amino or guanidino group or a group -NH-$R^c$,
$R^7$ is optionally protected amidino or guanidino,
$R^3$ is hydrogen or a readily cleavable ester group,
$R^4$ has the same significance as R' or is a group -$COR^8$ in which $R^8$ is an α-aminocarboxylic acid ester attached via the amino group,
whereby $R^2$ must contain at least one protected guanidino, amino or amidino group $R^5$, $R^6$ or $R^7$ where $R^3$ is hydrogen and $R^4$ has the same significance as R',
and $R^a$, $R^b$ $R^c$, R' and T have the significance given in claim 1.

**8.** Compounds according to any one of claims 1-6 for use as medicaments, especially as inhibitors of the binding of adhesive proteins to blood platelets as well as of blood platelet aggregation and of cell-cell adhesion.

**9.** A process for the manufacture of the compounds according to any one of claims 1-6, characterized by

a) cleaving off the ester group(s) present and one or more protected amino, amidino or guanidino groups

present in a compound of the formula

$$R^2\text{-CONH-CH}_2\text{-CONH-CH}(R^4)\text{-CH}_2\text{COOR}^3 \qquad II$$

wherein

$R^2$ is a group of the formula

$$\text{-CH(NH-}R^a\text{)-(CH}_2)_{1\text{-}6}\text{-}R^5 \qquad (R\text{-}1a)$$

$$\text{-(T)}_{1\text{ or }0}\text{-C}_6H_4\text{-CH}_2\text{-}R^6 \qquad (R\text{-}2a)$$

or

$$\text{-(T)}_{1\text{ or }0}\text{-C}_6H_4\text{-}R^7 \qquad (R\text{-}3a)$$

in which
$R^5$ is a protected guanidino group or a group -NH-$R^b$,
$R^6$ is a protected amino or guanidino group or a group -NH-$R^c$,
$R^7$ is optionally protected amidino or guanidino,
$R^3$ is hydrogen or a readily cleavable ester group,
$R^4$ has the same significance as R' or is a group -COR$^8$ in which $R^8$ is an α-aminocarboxylic acid ester attached via the amino group,
whereby $R^2$ must contain at least one protected guanidino, amino or amidino group $R^5$, $R^6$ or $R^7$ where $R^3$ is hydrogen and $R^4$ has the same significance as R',
and $R^a$, $R^b$, $R^c$, R' and T have the significance given in claim 1,

or

b) reacting an amine of the formula

CH(NH$_2$)-(CH$_2$)$_{1\text{-}6}$-NH-C(NH)NH$_2$
|
CONH-CH$_2$-CONH-CH(R')-CH$_2$COOH — III

with an agent which introduces the group $-COC_6H_5$, $-COC_6H_4N_3$, $-SO_2C_6H_5$, $-SO_2$-naphthyl or $-COCH_2N(Y)-CH_2CH_2NH-Y$, wherein R' and Y have the above significance, or

c) reacting a guanidine derivative of the formula

CH(NH-R$^9$)-(CH$_2$)$_{1\text{-}6}$-NHC(NH)-NH$_2$
|
CONH-CH$_2$-CONH-CH(R')-CH$_2$COOH — IV

wherein $R^9$ is -COO-$C_{1\text{-}4}$-alkyl, Z, $-COC_6H_5$, $-COC_6H_4N_3$, $-SO_2C_6H_5$, $-SO_2$-naphthyl or $-COCH_2N(Y')-CH_2CH_2NH-Y'$, in which Y' represents Boc or Z, and R' has the above significance,
with 1,2-cyclohexanedione, or

d) converting the amino group in an amine of the formula

$$\begin{array}{l} NH\text{-}CH_2\text{-}CONH\text{-}CH_2\text{-}CONH\text{-}CH(R')\text{-}CH_2COOH \\ | \\ CO\text{-}(L)_{1\ or\ 0}\text{-}C_6H_4\text{-}(CH_2)_{1\ or\ 0}\text{-}NH_2 \end{array} \qquad V$$

wherein L is a group $-CH_2(O)_{1\ or\ 0}-$, $-CH=CH-$ or $-CH(NH-R^9)-CH_2-$ and R' and $R^9$ have the above significance, or

e) hydrogenating a nitrile of the formula

$$N{\equiv}C\text{-}C_6H_4\text{-}(T)_{1\ or\ 0}\text{-}CONH\text{-}CH_2\text{-}CONH\text{-}CH(R')\text{-}CH_2COOH \qquad VI$$

to the amine, or

f) if desired, functionally modifying reactive substituents present in the group R of a compound of formula I, and

g) if desired, converting a compound of formula I into a salt or converting a salt of a compound of formula I into the free compound of formula I.

**10.** A process for the production of a pharmaceutical preparation, especially for the treatment or prophylaxis of illnesses which are caused by the binding of adhesive proteins to blood platelets as well as by blood platelet aggregation and cell-cell adhesion, containing a compound according to any one of claims 1-6 as the active ingredient.

**11.** The use of a compound according to any one of claims 1-6 for the production of medicaments for the treatment or prophylaxis of illnesses which are caused by the binding of adhesive proteins to blood platelets as well as by blood platelet aggregation and cell-cell adhesion.

**Claims for the following Contracting State : ES**

**1.** A process for the manufacture of glycine derivatives of the formula

$$R\text{-}CONH\text{-}CH_2\text{-}CONH\text{-}CH(R')\ \text{-}CH_2COOH \qquad (I)$$

wherein

R is a group of the formula

$$-CH(NH-R^a)-(CH_2)_{1-6}-NH-R^b \qquad (R-1)$$

$$-(T)_{1\ or\ 0}-C_6H_4-CH_2NH-R^c \qquad (R-2)$$

or

$$-(T)_{1\ or\ 0}-C_6H_4-(NH)_n-C(NH)-L \qquad (R-3)$$

and

$R^a$ is hydrogen, $-COO-C_{1-4}$-alkyl, Z, $-COC_6H_5$, $-COC_6H_4N_3$, $-SO_2C_6H_5$, $-SO_2$-naphthyl or $-COCH_2N(Y)-CH_2CH_2NH-Y$,
Y is hydrogen, Boc or Z,
$R^b$ is a group of the formula $-C(NH)(CH_2)_{0-3}-CH_3$ or

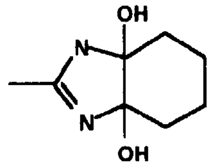

or, where
$R^a$ is a group of the formula $-COC_6H_4N_3$, $-SO_2C_6H_5$, $-SO_2$-naphthyl or $-COCH_2N(Y)-CH_2N(Y)-CH_2CH_2NH-Y$, also amidino,
$R^c$ is hydrogen or amidino,
n is the number 1 or 0,
L is amino,
T is a group of the formula $-(CH_2)-(O)_{1\text{ or }0}-$, -CH=CH- or $-CH(R^d)-CH_2-$,
$R^d$ is hydrogen or $-NH-R^a$,
R' is hydrogen or $-CO-R^o$,
$R^o$ is amino, $-NH-C_{1-4}$-alkyl, $-NH(CH_2)_{1-4}-C_6H_5$, $-NH(CH_2)_{1-4}-C_6H_4$-Hal, $-NH-C_6H_4-COOH$, $-NH-C_6H_4-COO-C_{1-4}$-alkyl or the residue of an α-aminocarboxylic acid attached via the amino group,

as well as hydrates or solvates and physiologically usable salts thereof, characterized by

a) cleaving off the ester group(s) present and one or more protected amino, amidino or guanidino groups present in a compound of the formula

$$R^2-CONH-CH_2-CONH-CH(R^4)-CH_2COOR^3 \qquad II$$

wherein

$R^2$ is a group of the formula

$$-CH(NH-R^a)-(CH_2)_{1-6}-R^5 \qquad (R-1a)$$

$$-(T)_{1\text{ or }0}-C_6H_4-CH_2-R^6 \qquad (R-2a)$$

or

$$-(T)_{1\text{ or }0}-C_6H_4-R^7 \qquad (R-3a)$$

in which
$R^5$ is a protected guanidino group or a group $-NH-R^b$,
$R^6$ is a protected amino or guanidino group or a group $-NH-R^c$,
$R^7$ is optionally protected amidino or guanidino,
$R^3$ is hydrogen or a readily cleavable ester group,
$R^4$ has the same significance as R' or is a group $-COR^8$ in which $R^8$ is an α-aminocarboxylic acid ester attached via the amino group,
whereby $R^2$ must contain at least one protected guanidino, amino or amidino group $R^5$, $R^6$ or $R^7$ where

$R^3$ is hydrogen and $R^4$ has the same significance as R',
and $R^a$, $R^b$ $R^c$, R' and T have the significance given above,

or

b) reacting an amine of the formula

$$\begin{array}{l} CH(NH_2)\text{-}(CH_2)_{1\text{-}6}\text{-}NH\text{-}C(NH)NH_2 \\ | \\ CONH\text{-}CH_2\text{-}CONH\text{-}CH(R')\text{-}CH_2COOH \end{array} \qquad III$$

with an agent which introduces the group $-COC_6H_5$, $-COC_6H_4N_3$, $-SO_2C_6H_5$, $-SO_2$-naphthyl or $-COCH_2N(Y)-CH_2CH_2NH-Y$, wherein R' and Y have the above significance, or

c) reacting a guanidine derivative of the formula

$$\begin{array}{l} CH(NH\text{-}R^9)\text{-}(CH_2)_{1\text{-}6}\text{-}NHC(NH)\text{-}NH_2 \\ | \\ CONH\text{-}CH_2\text{-}CONH\text{-}CH(R')\text{-}CH_2COOH \end{array} \qquad IV$$

wherein $R^9$ is $-COO-C_{1-4}$-alkyl, Z, $-COC_6H_5$, $-COC_6H_4N_3$, $-SO_2C_6H_5$, $-SO_2$-naphthyl or $-COCH_2N(Y')-CH_2CH_2NH-Y'$, in which Y' represents Boc or Z, and R' has the above significance,
with 1,2-cyclohexanedione, or

d) converting the amino group in an amine of the formula

$$\begin{array}{l} NH\text{-}CH_2\text{-}CONH\text{-}CH_2\text{-}CONH\text{-}CH(R')\text{-}CH_2COOH \\ | \\ CO\text{-}(L)_{1\text{ or }0}\text{-}C_6H_4\text{-}(CH_2)_{1\text{ or }0}\text{-}NH_2 \end{array} \qquad V$$

wherein L is a group $-CH_2(O)_{1\text{ or }0}-$, $-CH=CH-$ or $-CH(NH-R^9)-CH_2-$ and R' and $R^9$ have the above significance,
or

e) hydrogenating a nitrile of the formula

$$N{\equiv}C\text{-}C_6H_4\text{-}(T)_{1\text{ or }0}\text{-}CONH\text{-}CH_2\text{-}CONH\text{-}CH(R')\text{-}CH_2COOH \qquad VI$$

to the amine, or

f) if desired, functionally modifying reactive substituents present in the group R of a compound of formula I, and

g) if desired, converting a compound of formula I into a salt or converting a salt of a compound of formula I into the free compound of formula I.

**2.** A process according to claim 1, wherein R is a group of formula R-1, especially those in which R-CO- is the Aeg-Arg-, Z-Aeg(7)-Arg-, 2-naphthyl-$SO_2$-Arg-, o-azido-benzoyl-Arg-, $N^2$-Boc-$N^6$-(1-iminoethyl)-Lys- or $N^2$-Boc-$N^5$-(3a,4,5,6,7,7a-hexahydro-3a,7a-dihydroxy-1H-benzimidazol-2-yl)-Orn- group.

3. A process according to claim I, wherein R is a group R-2, especially those in which R-CO- is the p-(aminomethyl) hydrocinnamoyl group.

4. A process according to claim 1, wherein R is a group R-3, especially those in which R-CO- is the p-amidinohydrocinnamoyl, 3-(p-amidinophenyl or p-guanidino-phenyl)-alanyl, N-Z- or N-Boc-3-(p-amidinophenyl)alanyl, N-Boc-3-(p-guanidinophenyl)-alanyl, p-amidinophenoxyacetyl, p-amidinophenacetyl, 2-[(p-amidinophenyl)-1,3-dioxolan-2-yl]acetyl, (p-amidinobenzoyl)acetyl or N-Boc-3-(p-acetimidoylaminophenyl)alanyl group.

5. A process according to any one of claims 1-3, wherein R' is hydrogen, -CO-Val-OH, -CO-Ser-OH, -CO-Phe-OH, 1-carboxy-2-(1-naphthyl)ethylidenecarbamoyl, -Co-Ile-OH, carboxyphenylcarbamoyl, isobutylcarbamoyl, p-fluorophenethylcarbamoyl or carboxycyclopentanecarbamoyl.

6. A process according to claim 1, wherein the compound manufactured is

[3-(p-Amidinophenyl)-DL-alanyl]-Gly-Asp-Val-OH,
Z-Aeg(Z)-Arg-Gly-Asp-Val-OH,
Aeg-Arg-Gly-Asp-Val-OH,
Aeg-Arg-Gly-Asp-Ser-OH,
N-Aeg-Arg-Gly-Asp-Nal(1)-OH,
Aeg-Arg-Gly-Asp-Ile-OH,
[$N^2$-Boc-$N^6$-(1-iminoethyl)-L-lysyl]-Gly-Asp-Val-OH,
N-[(o-azidobenzoyl)-Arg-Gly-Asp]-anthranilic acid,
$N^2$-Boc-$N^5$-(3a,4,5,6,7,7a-hexahydro-3a,7a-dihydroxy-1H-benzimidazol-2-yl)-L-ornithyl]-Gly-Asp-Val-OH,
[N-Boc-3-(p-guanidinophenyl)-DL-alanyl]-Gly-Asp-Val-OH,
[3-(p-amidinophenyl)-DL-alanyl]-Gly-Asp-Nal(1)-OH,
[N-Boc-3-(p-amidinophenyl)-DL-alanyl]-Gly-Asp-Val-OH,
(p-amidinohydrocinnamoyl)-Gly-Asp-Nal(1)-OH,
[3-(p-amidinophenyl)-D-alanyl]-Gly-Asp-Val-OH,
(p-aminomethylhydrocinnamoyl) -Gly-Asp-Val-OH,
(p-amidinohydrocinnamoyl)-Gly-Asp-Val-OH,
[3-(p-amidinophenyl)-L-alanyl]-Gly-Asp-Val-OH,
(p-amidinophenoxy)acetyl- Gly-Asp-Val-OH or
(p-amidinophenyl)acetyl-Gly-Asp-Val-OH.

7. A process according to claim 1, characterized in that

a) the cleavage of protecting groups is carried out with an acid, with a base or by hydrogenation in the presence of a noble metal catalyst, in each case in a solvent, at a temperature between about 0°C and 40°C, or
b) an amine of formula III is reacted with pyridine hydrochloride in the presence of a base and of 1,1,3,3-tetramethyl-2-[4-oxo-1,2,3-benzotriazin-3(4H)-yl]uronium hexafluorophosphate or with naphthalene-2-sulphonyl chloride and a base or with Z-Aeg(Z)-O-Su in the presence of a base, in each case in a solvent, at a temperature up to about 40°C, or
c) a guanidine derivative of formula IV is reacted with 1,2-cyclohexanedione in a sodium borate buffer under an inert atmosphere at a temperature up to about 40°C,
d) a nitrile of formula VI is catalytically hydrogenated in an alcoholic ammonia solution, or
e) the protecting groups are cleaved off from a protected group R-CO- or a primary amino group is reacted with di-t-butyl dicarbonate in a solvent in the presence of a base at a temperature up to about 40°C.

8. A process for the preparation of a compound of the formula

$$R^2\text{-CONH-CH}_2\text{-CONH-CH(}R^4\text{)-CH}_2\text{COOR}^3 \qquad II$$

wherein

$R^2$ is a group of the formula

$$-CH(NH-R^a)-(CH_2)_{1-6}-R^5 \quad \text{(R-1a)}$$

$$-(T)_{1 \text{ or } 0}-C_6H_4-CH_2-R^6 \quad \text{(R-2a)}$$

or

$$-(T)_{1 \text{ or } 0}-C_6H_4-R^7 \quad \text{(R-3a)}$$

in which
$R^5$ is a protected guanidino group or a group -NH-$R^b$,
$R^6$ is a protected amino or guanidino group or a group -NH-$R^c$,
$R^7$ is optionally protected amidino or guanidino,
$R^3$ is hydrogen or a readily cleavable ester group,
$R^4$ has the same significance as R' or is a group -$COR^8$ in which $R^8$ is an $\alpha$-aminocarboxylic acid ester attached via the amino group,
whereby $R^2$ must contain at least one protected guanidino, amino or amidino group $R^5$, $R^6$ or $R^7$ where $R^3$ is hydrogen and $R^4$ has the same significance as R',
and $R^a$, $R^b$, $R^c$, R' and T have the significance given in claim 1,

characterized by

a) converting a nitrile of the formula

$$NC-C_6H_4-(T)_{1 \text{ or } 0}-CONH-CH_2-CONH-CH(R^4)-CH_2COOR^3 \quad \text{VII}$$

via the corresponding thioamide and S-methylimino ester into a compound of formula II in which $R^2$ is a group $-(T)_{1 \text{ or } 0}-C_6H_4-C(NH)NH_2$,

b) converting a compound of the formula

$$\begin{array}{l} NHCO-(T)_{1 \text{ or } 0}-C_6H_4-NO_2 \\ | \\ CH_2-CONH-CH(R^4)-CH_2COOR^3 \end{array} \quad \text{VIII}$$

via the corresponding amine and the corresponding nitro-protected guanidine derivative into a compound of formula II in which $R^2$ is a group of the formula $-(T)_{1 \text{ or } 0}-C_6H_4-NHC(NH)NH_2$, or

c) preparing a compound of formula II on a carrier bonded by solid phase synthesis.

**9.** A process for the production of a pharmaceutical preparation, especially for the treatment or prophylaxis of illnesses which are caused by the binding of adhesive proteins to blood platelets as well as by blood platelet aggregation and cell-cell adhesion, characterized by bringing a compound obtainable according to any one of claims 1-6 into a galenical administration form.

**10.** The use of a compound obtainable according to any one of claims 1-6 for the production of medicaments for the treatment or prophylaxis of illnesses which are caused by the binding of adhesive proteins to blood platelets as well as by blood platelet aggregation and cell-cell adhesion.

## Revendications

**Revendications pour les Etats contractants suivants : AT, BE, CH, DE, DK, FR, GB, IT, LI, LU, NL, SE**

1. Dérivés de glycine de formule

$$R\text{-}CONH\text{-}CH_2\text{-}CONH\text{-}CH(R')\text{-}CH_2COOH \qquad I$$

dans laquelle

R est un groupe de formule

$$-CH(NH\text{-}R^a)\text{-}(CH_2)_{1\text{-}6}\text{-}NH\text{-}R^b \qquad (R\text{-}1)$$

$$-(T)_{1\ ou\ 0}\text{-}C_6H_4\text{-}CH_2NH\text{-}R^c \qquad (R\text{-}2)$$

ou

$$-(T)_{1\ ou\ 0}\ C_6H_4\text{-}(NH)_n\text{-}C(NH)\text{-}L \qquad (R\text{-}3)$$

et
$R^a$ représente un atome d'hydrogène ou un groupe -COO-alkyle($C_{1-4}$), Z, -$COC_6H_5$, -$COC_6H_4N_3$, -$SO_2C_6H_5$, -$SO_2$-naphtyle ou -$COCH_2N(Y)$-$CH_2CH_2NH$-Y,
Y représentant un atome d'hydrogène ou le groupe Boc ou Z,
$R^b$ représente un groupe de formule -C(NH)$(CH_2)_{0-3}$ ou

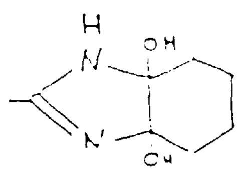

ou, lorsque $R^a$ est un groupe de formule -$COC_6H_4N_3$, -$SO_2C_6H_5$, $SO_2$-naphtyle ou -$COCH_2N(Y)$-$CH_2CH_2NH$-Y, représente également le groupe amidino,
$R^c$ représente un atome d'hydrogène ou le groupe amidino,
n représente le nombre 1 ou 0,
L représente le groupe amino,
T représente un groupe de formule -$CH_2$-$(0)_1$ ou $_0$-, -CH=CH- ou -CH($R^d$)-$CH_2$-,
$R^d$ représentant un atome d'hydrogène ou -NH-$R^a$,
R' représentant un atone d'hydrogène ou -CO-$R^o$,
$R^o$ représentant un groupe amino, NH-alkyle($C_{1-4}$), NH$(CH_2)_{1-4}$ -$C_6H_5$, -NH$(CH_2)_{1-4}$-$C_6H_4$-Hal, -NH-$C_6H_4$-COOH, -NH-$C_6H_4$-COO-alkyle($C_{1-4}$) ou le reste d'un acide $\alpha$-aminocarboxylique rattaché par le groupe amino, et hydrates ou produits de solvatation et sels physiologiquement utilisables de tels composés.

2. Composés selon la revendication 1, dans lesquels R est un groupe de formule R-1, en particulier ceux dans lesquels R-CO- est le groupe Aeg-Arg, Z-Aeg(Z)-Arg-, 2-naphtyl-$SO_2$Arg-, o-azido-benzoyl-Arg-, $N^2$-Boc-$N^6$-(1-iminoéthyl)-Lys- ou $N^2$-Boc-$N^5$-(3a,4,5,6,7,7a-hexahydro-3a,7a,dihydroxy-1H-benzimidazol-2-yl)-Orn.

**3.** Composés selon la revendication 1, dans lesquels R est un groupe R-2, en particulier ceux dans lesquels R-CO- est le groupe p-(aminométhyl)hydrocinnamoyle.

**4.** Composés selon la revendication 1, dans lesquels R est un groupe R-3, en particulier ceux dans lesquels R-CO- est le groupe p-amidinohydrocinnamoyle, 3-(p-amidinophényl ou p-guanidinophényl)alanyle, N-Z- ou N-Boc-3-(p-amidinophényl)alanyle, N-Boc-3-(p-guanidinophényl)-alanyle, p-amidinophénoxyacétyle, p-amidinophénacétyle, 2-[(p-amidinophényl)-1,3-dioxolann-2-yl]-acétyle, (p-amidinobenzoyl)acétyle ou N-Boc-3-(p-acétimidoylamino-phényl)alanyle.

**5.** Composés selon l'une des revendications 1 à 3, dans lesquels R' représente un atome d'hydrogène ou le groupe -CO-Val-OH, -CO-Ser-OH, -CO-Phe-OH, 1-carboxy-2-(1-naphtyl)éthylidènecarbamoyle, -CO-Ile-OH, carboxyphénylcarbamoyle, isobutylcarbamoyle, p-fluorophénéthylcarbamoyle ou carboxycyclopentanecarbamoyle.

**6.** Composés selon la revendication 1, choisi dans l'ensemble constitué par les composés suivants:

[3-(p-amidinophényl)-DL-alanyl]-Gly-Asp-Val-OH,
Z-Aeg(Z)-Arg-Gly-Asp-Val-OH,
Aeg-Arg-Gly-Asp-Val-OH,
Aeg-Arg-Gly-Asp-Ser-OH,
N-Aeg-Arg-Gly-Asp-Nal(1)-OH,
Aeg-Arg-Gly-Asp-Ile-OH,
[$N^2$-Boc-$N^6$-(1-iminoéthyl)-L-lysyl]-Gly-Asp-Val-OH, acide N-[(o-azidobenzoyl)-Arg-Gly-Asp]anthranilique,
$N^2$-Boc-$N^5$-(3a,4,5,6,7,7a-hexahydro-3a,7a,-dihydroxy-1H-benzimidazol-2-yl)-L-ornithyl]-Gly-Asp-Val-OH,
[N-Boc-3-(p-guanidinophényl)-DL-alanyl]-Gly-Asp-Val-OH,
[3-(p-amidinophényl)-DL-alanyl]-Gly-Asp-Nal(1)-OH,
[N-Boc-3-(p-amidinophényl)-DL-alanyl]-Gly-Asp-Val-OH,
(p-amidinohydrocinnamoyl)-Gly-Asp-Nal(1)-OH,
[3-(p-amidinophényl)-D-alanyl]-Gly-Asp-Val-OH,
(p-aminométhylhydrocinnamoyl)-Gly-Asp-Val-OH,
(p-amidinohydrocinnamoyl)-Gly-Asp-Val-OH,
[3-(p-amidinophényl)-L-alanyl]-Gly-Asp-Val-OH,
(p-amidinophénoxy)acétyl-Gly-Asp-Val-OH et
(p-amidinophényl)acétyl-Gly-Asp-Val-OH.

**7.** Composés de formule

$$R^2\text{-CONH-CH}_2\text{-CONH-CH}(R^4)\text{-CH}_2\text{COOR}^3 \qquad \text{II}$$

dans laquelle

$R^2$ est un groupe de formule

$$\text{-CH(NH-R}^a)\text{-(CH}_2)_{1\text{-}6}\text{-R}^5 \qquad \text{(R-1a)}$$

$$\text{-(T)}_{1 \text{ ou } 0}\text{-C}_6\text{H}_4\text{-CH}_2\text{-R}^6 \qquad \text{(R-2a)}$$

ou

$$\text{-(T)}_{1 \text{ ou } 0}\text{ -C}_6\text{H}_4\text{-R}^7 \qquad \text{(R-3a)}$$

où

$R^5$ représente un groupe guanidino protégé ou un groupe -NH-$R^b$,

$R^6$ représente un groupe amino ou guanidino protégé ou un groupe -NH-$R^c$,
$R^7$ représente un groupe amidino ou guanidino éventuellement protégé,

$R^3$ représente un atome d'hydrogène ou un groupe ester aisément séparable,
$R^4$ a la mène signification que R' ou est un groupe -$COR^8$ dans lequel $R^8$ est un ester d'acide α-aminocarboxylique aisément séparable, relié par le groupe amino,

lorsque $R^3$ est un atome d'hydrogène et $R^4$ a la même signification que R', $R^2$ devant contenir au moins un groupe guanidino, amino ou amidino protégé $R^5$, $R^6$ ou $R^7$,
et $R^a$, $R^b$, $R^c$, R' et T ont les significations données dans la revendication 1.

**8.** Composés selon l'une des revendications 1 à 6, pour utilisation en tant que médicament, en particulier en tant qu'inhibiteur de la fixation de protéines adhésives à des plaquettes sanguines, ainsi que de l'agrégation plaquettaire et de l'adhésion de cellule à cellule.

**9.** Procédé pour la préparation des composés selon l'une des revendication 1 à 6, caractérisé en ce que

a) dans un composé de formule

$$R^2\text{-CONH-CH}_2\text{-CONH-CH}(R^4)\text{-CH}_2\text{COOR}^3 \qquad \text{II}$$

dans laquelle

$R^2$ est un groupe de formule

$$\text{CH(NH-}R^a\text{)-(CH}_2)_{1\text{-}6}\text{-}R^5 \qquad \text{(R-1a)}$$

$$\text{-(T)}_{1\text{ ou }0}\text{-C}_6\text{H}_4\text{-CH}_2\text{-}R^6 \qquad \text{(R-2a)}$$

ou

$$\text{-(T)}_{1\text{ ou }0}\text{-C}_6\text{H}_4\text{-}R^7 \qquad \text{(R-3a)}$$

où

$R^5$ représente un groupe guanidino protégé ou un groupe -NH-$R^b$,
$R^6$ représente un groupe amino ou guanidino protégé ou un groupe -NH-$R^c$,
$R^7$ représente un groupe amidino ou guanidino éventuellement protégé,

$R^3$ représente un atome d'hydrogène ou un groupe ester aisément séparable,
$R^4$ a la même signification que R' ou est un groupe -$COR^8$ dans lequel $R^8$ est un ester d'acide α-aminocarboxylique aisément séparable, relié par le groupe amino,

lorsque $R^3$ est un atome d'hydrogène et $R^4$ a la même signification que R', $R^2$ devant contenir au moins un groupe guanidino, amino ou amidino protégé $R^5$, $R^6$ ou $R^7$,
et $R^a$, $R^b$ $R^c$, R' et T ont les significations données dans la revendication 1,
on élimine le(s) groupe(s) ester présent(s) et un ou plusieurs groupes amino, amidino ou guanidino protégés présents, ou
b) on fait réagir une amine de formule

$$\begin{array}{l} CH(NH_2)-(CH_2)_{1-6}-NH-C(NH)NH_2 \\ | \\ CONH-CH_2-CONH-CH(R')-CH_2COOH \end{array} \qquad III$$

avec un agent introduisant le groupe $-COC_6H_5$, $-COC_6H_4N_3$, $-SO_2C_6H_5$, $-SO_2$-naphtyle ou $-COCH_2N(Y)-CH_2CH_2NH-Y$, R' et Y ayant les significations données plus haut, ou

c) on fait réagir avec de la 1,2-cyclohexanedione un dérivé de guanidine de formule

$$\begin{array}{l} CH(NH-R^9)-(CH_2)_{1-6}-NHC(NH)-NH_2 \\ | \\ CONH-CH_2-CONH-CH(R')-CH_2COOH \end{array} \qquad IV$$

dans laquelle $R^9$ représente un groupe -COO-alkyle($C_{1-4}$), Z, $-COC_6H_5$, $-COC_6H_4N_3$, $-SO_2C_6H_5$, $-SO_2$-naphtyle ou $-COCH_2N(Y')-CH_2CH_2NH-Y'$, dans lequel Y' représente le groupe Boc ou Z

et R' a la signification donnée plus haut, ou

d) dans une amine de formule

$$\begin{array}{l} NH-CH_2-CONH-CH_2-CONH-CH(R')-CH_2COOH \\ | \\ CO-(L)_{1\ ou\ 0}-C_6H_4-(CH_2)_{1\ ou\ 0}-NH_2 \end{array} \qquad V$$

dans laquelle L représente un groupe $-CH_2(O)_1$ ou $_0$-, -CH=CH- ou $-CH(NH-R^9)-CH_2-$, et R' et $R^9$ ont les significations données plus haut,

on convertit le groupe amino en un groupe guanidino ou

e) ou soumet un nitrile de formule

$$N{\equiv}C-C_6H_4-(T)_{1\ ou\ 0}-CONH-CH_2-CONH-CH(R')-CH_2COOH \qquad VI$$

à une hydrogénation en l'amine,

f) si on le désire, on modifie la fonction d'un substituant réactif contenu dans le groupe R d'un composé de formule I, et

g) si on le désire, on convertit un composé de formule I en un sel ou on convertit un sel d'un composé de formule I en le composé libre de formule I.

**10.** Compositions pharmaceutiques, en particulier pour le traitement ou la prophylaxie de maladies qui sont causées par la fixation de protéines adhésives à des plaquettes sanguines, ainsi que par l'agrégation plaquettaire et l'adhésion de cellule à cellule, contenant en tant que substance active un composé selon l'une des revendications 1 à 6.

**11.** Utilisation d'un composé selon l'une des revendications 1 à 6, pour la fabrication de médicaments destinés au traitement ou à la prophylaxie de maladies qui sont causées par la fixation de protéines adhésives à des plaquettes sanguines, ainsi que par l'agrégation plaquettaire et l'adhésion de cellule à cellule.

**Revendications pour l'Etat contractant suivant : GR**

**1.** Dérivés de glycine de formule

$$R-CONH-CH_2-CONH-CH(R')-CH_2COOH \qquad I$$

dans laquelle

R est un groupe de formule

$$-CH(NH-R^a)-(CH_2)_{1-6}-NH-R^b \quad (R-1)$$

$$-(T)_{1\ ou\ 0}-C_6H_4-CH_2NH-R^c \quad (R-2)$$

ou

$$-(T)_{1\ ou\ 0}-C_6H_4-(NH)_n-C(NH)-L \quad (R-3)$$

et
$R^a$ représente un atome d'hydrogène ou un groupe -COO-alkyle($C_{1-4}$), Z, $-COC_6H_5$, $-COC_6H_4N_3$, $-SO_2C_6H_5$, $-SO_2$-naphtyle ou $-COCH_2N(Y)-CH_2CH_2NH-Y$,
Y représentant un atome d'hydrogène ou le groupe Boc ou Z,
$R^b$ représente un groupe de formule $-C(NH)(CH_2)_{0-3}$ ou

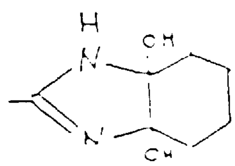

ou, lorsque $R^a$ est un groupe de formule $-COC_6H_4N_3$, $-SO_2C_6H_5$, $SO_2$-naphtyle ou $-COCH_2N(Y)-CH_2CH_2NH-Y$, représente également le groupe amidino,
$R^c$ représente un atome d'hydrogène ou le groupe amidino,
n représente le nombre 1 ou 0,
L représente le groupe amino,
T représente un groupe de formule $-CH_2-(O)_1$ ou $_0$-, -CH=CH- ou $-CH(R^d)-CH_2$-,
$R^d$ représentant un atome d'hydrogène ou $-NH-R^a$,
R' représente un atome d'hydrogène ou $-CO-R^o$,

$R^o$ représentant un groupe amino, NH-alkyle($C_{1-4}$), $NH(CH_2)_{1-4}-C_6H_5$, $-NH(CH_2)_{1-4}-C_6H_4$-Hal, $-NH-C_6H_4-COOH$, $-NH-C_6H_4$-COO-alkyle($C_{1-4}$) ou le reste d'un acide $\alpha$-aminocarboxylique rattaché par le groupe amino,

et hydrates ou produits de solvatation et sels physiologiquement utilisables de tels composés.

2. Composés selon la revendication 1, dans lesquels R est un groupe de formule R-1, en particulier ceux dans lesquels R-CO- est le groupe Aeg-Arg, Z-Aeg(Z)-Arg-, 2-naphtyl-$SO_2$Arg-, o-azido-benzoyl-Arg-, $N^2$-Boc-$N^6$-(1-iminoéthyl)-Lys- ou $N^2$ -Boc-$N^5$-(3a,4,5,6,7,7a-hexahydro-3a,7a,dihydroxy-1H-benzimidazol-2-yl)-Orn.

3. Composés selon la revendication 1, dans lesquels R est un groupe R-2, en particulier ceux dans lesquels R-CO-est le groupe p-(aminométhyl)hydrocinnamoyle.

4. Composés selon la revendication 1, dans lesquels R est un groupe R-3, en particulier ceux dans lesquels R-CO-est le groupe p-amidinohydrocinnamoyle, 3-(p-amidinophényl ou p-guanidinophényl)alanyle, N-Z- ou N-Boc-3-(p-amidinophényl)alanyle, N-Boc-3-(p-guanidinophényl)-alanyle, p-amidinophénoxyacétyle, p-amidinophénacétyle, 2-[(p-amidinophényl)-1,3-dioxolann-2-yl]-acétyle, (p-amidinobenzoyl)acétyle ou N-Boc-3-(p-acétimidoylamino-phényl)alanyle.

**5.** Composés selon l'une des revendications 1 à 3, dans lesquels R' représente un atome d'hydrogène ou le groupe -CO-Val-OH, -CO-Ser-OH, -CO-Phe-OH, 1-carboxy-2-(1-naphtyl)éthylidènecarbamoyle, -CO-Ile-OH, carboxyphényl-carbamoyle, isobutylcarbamoyle, p-fluorophénéthylcarbamoyle ou carboxycyclopentanecarbamoyle.

**6.** Composés selon la revendication 1, choisi dans l'ensemble constitué par les composés suivants:

[3-(p-amidinophényl)-DL-alanyl]-Gly-Asp-Val-OH,
Z-Aeg(Z)-Arg-Gly-Asp-Val-OH,
Aeg-Arg-Gly-Asp-Val-OH,
Aeg-Arg-Gly-Asp-Ser-OH,
N-Aeg-Arg-Gly-Asp-Nal(1) -OH,
Aeg-Arg-Gly-Asp-Ile-OH,
[$N^2$-Boc-$N^6$-(1-iminoéthyl)-L-lysyl]-Gly-Asp-Val-OH, acide N-[(o-azidobenzoyl)-Arg-Gly-Asp]anthranilique,
$N^2$ -Boc-$N^5$-(3a,4,5,6,7,7a-hexahydro-3a,7a,-dihydroxy-1H-benzimidazol-2-yl)-L-ornithyl]-Gly-Asp-Val-OH,
[N-Boc-3-(p-guanidinophényl)-DL-alanyl]-Gly-Asp-Val-OH,
[3-(p-amidinophényl)-DL-alanyl]-Gly-Asp-Nal(1)-OH,
[N-Boc-3-(p-amidinophényl)-DL-alanyl]-Gly-Asp-Val-OH,
(p-amidinohydrocinnamoyl)-Gly-Asp-Nal(1)-OH,
[3-(p-amidinophényl)-D-alanyl]-Gly-Asp-Val-OH,
(p-aminométhylhydrocinnamoyl)-Gly-Asp-Val-OH,
(p-amidinohydrocinnamoyl)-Gly-Asp-Val-OH,
[3-(p-amidinophényl)-L-alanyl]-Gly-Asp-Val-OH,
(p-amidinophénoxy)acétyl-Gly-Asp-Val-OH et
(p-amidinophényl)acétyl-Gly-Asp-Val-OH.

**7.** Composés de formule

$$R^2\text{-CONH-CH}_2\text{-CONH-CH}(R^4)\text{-CH}_2\text{COOR}^3 \qquad \text{II}$$

dans laquelle

$R^2$ est un groupe de formule

$$CH(NH\text{-}R^a)\text{-}(CH_2)_{1\text{-}6}\text{-}R^5 \qquad \text{(R-1a)}$$

$$\text{-}(T)_{1\ ou\ 0}\text{-}C_6H_4\text{-}CH_2\text{-}R^6 \qquad \text{(R-2a)}$$

ou

$$\text{-}\ (T)_{1\ ou\ 0}\text{-}C_6H_4\text{-}R^7 \qquad \text{(R-3a)}$$

où

$R^5$ représente un groupe guanidino protégé ou un groupe -NH-$R^b$,
$R^6$ représente un groupe amino ou guanidino protégé ou un groupe -NH-$R^c$,
$R^7$ représente un groupe amidino ou guanidino éventuellement protégé,

$R^3$ représente un atome d'hydrogène ou un groupe ester aisément séparable,
$R^4$ a la même signification que R' ou est un groupe -$COR^8$ dans lequel $R^8$ est un ester d'acide α-aminocarboxylique aisément séparable, relié par le groupe amino,

lorsque $R^3$ est un atome d'hydrogène et $R^4$ a la même signification que R', $R^2$ devant contenir au moins un groupe guanidino, amino ou amidino protégé $R^5$, $R^6$ ou $R^7$,

et $R^a$, $R^b$, $R^c$, R' et T ont les significations données dans la revendication 1.

**8.** Composés selon l'une des revendications 1 à 6, pour utilisation en tant que médicament, en particulier en tant qu'inhibiteur de la fixation de protéines adhésives à des plaquettes sanguines, ainsi que de l'agrégation plaquettaire et de l'adhésion de cellule à cellule.

**9.** Procédé pour la préparation des composés selon l'une des revendication 1 à 6, caractérisé en ce que

a) dans un composé de formule

$$R^2\text{-CONH-CH}_2\text{-CONH-CH}(R^4)\text{-CH}_2\text{COOR}^3 \qquad \text{II}$$

dans laquelle

$R^2$ est un groupe de formule

$$\text{-CH(NH-}R^a\text{)-(CH}_2)_{1\text{-}6}\text{-}R^5 \qquad \text{(R-1a)}$$

$$\text{-(T)}_{1\text{ ou }0}\text{-C}_6\text{H}_4\text{-CH}_2\text{-}R^6 \qquad \text{(R-2a)}$$

ou

$$\text{-(T)}_{1\text{ ou }0}\text{-C}_6\text{H}_4\text{-}R^7 \qquad \text{(R-3a)}$$

où

$R^5$ représente un groupe guanidino protégé ou un groupe -NH-$R^b$,
$R^6$ représente un groupe amino ou guanidino protégé ou un groupe -NH-$R^c$,
$R^7$ représente un groupe amidino ou guanidino éventuellement protégé,

$R^3$ représente un atome d'hydrogène ou un groupe ester aisément séparable,
$R^4$ a la même signification que R' ou est un groupe -$COR^8$ dans lequel $R^8$ est un ester d'acide α-aminocarboxylique aisément séparable, relié par le groupe amino,

lorsque $R^3$ est un atome d'hydrogène et $R^4$ a la même signification que R', $R^2$ devant contenir au moins un groupe guanidino, amino ou amidino protégé $R^5$, $R^6$ ou $R^7$,
et $R^a$, $R^b$, $R^c$, R' et T ont les significations données dans la revendication 1,
on élimine le(s) groupe(s) ester présent(s) et un ou plusieurs groupes amino, amidino ou guanidino protégés présents, ou
b) on fait réagir une amine de formule

```
CH(NH2)-(CH2)1-6-NH-C(NH)NH2
|                                        III
CONH-CH2-CONH-CH(R')-CH2COOH
```

avec un agent introduisant le groupe -$COC_6H_5$, -$COC_6H_4N_3$, -$SO_2C_6H_5$, -$SO_2$-naphtyle ou -$COCH_2N(Y)$-$CH_2CH_2NH$-Y, R' et Y ayant les significations données plus haut, ou
c) on fait réagir avec de la 1,2-cyclohexanedione un dérivé de guanidine de formule

$$CH(NH\text{-}R^9)\text{-}(CH_2)_{1\text{-}6}\text{-}NHC(NH)\text{-}NH_2$$
$$|$$
$$CONH\text{-}CH_2\text{-}CONH\text{-}CH(R')\text{-}CH_2COOH \qquad IV$$

dans laquelle $R^9$ représente un groupe -COO-alkyle($C_{1-4}$), Z, $-COC_6H_5$, $-COC_6H_4N_3$, $-SO_2C_6H_S$, $-SO_2$-naphtyle ou $-COCH_2N(Y')-CH_2CH_2NH-Y'$, dans lequel Y' représente le groupe Boc ou Z et R' a la signification donnée plus haut, ou

d) dans une amine de formule

$$NH\text{-}CH_2\text{-}CONH\text{-}CH_2\text{-}CONH\text{-}CH(R')\text{-}CH_2COOH$$
$$|$$
$$CO\text{-}(L)_{1\ ou\ 0}\text{-}C_6H_4\text{-}(CH_2)_{1\ ou\ 0}\text{-}NH_2 \qquad V$$

dans laquelle L représente un groupe $-CH_2(O)_{1}$ ou $_0$-, -CH=CH- ou $-CH(NH-R^9)-CH_2-$, et R' et $R^9$ ont les significations données plus haut,

on convertit le groupe amino en un groupe guanidino ou

e) ou soumet un nitrile de formule

$$N{\equiv}C\text{-}C_6H_4\text{-}(T)_{1\ ou\ 0}\text{-}CONH\text{-}CH_2\text{-}CONH\text{-}CH(R')\text{-}CH_2COOH \qquad VI$$

à une hydrogénation en l'amine,

f) si on le désire, on modifie la fonction d'un substituant réactif contenu dans le groupe R d'un composé de formule I, et

g) si on le désire, on convertit un composé de formule I en un sel ou on convertit un sel d'un composé de formule I en le composé libre de formule I.

**10.** Procédé pour la préparation de compositions pharmaceutiques, en particulier pour le traitement ou la prophylaxie de maladies qui sont causées par la fixation de protéines adhésives à des plaquettes sanguines, ainsi que par l'agrégation plaquettaire et l'adhésion de cellule à cellule, contenant en tant que substance active un composé selon l'une des revendications 1 à 6.

**11.** Utilisation d'un composé selon l'une des revendications 1 à 6, pour la fabrication de médicaments destinés au traitement ou à la prophylaxie de maladies qui sont causées par la fixation de protéines adhésives à des plaquettes sanguines, ainsi que par l'agrégation plaquettaire et l'adhésion de cellule à cellule.

**Revendications pour l'Etat contractant suivant : ES**

**1.** Procédé pour la préparation de dérivés de glycine de formule

$$R\text{-}CONH\text{-}CH_2\text{-}CONH\text{-}CH(R')\text{-}CH_2COOH \qquad I$$

dans laquelle

R est un groupe de formule

$$\text{-}CH(NH\text{-}R^a)\text{-}(CH_2)_{1\text{-}6}\text{-}NH\text{-}R^b \qquad (R\text{-}1)$$

$$-(T)_{1\ ou\ 0}-C_6H_4-CH_2NH-R^c \qquad (R\text{-}2)$$

ou

$$-(T)_{1\ ou\ 0}-C_6H_4-(NH)_n-C(NH)-L \qquad (R\text{-}3)$$

et
$R^a$ représente un atome d'hydrogène ou un groupe -COO-alkyle($C_{1-4}$), Z, $-COC_6H_5$, $-COC_6H_4N_3$, $-SO_2C_6H_5$, $-SO_2$-naphtyle ou $-COCH_2N(Y)-CH_2CH_2NH-Y$,
Y représentant un atome d'hydrogène ou le groupe Boc ou Z,
$R^b$ représente un groupe de formule $-C(NH)(CH_2)_{0-3}$ ou

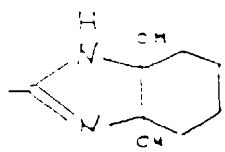

ou, lorsque $R^a$ est un groupe de formule $-COC_6H_4N_3$, $-SO_2C_6H_5$, $SO_2$-naphtyle ou $-COCH_2N(Y)-CH_2CH_2NH-Y$, représente également le groupe amidino,
$R^c$ représente un atome d'hydrogène ou le groupe amidino,
n représente le nombre 1 ou 0,
L représente le groupe amino,
T représente un groupe de formule $-CH_2-(O)_1$ ou $_0$-, -CH=CH- ou $-CH(R^d)-CH_2-$,

$R^d$ représentant un atome d'hydrogène ou $-NH-R^a$,

R' représentant un atome d'hydrogène $-CO-R^o$,

$R^o$ représentant un groupe amino, NH-alkyle($C_{1-4}$), $NH(CH_2)_{1-4}$ $-C_6H_5$, $NH(CH_2)_{1-4}$ $C_6H_4$ Hal, $-NH-C_6H_4-COOH$, $-NH-C_6H_4-COO$-alkyle($C_{1-4}$) ou le reste d'un acide α-aminocarboxylique rattaché par le groupe amino,

ainsi que d'hydrates ou de produits de solvatation et de sels physiologiquement utilisables de tels composés, caractérisé en ce que

a) dans un composé de formule

$$R^2-CONH-CH_2-CONH-CH(R^4)-CH_2COOR^3 \qquad II$$

dans laquelle

$R^2$ est un groupe de formule

$$-CH(NH-R^a)-(CH_2)_{1-6}-R^5 \qquad (R\text{-}1a)$$

$$-(T)_{1\ ou\ 0}-C_6H_4-CH_2-R^6 \qquad (R\text{-}2a)$$

ou

$-(T)_{1\ ou\ 0}\ C_6H_4-R^7$ (R-3a)

où

$R^5$ représente un groupe guanidino protégé ou un groupe $-NH-R^b$,
$R^6$ représente un groupe amino ou guanidino protégé ou un groupe $-NH-R^c$,
$R^7$ représente un groupe amidino ou guanidino éventuellement protégé,

$R^3$ représente un atome d'hydrogène ou un groupe ester aisément séparable,
$R^4$ a la même signification que R' ou est un groupe $-COR^8$ dans lequel $R^8$ est un ester d'acide α-aminocarboxylique aisément séparable, relié par le groupe amino,

lorsque $R^3$ est un atome d'hydrogène et $R^4$ a la même signification que R', $R^2$ devant contenir au moins un groupe guanidino, amino ou amidino protégé $R^5$, $R^6$ ou $R^7$,
et $R^a$, $R^b$, $R^c$, R' et T ont les significations données plus haut,
on élimine le(s) groupe(s) ester présent(s) et un ou plusieurs groupes amino, amidino ou guanidino protégés présents, ou

b) on fait réagir une amine de formule

$$\begin{array}{l} CH(NH_2)-(CH_2)_{1-6}-NH-C(NH)NH_2 \\ | \\ CONH-CH_2-CONH-CH(R')-CH_2COOH \end{array} \qquad III$$

avec un agent introduisant le groupe $-COC_6H_5$, $-COC_6H_4N_3$, $-SO_2C_6H_5$, $-SO_2$-naphtyle ou $-COCH_2N(Y)-CH_2CH_2NH-Y$, R' et Y ayant les significations données plus haut, ou

c) on fait réagir avec de la 1,2-cyclohexanedione un dérivé de guanidine de formule

$$\begin{array}{l} CH(NH-R^9)-(CH_2)_{1-6}-NHC(NH)-NH_2 \\ | \\ CONH-CH_2-CONH-CH(R')-CH_2COOH \end{array} \qquad IV$$

dans laquelle $R^9$ représente un groupe $-COO$-alkyle$(C_{1-4})$, Z, $-COC_6H_5$, $-COC_6H_4N_3$, $-SO_2C_6H_5$, $-SO_2$-naphtyle ou $-COCH_2N(Y')-CH_2CH_2NH-Y'$, dans lequel Y' représente le groupe Boc ou Z
et R' a la signification donnée plus haut, ou d) dans une amine de formule

$$\begin{array}{l} NH-CH_2-CONH-CH_2-CONH-CH(R')-CH_2COOH \\ | \\ CO-(L)_{1\ ou\ 0}-C_6H_4-(CH_2)_{1\ ou\ 0}-NH_2 \end{array} \qquad V$$

dans laquelle L représente un groupe $-CH_2(O)_1$ ou $_0$-, -CH=CH- ou $-CH(NH-R^9)-CH_2-$, et R' et $R^9$ ont les significations données plus haut,
on convertit le groupe amino en un groupe guanidino ou

e) ou soumet un nitrile de formule

$$N{\equiv}C-C_6H_4-(T)_{1\ ou\ 0}-CONH-CH_2-CONH-CH(R')-CH_2COOH \qquad VI$$

à une hydrogénation en l'amine,

f) si on le désire, on modifie la fonction d'un substituant réactif contenu dans le groupe R d'un composé de formule I, et

g) si on le désire, on convertit un composé de formule I en un sel ou on convertit un sel d'un composé de formule I en le composé libre de formule I.

**2.** Procédé selon la revendication 1, dans lequel R est un groupe de formule R-1, en particulier dans lequel R-CO- est le groupe Aeg-Arg, Z-Aeg(Z)-Arg-, 2-naphtyl-$SO_2$Arg-, o-azido-benzoyl-Arg-, $N^2$-Boc-$N^6$-(1-iminoéthyl)-Lys- ou $N^2$-Boc-$N^5$-(3a,4,5,6,7,7a-hexahydro-3a,7a-dihydroxy-1H-benzimidazol-2-yl)-Orn.

**3.** Procédé selon la revendication 1, dans lequel R est un groupe R-2, en particulier dans lequel R-CO- est le groupe p-(aminométhyl)hydrocinnamoyle.

**4.** Procédé selon la revendication 1, dans lequel R est un groupe R-3, en particulier dans lequel R-CO- est le groupe p-amidinohydrocinnamoyle, 3-(p-amidinophényl ou p-guanidinophényl)alanyle, N-Z- ou N-Boc-3-(p-amidinophényl)alanyle, N-Boc-3-(p-guanidinophényl)alanyle, p-amidinophénoxyacétyle, p-amidinophénacétyle, 2-[(p-amidinophényl)-1,3-dioxolann-2-yl]-acétyle, (p-amidinobenzoyl)acétyle ou N-Boc-3-(p-acétimidoylaminophényl)-alanyle.

**5.** Procédé selon l'une des revendications 1 à 3, dans lequel R' représente un atome d'hydrogène ou le groupe -CO-Val-OH, -CO-Ser-OH, -CO-Phe-OH, 1-carboxy-2-(1-naphtyl)éthylidènecarbamoyle, -CO-Ile-OH, carboxyphénylcarbamoyle, isobutylcarbamoyle, p-fluorophénéthylcarbamoyle ou carboxycyclopentanecarbamoyle.

**6.** Procédé selon la revendication 1, dans lequel le composé préparé est le suivant:

[3-(p-amidinophényl)-DL-alanyl]-Gly-Asp-Val-OH,
Z-Aeg(Z)-Arg-Gly-Asp-Val-OH,
Aeg-Arg-Gly-Asp-Val-OH,
Aeg-Arg-Gly-Asp-Ser-OH,
N-Aeg-Arg-Gly-Asp-Nal(1) -OH,
Aeg-Arg-Gly-Asp-Ile-OH,
[$N^2$-Boc-$N^6$-(1-iminoéthyl)-L-lysyl]-Gly-Asp-Val-OH, acide N-[(o-azidobenzoyl)-Arg-Gly-Asp]anthranilique,
$N^2$-Boc-$N^5$-(3a,4,5,6,7,7a-hexahydro-3a,7a,-dihydroxy-1H-benzimidazol-2-yl)-L-ornithyl]-Gly-Asp-Val-OH,
[N-Boc-3-(p-guanidinophényl)-DL-alanyl]-Gly-Asp-Val-OH, [3-(p-amidinophényl)-DL-alanyl]-Gly-Asp-Nal(1)-OH,
[N-Boc-3-(p-amidinophényl)-DL-alanyl]-Gly-Asp-Val-OH, (p-amidinohydrocinnamoyl)-Gly-Asp-Nal(1)-OH,
[3-(p-amidinophényl)-D-alanyl]-Gly-Asp-Val-OH,
(p-aminométhylhydrocinnamoyl)-Gly-Asp-Val-OH,
(p-amidinohydrocinnamoyl)-Gly-Asp-Val-OH,
[3-(p-amidinophényl)-L-alanyl]-Gly-Asp-Val-OH,
(p-amidinophénoxy)acétyl-Gly-Asp-Val-OH ou
(p-amidinophényl)acétyl-Gly-Asp-Val-OH.

**7.** Procédé selon la revendication 1, caractérisé en ce que

a) l'élimination des groupes protecteurs est effectuée avec un acide, avec une base ou par hydrogénation en présence d'un catalyseur à base de métal noble, dans chaque cas dans un solvant, à une température comprise entre environ 0°C et 40°C, ou
b) on fait réagir une amine de formule III avec du chlorhydrate de pyridine, en présence d'une base et d'hexafluorophosphate de 1,1,3,3-tétraméthyl-2-[4-oxo-1,2,3-benzotriazin-3(4H)-yl]uronium, ou avec du chlorure de naphtalène-2-sulfonyle et une base, ou avec Z-Aeg(Z)-O-Su en présence d'une base, dans chaque cas dans un solvant, à une température de jusqu'à 40°C environ, ou
c) on fait réagir un dérivé de guanidine de formule IV avec de 1,2-cyclohexanedione dans un tampon borate de sodium, sous une atmosphère inerte, à une température de jusqu'à 40°C environ,
d) on soumet un nitrile de formule VI à une hydrogénation catalytique dans une solution alcoolique d'ammoniac, ou

e) on élimine les groupes protecteurs d'un groupe protégé R-CO- ou on fait réagir un groupe amino primaire avec du dicarbonate de di-tert-butyle dans un solvant, en présence d'une base, à une température de jusqu'à 40°C environ.

**8.** Procédé pour la préparation d'un composé de formule

$$R^2\text{-CONH-CH}_2\text{-CONH-CH}(R^4)\text{-CH}_2\text{COOR}^3 \qquad \text{II}$$

dans laquelle

$R^2$ est un groupe de formule

$$\text{-CH(NH-}R^a\text{)-(CH}_2)_{1\text{-}6}\text{-}R^5 \qquad \text{(R-1a)}$$

$$\text{-(T)}_{1\text{ ou }0}\text{-C}_6\text{H}_4\text{-CH}_2\text{-}R^6 \qquad \text{(R-2a)}$$

ou

$$\text{-(T)}_{1\text{ ou }0}\text{-C}_6\text{H}_4\text{-}R^7 \qquad \text{(R-3a)}$$

où

$R^5$ représente un groupe guanidino protégé ou un groupe -NH-$R^b$,
$R^6$ représente un groupe amino ou guanidino protégé ou un groupe -NH-$R^c$,
$R^7$ représente un groupe amidino ou guanidino éventuellement protégé,

$R^3$ représente un atome d'hydrogène ou un groupe ester aisément séparable,
$R^4$ a la même signification que R' ou est un groupe -$COR^8$ dans lequel $R^8$ est un ester d'acide α-aminocarboxylique aisément séparable, relié par le groupe amino,

lorsque $R^3$ est un atome d'hydrogène et $R^4$ a la même signification que R', $R^2$ devant contenir au moins un groupe guanidino, amino ou amidino protégé $R^5$, $R^6$ ou $R^7$,
et $R^a$, $R^b$, $R^c$, R' et T ont les significations données dans la revendication 1,
caractérisé en ce que

a) on convertit un nitrile de formule

$$\text{NC-C}_6\text{H}_4\text{-(T)}_{1\text{ ou }0}\text{-CONH-CH}_2\text{-CONH-CH}(R^4)\text{-CH}_2\text{COOR}^3 \qquad \text{VII}$$

en passant par les thioamides et S-méthyliminoesters correspondants, en un composé de formule II dans lequel $R^2$ est un groupe -$(T)_{1\text{ ou }0}$-$C_6H_4$-C(NH)$NH_2$,
b) on convertit un composé de formule

$$\begin{array}{l}\text{NHCO-(T)}_{1\text{ ou }0}\text{-C}_6\text{H}_4\text{-NO}_2\\ |\\ \text{CH}_2\text{-CONH-CH}(R^4)\text{-CH}_2\text{COOR}^3\end{array} \qquad \text{VIII}$$

en passant par l'amine correspondante et le dérivé de de guanidine correspondant protégé par le groupe nitro, en un composé de formule II dans lequel $R^2$ est un groupe de formule -$(T)_{1\text{ ou }0}$-$C_6H_4$-NHC(NH)$NH_2$, ou
c) on prépare un composé de formule II, fixé sur un support, par synthèse en phase solide.

**9.** Procédé pour la préparation d'une composition pharmaceutique, en particulier pour le traitement ou la prophylaxie de maladies qui sont causées par la fixation de protéines adhésives à des plaquettes sanguines, ainsi que par l'agrégation plaquettaire et l'adhésion de cellule à cellule, caractérisé en ce que l'on met sous une forme galénique d'administration un composé pouvant être obtenu selon l'une des revendications 1 à 6.

**10.** Utilisation d'un composé pouvant être obtenu selon l'une des revendications 1 à 6, pour la fabrication de médicaments destinés au traitement ou à la prophylaxie de maladies qui sont causées par la fixation de protéines adhésives à des plaquettes sanguines, ainsi que par l'agrégation plaquettaire et l'adhésion de cellule à cellule.